# EUROPÄISCHE PATENTSCHRIFT

(11) **EP 2 673 287 B1**
(45) Veröffentlichungstag und Bekanntmachung des Hinweises auf die Patenterteilung: **30.08.2017**
(21) Anmeldenummer: 12700091.7
(22) Anmeldetag: 09.01.2012
(51) Int. Cl.: C07K 5/02, A61P 37/00, A61P 35/00, A61P 25/14, A61P 17/00, A61P 17/06, A61P 19/02, A61P 19/08, A61K 38/05

(54) **AMINOSTATIN-DERIVATE ZUR BEHANDLUNG VON ARTHROSE**
AMINO STATIN DERIVATIVES FOR THE TREATMENT OF ARTHROSIS
DÉRIVÉS D'AMINOSTATINE POUR LE TRAITEMENT DE L'ARTHROSE

(30) Priorität: 08.02.2011 EP 11000974
(43) Veröffentlichungstag der Anmeldung: 18.12.2013
(73) Patentinhaber: Merck Patent GmbH, 64293 Darmstadt (DE)
(72) Erfinder: KLEIN, Markus, 64291 Darmstadt (DE); TSAKLAKIDIS, Christos, 69469 Weinheim (DE); GUEHRING, Hans, 65343 Eltville (DE); LEUTHNER, Birgitta, 64287 Darmstadt (DE)
(86) Internationale Anmeldenummer: PCT/EP2012/000069
(87) Internationale Veröffentlichungsnummer: WO 2012/107153

(56) Entgegenhaltungen:
- EP-A2- 0 161 588
- EP-A2- 0 198 271
- US-A- 3 971 736
- US-A- 5 849 691
- KATANO MASAYOSHI ET AL: "Implication of granulocyte-macrophage colony-stimulating factor induced neutrophil gelatinase-associated lipocalin in pathogenesis of rheumatoid arthritis revealed by proteome analysis", ARTHRITIS RESEARCH AND THERAPY, BIOMED CENTRAL, LONDON, GB, Bd. 11, Nr. 1, 8. Januar 2009 (2009-01-08) , Seite R3, XP021053368, ISSN: 1478-6354, DOI: 10.1186/AR2587
- R.A.JUPP, B.M.DUNN, J.W.JACOBS, G.VLASUK, K.E.ARCURI, D.F.VEBER, D.S.PERLOW, L.S.PAYNE, J.BOGER, S. DE LASZLO ET AL.: "The selectivity of statine-based inhibitors against various human aspartic proteinases", BIOCHEMICAL JOURNAL, Bd. 265, Nr. 3, 1. Februar 1990 (1990-02-01), Seiten 871-878, XP002677098,

## Beschreibung

Die vorliegende Erfindung betrifft Verbindungen der Formel I und insbesondere Arzneimittel enthaltend wenigstens eine Verbindung der Formel I zur Verwendung bei der Behandlung und/oder Prophylaxe von physiologischen und/oder pathophysiologischen Zuständen, an deren Auslösung Cathepsin D beteiligt ist, insbesondere zur Verwendung bei der Behandlung und/oder Prophylaxe von Arthrose, traumatischen Knorpelverletzungen Arthritis, Schmerz, Allodynie oder Hyperalgesie.

### Hintergrund der Erfindung

Arthrose ist die weltweit am weitesten verbreitete Gelenkserkrankung und radiologische Hinweise auf Arthrose findet man in der Mehrheit der über 65-Jährigen. Trotz dieser wesentlichen Bedeutung für das Gesundheitssystem bleiben die Ursachen der Arthrose bislang unklar und wirksame vorbeugende Maßnahmen weiterhin ein entferntes Ziel. Die Verringerung des Gelenkspaltes (bedingt durch die Zerstörung des Gelenkknorpels), verbunden mit Veränderungen im subchondralen Knochen und Osteophytenbildung sind die radiologischen Charakteristiken der Erkrankung. Für den Patienten stehen jedoch Schmerzen (belastungsabhängige und nächtliche Ruheschmerzen) mit nachfolgenden Funktionseinschränkungen im Fordergrund. Diese sind es auch, die den Patienten in die soziale Isolierung mit entsprechenden Folgeerkrankungen treiben.

Der Begriff Arthrose bezeichnet nach einer nicht-amtlichen Definition in Deutschland einen "Gelenkverschleiß", der das altersübliche Maß übersteigt. Ursächlich werden ein Übermaß an Belastung (etwa erhöhtes Körpergewicht), angeborene oder traumatisch bedingte Ursachen, wie Fehlstellungen der Gelenke, oder auch knöcherne Deformierungen durch Knochenerkrankungen wie Osteoporose gesehen. Die Arthrose kann ebenfalls als Folge einer anderen Erkrankung, beispielsweise einer Gelenkentzündung (Arthritis) entstehen (sekundäre Arthrose) oder mit überlastungsbedingter Ergussbildung (sekundäre Entzündungsreaktion) einhergehen (aktivierte Arthrose). Die anglo-amerikanische Fachliteratur unterscheidet zwischen der Osteoarthrose (engl. osteoarthritis [OA]), bei welcher die Zerstörung der Gelenkflächen wahrscheinlich hauptsächlich auf Belastungseinwirkungen zurückzuführen sind, und der Arthritis (engl. arthritis, rheumatoid arthritis [RA]), bei welcher die Gelenkdegeneration durch eine Entzündungskomponente im Vordergrund steht.

Grundsätzlich unterscheidet man die Arthrose auch nach ihrer Ursache. Der Arthrosis alcaptonurica liegt eine vermehrte Ablagerung von Homogenitinsäure in Gelenken bei vorbestehender Alkaptonurie zugrunde. Bei der hämophilen Arthrose liegen regelmäßige intraartikuläre Blutungen bei Hämophilie (Blutergelenk) vor. Die Arthrosis urica wird durch den mechanischen Einfluss von Uratkristallen (Harnsäure) auf den gesunden Knorpel hervorgerufen (W. Pschyrembel et al.: Klinisches Wörterbuch mit klinischen Syndromen und einem Anhang Nomina Anatomica. Verlag Walter de Gruyter & Co, 253. Auflage, 1977).

Klassische Ursache einer Arthrose stellt die Dysplasie von Gelenken dar. Am Beispiel der Hüfte wird deutlich, dass die mechanisch am meisten belastete Zone bei einer physiologischen Hüftstellung eine deutlich größere Fläche darstellt, als bei einer dysplastischen Hüfte. Die Belastungen durch die auf das Gelenk einwirkenden Kräfte sind von der Gelenkform jedoch weitgehend unabhängig. Sie verteilen sich im Wesentlichen auf die Hauptbelastungszone(n). Dadurch wird bei einer kleineren Zone eine höhere Druckbelastung als bei einer größeren auftreten. Die biomechanische Druckbelastung des Gelenkknorpels ist somit bei einer dysplastischen Hüfte größer als bei physiologischer Hüftstellung. Diese Gesetzmäßigkeit wird allgemein ursächlich für das gehäufte Auftreten arthrotischer Veränderungen an von der anatomischen Idealform abweichenden tragenden Gelenken gesehen.

Sind die Folgen einer Verletzung für einen vorzeitigen Verschleiß verantwortlich, so spricht man von einer posttraumatischen Arthrose. Als weitere Ursachen einer sekundären Arthrose werden mechanische, entzündliche, metabolische, chemische (Chinolone), trophische, hormonelle, neurologische und genetische Gründe diskutiert. In den meisten Fällen wird als Diagnose jedoch eine idiopatische Arthrose angegeben, womit der Arzt ein scheinbares Fehlen einer ursächlichen Erkrankung zum Ausdruck bringt (H. I. Roach und S. Tilley, Bone and Osteoarthritis F. Bronner und M. C. Farach-Carson (Editors), Verlag Springer, Band 4, 2007).

Medikamentöse Ursachen für eine Arthrose können beispielsweise Antibiotika vom Typ der Gyrasehemmer (Fluorchinnolone, wie Ciprofloxacin, Levofloxacin) sein. Diese Arzneimittel führen in schlecht vaskularisierten Geweben (hyaliner Gelenkknorpel, Sehnengewebe) zu einer Komplexierung von Magnesium-Ionen, was zur Folge hat, dass irreversible Schäden am Bindegewebe entstehen. Diese Schäden sind bei Kindern und Jugendlichen in der Regel in der Wachstumsphase ausgeprägter. Tendopathien und Arthropathien sind bekannte Nebenwirkungen dieser Medikamentenklasse. Beim Erwachsenen führen diese Antibiotika nach Informationen von unabhängigen Pharmakologen und Rheumatologen zu einem beschleunigten physiologischen Abbau des hyalinen Gelenkknorpels (M. Menschik et al., Antimicrob. Agents Chemother. 41, 1997, S. 2562-2565; M. Egerbacher et al., Arch. Toxicol. 73, 2000, S. 557-563; H. Chang et al., Scand. J. Infect. Dis. 28, 1996, S. 641-643; A. Chaslerie et al.,Therapie 47, 1992, S. 80). Auch eine langjährige Behandlung mit Phenprocoumon kann durch Abnahme der Knochendichte bei Belastungen der Gelenkbinnenstruktur eine Arthrose begünstigen.

Neben dem Alter sind mechanische Überbelastungen, (Mikro-) Traumata, durch Verlust der Sicherungsmechanismen verursachte Destabilisierungen der Gelenke, sowie genetische Faktoren als Risikofaktoren für Osteoarthrose bekannt. Jedoch sind weder Entstehung noch Interventionsmöglichkeiten vollständig geklärt (H. I. Roach und S. Tilley, Bone and Osteoarthritis F. Bronner und M. C. Farach-Carson (Editors), Verlag Springer, Band 4, 2007).

In einem von Arthrose betroffenen Gelenk ist der Gehalt von Stickstoffmonoxid zeitweise erhöht. Ähnliches konnte durch hohe mechanische Reizung von Knorpelgewebe beobachtet werden (P. Das et al., Journal of Orthopaedic Research 15, 1997, S. 87-93. A. J. Farrell et al. Annals of the Rheumatic Diseases 51, 1992, S. 1219-1222; B. Fermor et al., Journal of Orthopaedic Research 19, 2001, S. 729-737), wohingegen eine mäßige mechanische Stimulation sich eher positiv auswirkt. Damit sind mechanische Krafteinwirkungen ursächlich an dem Voranschreiten der Osteoarthrose beteiligt (X. Liu et al., Biorheology 43, 2006, S. 183-190).

Grundsätzlich verfolgt die Therapie der Arthrose zwei Ziele. Zum einen die Schmerzfreiheit unter üblicher Belastung und zum anderen die Verhinderung mechanischer Einschränkungen oder Veränderungen eines Gelenkes. Diese Ziele können langfristig nicht durch eine Schmerzbehandlung als rein symptomatischer Therapieansatz erreicht werden, da durch diese das Voranschreiten der Erkrankung nicht aufgehalten werden kann. Soll letzteres erreicht werden, muss die Knorpelzerstörung gestoppt werden. Da sich der Gelenksknorpel beim erwachsenen Patienten nicht regenerieren kann, ist die Beseitigung pathogenetischer Faktoren wie Gelenkdysplasien oder Fehlstellungen, die zu vermehrter punktueller Druckbelastung des Gelenkknorpels führen, zusätzlich enorm wichtig.

Schließlich versucht man mit medikamentöser Hilfe die Degenerationsprozesse im Knorpelgewebe zu verhindern oder zu stoppen. Wesentlich für den Funktionszustand und damit der Widerstandsfähigkeit gegenüber Belastungen des Gelenkknorpels ist die extrazelluäre Matrix, die in erster Line aus Kollagenen, Proteoglykanen und Wasser besteht. Zu den Enzymen, die an der Degradation der extrazellulären Matrix beteiligt sind, zählen vor allem die Metalloproteasen, Aggrecanasen und die Cathepsin-Enzyme. Aber auch weitere Enzyme können prinzipiell Knorpelmatrix abbauen, beispielhaft werden Plasmin, Kallikrein, Neutrophilelastase, Tryptase und Chymase genannt.

Cathepsine gehören der Papain-Superfamilie der lysosomalen Proteasen an. Cathepsine sind an der normalem Proteolyse und dem Umsatz von Zielproteinen und Geweben beteiligt, sowie an der Initiierung von proteolytischen Kaskaden und Proenmzyaktivierungen. Darüber hinaus sind sie an der MHC Klasse II Expression beteiligt (Baldwin (1993) Proc. Natl. Acad. Sci., 90: 6796-6800; Mixuochi (1994) Immunol. Lett., 43:189-193). Eine abnorme Cathepsin-Expression kann allerdings zu schwerwiegenden Erkrankungen führen. So konnte eine erhöhte Cathepsin-Expression in Krebszellen, beispielsweise bei Brust-, Lungen-, Prostata-, Glioblastom-, und Kopf- und Halskrebs nachgewiesen werden und es konnte gezeigt werden, dass Cathepsine mit einem ungenügenden Therapieerfolg bei Brust-, Lungen-, Kopf- und Halskrebs, sowie Gehirntumoren assoziiert sind (Kos et al. (1998) Oncol. Rep., 5:1349-1361; Yan et al. (1998) Biol. Chem., 379:113-123; Mort et al. ; (1997) Int. J. Biochem. Cell Biol., 29: 715-720; Friedrick et al. (1999) Eur. J Cancer, 35:138-144). Außerdem ist augenscheinlich eine abnorme Cathepsin-Expression and der Ausbildung von entzündlichen und nicht-entzündlichen Erkrankungen beteiligt, wie beispielsweise rheumatoider Arthritis und Osteoarthrose (Keyszer (1995) Arthritis Rheum., 38:976-984).

Der molekulare Mechanismus der Cathepsinaktivität ist nicht vollständig aufgeklärt. Auf der einen Seite wurde gefunden, dass beispielweise eine induzierte Cathepsin-Expression B-Zellen, denen Serum entzogen wird, vor der Apoptose bewahrt und dass eine Behandlung der Zellen mit Antisense-Oligonukleotiden von Cathepsin B eine Apoptose induziert (Shibata et al. (1998) Biochem. Biophys. Res. Commun., 251: 199-20; Isahara et at. (1999) Neuroscience, 91:233-249). Diese Berichte legen eine anti-apoptotische Rolle der Cathepsine nahe. Diese stehen jedoch im genauen Gegensatz zu früheren Berichten, die Cathepsine als Apoptosemediatoren beschreiben (Roberts et al (1997) Gastroenterology, 113: 1714-1726; Jones et al. (1998) Am. J. Physiol., 275: G723-730).

Cathepsine werden als inaktive Zymogene an Ribosomen synthetisiert und ins lysosomale System überführt. Nach proteolytischer Abspaltung des N-terminalen Propeptids steigt die Cathepsinkonzentration im saueren Milieu der Lysosomen auf bis zu 1 mM an und die Cathepsine werden von den Lysosomen ins extrazelluläre Medium freigesetzt.

Bei den Cathepsinen unterscheidet man die Cysteincathepsine B, C, H, F, K, L, O, S, V und W, die Aspartylcathepsine D und E und das Serincathepsin G.

Beispiele für Cathepsin-Inhibitoren in der klinischen Entwicklung sind Cathepsin K Inhibitoren zur Behandlung von Arthrose und Cathepsin S Inhibitoren zur Behandlung von Arthritis, neuropathischem Schmerz und Psoriasis.

Zu den Aspartylproteasen zählt man neben Cathepsin D auch die HIV Aspartylprotease (HIV-1 Protease), Renin, Pepsin A and C, BACE (Asp2, Memapsin), Plasmepsine und die Aspartylhämoglobinasen (Takahashi, T. et al., Ed. Aspartic Proteinases Structure, Function, Biology and Biomedical Implications (Plenum Press, New York, 1995), Adams, J. et al., Ann. Rep. Med. Chem. 31, 279-288, 1996; Edmunds J. et al., Ann. Rep. Med. Chem. 31, 51-60, 1996; Miller, D. K. et al., Ann. Rep. Med. Chem 31, 249-268, 1996). Cathepsin D ist normalerweise an der Degradation von intrazellulären oder phagozytierten Proteinen beteiligt und spielt somit eine wichtige Rolle im Proteinmetabolismus (Helseth, et al., Proc. Natl. Acad. Sci. USA 81, 3302-3306, 1984), beim Proteinkatabolismus (Kay, et al., Intracellular Protein Catabolism (eds. Katunuma, et al., 155-162, 1989) und bei der Antigenprozessierung (Guagliardi, et al., Nature, 343, 133-139, 1990; Van Noort, et al., J. Biol. Chem., 264, 14159-14164, 1989).

Erhöhte Cathepsin D-Spiegel werden mit einer Reihe von Krankheiten in Zusammenhang gebracht. So korrelieren erhöhte Cathepsin D-Spiegel mit schlechter Prognose bei Brustkrebs und mit erhöhter Zellinvasion und erhöhtem Risiko von Metastasen, sowie kürzerer rezidivfreien Überlebenszeit nach Therapie und einer insgesamt niedrigeren Überlebensrate (Westley B. R. et al., Eur. J. Cancer 32, 15-24, 1996; Rochefort, H., Semin. Cancer Biol. 1:153, 1990; Tandon, A. K. et al., N. Engl. J. Med. 322, 297, 1990). Die Cathepsin D-Sekretionsrate bei Brustkrebs wird durch eine Überexpression des Gens und durch ein verändertes Processing des Proteins vermittelt. Erhöhte Spiegel von Cathepsin D und anderer Proteasen, wie beispielsweise Kollagenase, hergestellt in unmittelbarer Nähe zu einem wachsenden Tumor, könnten dabei die extrazelluläre Matrix in der Tumorumgebung degradieren und hierdurch die Ablösung von Tumorzellen und die Invasion in neue Gewebe über das Lymph- und Kreislaufsystem vermitteln (Liotta L. A., Scientific American Feb:54, 1992; Liotta L. A. and Stetler-Stevenson W. G., Cancer Biol. 1:99, 1990; Liaudet E., Cell Growth Differ. 6:1045-1052, 1995; Ross J. S., Am. J. Clin. Pathol. 104:36-41, 1995; Dickinson A. J., J. Urol. 154:237-241, 1995).

Cathepsin D wird außerdem mit degenerativen Veränderungen im Gehirn in Verbindung gebracht, wie beispielsweise Alzheimer Krankheit. So ist Catepsin D mit der Spaltung des Amyloid-β-Protein-Vorläufers bzw. eines mutanten Vorläufers assoziiert, der die Expression des Amyloid-Proteins in transfizierten Zellen erhöht (Cataldo, A. M. et al., Proc. Natl. Acad. Sci. 87:3861, 1990; Ladror, U. S. et al., J. Biol. Chem. 269:18422, 1994, Evin G., Biochemistry 34:14185-14192, 1995). Das Amyloid-β-Protein, das durch die Proteolyse des Amyloid-β-Protein-Vorläufers entsteht, führt zur Bildung von Plaques im Gehirn und scheint für die Ausbildung der Alzheimer-Krankheit verantwortlich zu sein. Erhöhte Cathepsin D-Spiegel wurden auch in der Zerebrospinalflüssigkeit von Alzheimer-Patienten gefunden und es konnte eine hohe proteolytische Aktivität von Cathepsin D gegenüber dem mutanten Amyloid-β-Protein-Vorläufer gezeigt werden (Schwager, A. L., et al. J. Neurochem. 64:443, 1995). Darüber hinaus wird eine signifikante Zunahme der Cathepsin D-Aktivität in Biopsien von Chorea Huntington-Patienten gemessen (Mantle D., J. Neurol. Sci. 131:65-70, 1995).

Bei der Ausprägung einer Arthrose spielt Cathepsin D vermutlich auf verschiedenen Ebenen eine wesentliche Rolle. So werden in Hunden mit spontaner Arthrose im Vergleich gesunden Hunden im Gelenkknorpel des Hüftgelenkkopfes erhöhte mRNA-Spiegel von Cathepsin D gemessen (Clements D. N. et al., Arthritis Res. Ther.. 2006; 8(6):R158; Ritchlin C. et al., Scand. J. Immunnol. 40:292-298, 1994). Auch Devauchelle V. et al. (Genes Immun. 2004, 5(8):597-608) zeigen bei menschlichen Patienten unterschiedliche Expressionsraten von Cathepsin D bei Arthrose im Vergleich zu rheumatoider Arthritis (siehe auch Keyszer G. M., Arthritis Rheum. 38:976-984, 1995). Auch bei Mucolipidose scheint Cathepsin D eine Rolle zu spielen (Kopitz J., Biochem. J. 295, 2:577-580, 1993).

Die lysosomale Endopeptidase Cathepsin D ist die am weitesten verbreitete Proteinase in den Chondrozyten (Ruiz-Romero C. et al., Proteomics. 2005, 5(12):3048-59). Die proteolytische Aktivität von Cathepsin D ist zudem in kultiviertem Synovium aus Osteoarthrose-Patienten nachgewiesen worden (Bo G. P. et al., Clin. Rheumatol. 2009, 28(2):191-9) und auch in Synovectomiegewebe von Patienten mit rheumatoider Arthritis findet man eine erhöhte proteolytische Aktivität (Taubert H. et al., Autoimmunity. 2002, 35(3):221-4). Lorenz et al. (Proteomics. 2003, 3(6):991-1002) schreiben so auch, dass zwar die lysosomale und sekretierte Aspartylprotease Cathepsin D im Gegensatz zu den Cathepsinen B und L noch nicht bezüglich Arthritis und Arthrose im Detail studiert wurde, jedoch fanden Lorenz et al. höhere Proteinspiegel von Cathepsin D im Synovialgewebe von Patienten mit Arthrose im Vergleich zu Patienten mit rheumatoider Arthritis.

Gedikoglu et al. (Ann. Rheum. Dis. 1986, 45(4):289-92) konnten ebenfalls eine erhöhte proteolytische Aktivität von Cathepsin D in Synovialgewebe und Byliss und Ali (Biochem. J. 1978, 171(1):149-54) im Knorpel von Patienten mit Arthrose nachweisen.

Bei Arthrose kommt es zu einem Absinken des pH-Werts in umschriebenen Bereichen des Knorpels. Dieses Absinken des pH-Werts ist für das Verständnis der katabolen Prozesse im Knorpel von entscheidender Bedeutung.

Bei Arthrose findet man so auch eine direkte Korrelation von niedrigem pH-Wert im Gelenkgewebe und der Schwere und dem Fortschreiten der Erkrankung. Bei einen pH-Wert von 5,5 kommt es zu einem Selbstverdau des Knorpels. Dieser kann in Explantkulturen (beispielsweise von Maus, Rind oder Mensch) fast vollständig durch Pepstatin oder Ritonavir gehemmt werden. Dies legt eine wesentliche Rolle, wenn nicht sogar eine Schlüsselrolle von Cathepsin D bei der Arthrose nahe, da Pepstatin Aspartylproteasen mit einer Ausnahme - BACE1 - hemmt und nur diese beiden Aspartylproteasen im Knorpelgewebe bisher identifiziert wurden. So beschreiben auch Bo G. P. et al. (Clin. Rheumatol. 2009, 28(2):191-9) die wichtige Rolle von Cathepsin D bei pathologischen Veränderungen in Gelenken..

Der bekannteste Aspartylproteaseinhibitor ist Pepstatin, ein Peptid das ursprünglich aus einer Streptomyces-Kultur isoliert wurde. Pepstatin ist wirksam gegenüber Pepsin, Cathepsin und Renin. Viele Aspartylproteaseinhibitoren wurden deshalb dem Vorbild der Struktur des Pepstatins nachempfunden (U.S. Pat. No. 4,746,648; Umezawa, H, et al., J Antibiot (Tokyo) 23:259-62, 1970; Morishima, H., et al., J. Antibiot. (Tokyo) 23:263-5, 1970; Lin, Ty and Williams, H R., J. Biol. Chem. 254: 11875-83, 1979; Jupp, R A, et al., Biochem. J. 265:871-8, 1990; Agarwal, N S and Rich, D H, J. Med. Chem. 29:2519-24, 1986; Baldwin, E T, et al., Proc. Natl. Acad. Sci., USA 90: 6796-800, 1993; Francis, S E et al., EMBO J 13: 306-17, 1994).

Aspartylproteasen bzw. Cathepsin D werden häufig als Zielproteine für Wirkstoffe zur Behandlung von neurodegenerativen Erkrankungen, kognitiven Störungen, Demenz, Alzheimer, Krebs, Malaria, HIV-Infektion und Erkrankungen des Herzkreislauf- und Gefäßsystems beschrieben und Inhibitoren von Aspartylproteasen oder Cathepsin D werden zur Behandlung dieser Erkrankungen offenbart, wie beispielsweise in der WO 2009013293, EP 1987834, EP 1872780, EP 1867329, EP 1745778, EP 1745777, EP 1745776, WO 1999002153, WO 1999055687, US 6150416, WO 2003106405, WO 2005087751, WO 2005087215, WO 2005016876, US 2006281729, WO 2008119772, WO 2006074950, WO 2007077004, WO 2005049585, US 6251928 und US 6150416.

Peptidische Aspartylprotease-Inhibitoren, insbesondere Renin-Inhibitoren bzw. Modulatoren des Renin-Angiotensin-Systems sind beispielsweise aus der EP 77028, EP 161588, EP 337334 und EP 198271 bekannt.

Peptidische Cathepsin D Inhibitoren zur Behandlung von Entzündungserkrankungen, Arthritis, insbesondere rheumatoider Arthritis sind aus der US 3971736 bekannt. Peptidische Cathepsin D und Plasmepsin-Inhibtoren zur Behandlung von Malaria und Alzheimer und Verhinderung der Invasion und Metastasierung von Krebszellen sind beispielsweise aus der US 5849691 bekannt.

Die bekannten Cathepsin D Inhibitoren und die beiden Modellverbindungen Pepstatin und Ritonavir hemmen zwar wirksam die Cathepsin D Aktivität, jedoch weisen sie eine recht geringe Selektivität gegenüber anderen Aspartylproteasen auf. Die Rolle des Renin-Angiotensin Systems (RAS) bei der Regulation des Blutdrucks und des Flüssigkeits- und Elektrolythaushalts (Oparil, S. et al., N. Engl. J. Med. 1974; 291:381-401/446-57) und die Wirksamkeit von Renin- und Pepsininhibitoren bei Erkrankungen des Herzkreislauf- und Gefäßsystems ist hinreichend bekannt und so ist insbesondere bei oraler bzw. systemischer Applikation dieser wenig selektiven Cathepsin D Inhibitoren mit zahlreichen Nebenwirkungen zu rechnen und auch bei lokaler Applikation ist durch die Diffusion der Verbindungen mit systemischen Komplikationen zu rechnen. Daneben weisen gerade die peptidischen Verbindungen eine geringe Stabilität auf und sie kommen deshalb nicht für eine orale oder systemische Gabe in Frage.

Der Erfindung lag die Aufgabe zugrunde, neue Verbindungen mit wertvollen Eigenschaften aufzufinden, insbesondere solche, die zur Herstellung von Arzneimitteln verwendet werden können.

Aufgabe der vorliegende Erfindung war es insbesondere, neue Wirkstoffe und besonders bevorzugt neue Cathepsin D Inhibitoren zu finden, die zur Vorbeugung und Behandlung von Arthrose eingesetzt werden können und insbesondere eine hohe Selektivität für Cathepsin D gegenüber Renin und Pepsin aufweisen. Daneben sollten neue Cathepsin D Inhibitoren gefunden werden, die wenigstens bei lokaler bzw. intraartikulärer Applikation ausreichend stabil sind.

### Zusammenfassung der Erfindung

Überraschenderweise wurde gefunden, dass die erfindungsgemäßen Aminostatin-Derivate hochwirksam Cathepsin D hemmen und dabei eine hohe Selektivität für Cathepsin D gegenüber Renin und Pepsin aufweisen und damit bei deren Anwendung zur Behandlung von Arthrose mit geringen Nebenwirkungen zu rechnen ist. Daneben weisen die erfindungsgemäßen Verbindungen eine ausreichend gute Stabilität in Synovialflüssikeit auf, so dass sie sich zur intraartikulären Applikation und damit zur Behandlung von Arthrose eignen. Ebenfalls überraschenderweise hatte sich gezeigt, dass die erfindungsgemäßen Aminostatin-Derivate eine entzündungsbedingte thermische Hyperalgesie dosisabhängig reduzieren können. Die Erfindung wird in den Ansprüchen definiert. Die Veröffentlichung betrifft Aminostatin-Derivate der allgemeinen Formel I, 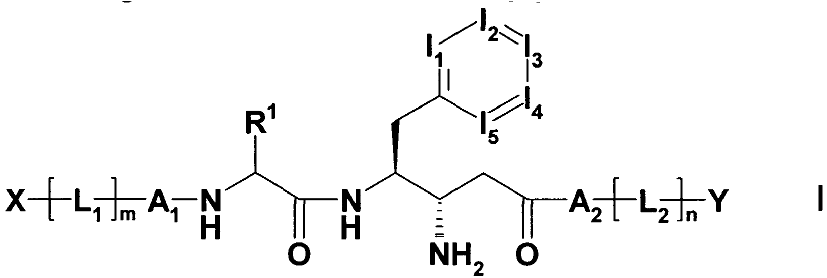 worin
- I₁, I₂, I₃, I₄, I₅: unabhängig voneinander N oder CR',
- T: ein unsubstituiertes oder ein-, zwei- drei- oder vierfach durch R substituiertes Phenyl oder Naphthyl, oder ein ein- oder zweikerniger gesättigter, ungesättigter oder aromatischer Heterozyklus mit 1 bis 4 N-, O- und/oder S-Atomen, der ein-, zwei- oder dreifach durch R, =S, =NR' und/oder =O substituiert sein kann,
- R¹: ein unsubstituiertes oder ein-, zwei- oder dreifach durch =S, =NR, =O, T, Hal, OH, NH₂, SO₂CH₃, SO₂NH₂, CN, CONH₂, NHCOCH₃, NHCONH₂, substituiertes, lineares oder verzweigtes Alkyl mit 1-10 C-Atomen, worin eine, zwei oder drei CH₂-Gruppen unabhängig voneinander durch O, S, SO, SO₂, NH, NCH₃, -OCO-, -NHCONH-, -NHCO-,-NRSO₂R'-, -COO-, -CONH-, -NCH₃CO-, -CONCH₃-,-C≡C-Gruppen und/oder -CH=CH-Gruppen, und/oder auch 1-20 H-Atome durch F und/oder Cl ersetzt sein können, oder unsubstituiertes oder ein-, zwei- oder dreifach durch =S, =NR, =O, T, OH, NH₂, SO₂CH₃, SO₂NH₂, CN, CONH₂, NHCOCH₃, und/oder NHCONH₂ substituiertes zyklisches Alkyl mit 3-7 C-Atomen, worin eine, zwei oder drei CH₂-Gruppen unabhängig voneinander durch O, S, SO, SO₂, NH, NCH₃, -OCO-, -NHCONH-, -NHCO-, - NRSO₂R' -COO-, -CONH-, -NCH₃CO-, -CONCH₃-, -C≡C-Gruppen und/oder durch -CH=CH-Gruppen und/oder auch 1-11 H-Atome durch F und/oder Cl ersetzt sein können,
- A₁: 0 bis 4 peptidartig miteinander verbundene Aminosäurereste, -CO-, -OCO-, -NRCO-, -SO₂- oder -NRSO₂-,
- A₂: 0 bis 4 peptidartig miteinander verbundene Aminosäurereste,
- L₁: eine Einfachbindung oder -CRR'-,
- L₂: eine Einfachbindung, -CRR'-, -NR-, -NRCR'R- oder-NRCRR'CRR'-,
- X, Y: H, T, unsubstituiertes oder ein-, zwei- oder dreifach durch =S, =NR, =O, R, T, OR, NRR', SOR, SO₂R, SO₂NRR', CN, COOR, CONRR', NRCOR', NRCONR'R", NRSO₂R' und/oder NRCOR' substituiertes, lineares oder verzweigtes Alkyl mit 1-10 C-Atomen, worin eine, zwei oder drei CH₂-Gruppen unabhängig voneinander durch O, S, SO, SO₂, NR, -OCO-, -NRCONR'-, -NRCO-, - NRSO₂R'-, -COO-, -CONR-,-C≡C-Gruppen und/oder durch -CH=CH-Gruppen und/oder auch 1-20 H-Atome durch F und/oder Cl ersetzt sein können, oder unsubstituiertes oder ein-, zwei- oder dreifach durch =S, =NR, =O, R, T, OR, NRR', SOR, SO₂R, SO₂NRR', CN, COOR, CONRR', NRCOR', NRCONR'R", NRSO₂R' und/oder NRCOR' substituiertes, lineares oder verzweigtes zyklisches Alkyl mit 3-7 C-Atomen, worin eine, zwei oder drei CH₂-Gruppen unabhängig voneinander durch O, S, SO, SO₂, NR, -OCO-, -NRCONR'-, -NRCO-, - NRSO₂R' -COO-,-CONR-, -C≡C-Gruppen und/oder durch -CH=CH-Gruppen und/oder auch 1-11 H-Atome durch F und/oder Cl ersetzt sein können,
- R,R': unabhängig voneinander H, unsubstituiertes oder ein-, zwei- oder dreifach durch =S, =NR, =O, Hal, OH, NH₂, SO₂CH₃, SO₂NH₂, CN, CONH₂, NHCOCH₃, und/oder NHCONH₂ substituiertes, lineares oder verzweigtes Alkyl mit 1-10 C-Atomen, worin eine, zwei oder drei CH₂-Gruppen unabhängig voneinander durch O, S, SO, SO₂, NH, NCH₃, -OCO-,-NHCONH-, -NHCO-, - NRSO₂R'-, -COO-, -CONH-,-NCH₃CO-, -CONCH₃-, -C≡C-Gruppen und/oder durch-CH=CH-Gruppen und/oder auch 1-20 H-Atome durch F und/oder Cl ersetzt sein können, oder unsubstituiertes oder ein-, zwei- oder dreifach durch =S, =NR, =O, OH, NH₂, SO₂CH₃, SO₂NH₂, CN, CONH₂, NHCOCH₃, und/oder NHCONH₂ substituiertes zyklisches Alkyl mit 3-7 C-Atomen, worin eine, zwei oder drei CH₂-Gruppen unabhängig voneinander durch O, S, SO, SO₂, NH, NCH₃, -OCO-,-NHCONH-, -NHCO-, - NRSO₂R'-, -COO-, -CONH-,-NCH₃CO-, -CONCH₃- und/oder durch -CH=CH-Gruppen und/oder auch 1-11 H-Atome durch F und/oder Cl ersetzt sein können,
- m: 0 - 4,
- n: 0 - 2, und
- Hal: F, Cl, Br oder I

ist, sowie deren physiologisch unbedenklichen Salze, Derivate, Solvate, Prodrugs und Stereoisomere, einschließlich deren Mischungen in allen Verhältnissen.

Ein bevorzugter Gegenstand der Veröffentlichung sind alle vorgenannten Verbindungen der Formel I, worin
- R¹: Ethyl, n-Propyl, iso-Propyl, Cyclopropyl, Butyl, iso-Butyl, sec-Butyl, Cyclobutyl, tert-Butyl, Phenyl, Benzyl, 2-Oxetanyl, 3-Oxetanyl, Tetrahydro-furan-3-yl, Tetrahydro-furan-2-yl, Cyclopentyl, Pentyl, Methylsulfanylmethyl, Ethylsulfanylmethyl, 2-Methylsulfanylethyl oder 1-Methylsulfanylethyl
ist, sowie deren physiologisch unbedenklichen Salze, Derivate, Solvate, Prodrugs und Stereoisomere, einschließlich deren Mischungen in allen Verhältnissen.

Ein weiterer bevorzugter Gegenstand der Veröffentlichung sind alle vorgenannten
Verbindungen der Formel I, worin
- A₁: 0 bis 4 peptidartig miteinander verbundene Aminosäurereste, ausgewählt aus der Gruppe bestehend aus Alanin, Glycin, Cyclopropylglycin, Cyclobutylglycin, Cyclopentylglycin, Cyclohexylglycin, 3-Oxetanylglycin, 3-Oxetanylglycin, Tetrahydro-furan-3-ylglycin, Tetrahydro-furan-2-ylglycin, Ethylsulfanylmethylglycin, 2-Methylsulfanylethylglycin, 1-Methylsulfanylethylglycin, Valin, Norvalin, Aminobuttersäure, Leucin, Isoleucin, Prolin, tert-Leucin, Norleucin, Methionin, Phenylalanin, Naphthylalanin, O-Methyl-Serin, O-Ethyl-Serin, -OCO-, -NRCO-,-SO₂- und -NRSO₂-
ist, sowie deren physiologisch unbedenklichen Salze, Derivate, Solvate, Prodrugs und Stereoisomere, einschließlich deren Mischungen in allen Verhältnissen.

Ein weiterer bevorzugter Gegenstand der Veröffentlichung sind alle vorgenannten
Verbindungen der Formel I, worin
- A₂: 0 bis 4 peptidartig miteinander verbundene Aminosäurereste, ausgewählt aus der Gruppe bestehend aus Alanin, Glycin, Cyclopropylglycin, Cyclobutylglycin, Cyclopentylglycin, Cyclohexylglycin, 3-Oxetanylglycin, 3-Oxetanylglycin, Tetrahydro-furan-3-ylglycin, Tetrahydro-furan-2-ylglycin, Ethylsulfanylmethylglycin, 2-Methylsulfanylethylglycin, 1-Methylsulfanylethylglycin, Valin, Norvalin, Aminobuttersäure, Leucin, Isoleucin, Prolin, tert-Leucin, Norleucin, Methionin, Phenylalanin, Naphthylalanin, O-Methyl-Serin und O-Ethyl-Serin
ist, sowie deren physiologisch unbedenklichen Salze, Derivate, Solvate, Prodrugs und Stereoisomere, einschließlich deren Mischungen in allen Verhältnissen. Die Erfindung betrifft Verbindungen der Formel I, worin
- R¹: Ethyl, n-Propyl, iso-Propyl, Cyclopropyl, Butyl, iso-Butyl, sec-Butyl, Cyclobutyl, tert-Butyl, Phenyl, Benzyl, 2-Oxetanyl, 3-Oxetanyl, Tetrahydro-furan-3-yl, Tetrahydro-furan-2-yl, Cyclopentyl, Pentyl, Methylsulfanylmethyl, Ethylsulfanylmethyl, 2-Methylsulfanylethyl oder 1-Methylsulfanylethyl,
- A₁: 0 bis 4 peptidartig miteinander verbundene Aminosäurereste, ausgewählt aus der Gruppe bestehend aus Alanin, Glycin, Cyclopropylglycin, Cyclobutylglycin, Cyclopentylglycin, Cyclohexylglycin, 3-Oxetanylglycin, 3-Oxetanylglycin, Tetrahydro-furan-3-ylglycin, Tetrahydro-furan-2-ylglycin, Ethylsulfanylmethylglycin, 2-Methylsulfanylethylglycin, 1-Methylsulfanylethylglycin, Valin, Norvalin, Aminobuttersäure, Leucin, Isoleucin, Prolin, tert-Leucin, Norleucin, Methionin, Phenylalanin, Naphthylalanin, O-Methyl-Serin, O-Ethyl-Serin, -OCO-, -NRCO-, -SO₂- und -NRSO₂- und
- A₂: 0 bis 4 peptidartig miteinander verbundene Aminosäurereste, ausgewählt aus der Gruppe bestehend aus Alanin, Glycin, Cyclopropylglycin, Cyclobutylglycin, Cyclopentylglycin, Cyclohexylglycin, 3-Oxetanylglycin, 3-Oxetanylglycin, Tetrahydro-furan-3-ylglycin, Tetrahydro-furan-2-ylglycin, Ethylsulfanylmethylglycin, 2-Methylsulfanylethylglycin, 1-Methylsulfanylethylglycin, Valin, Norvalin, Aminobuttersäure, Leucin, Isoleucin, Prolin, tert-Leucin, Norleucin, Methionin, Phenylalanin, Naphthylalanin, O-Methyl-Serin und O-Ethyl-Serin
ist, sowie deren physiologisch unbedenklichen Salze, Solvate, und Stereoisomere, einschließlich deren Mischungen in allen Verhältnissen. Veröffentlicht sind auch alle vorgenannten Verbindungen der Formel 1, worin
A₁ zwei peptidartig miteinander verbundene Aminosäurereste ist, sowie deren physiologisch unbedenklichen Salze, Derivate, Solvate, Prodrugs und Stereoisomere, einschließlich deren Mischungen in allen Verhältnissen. Veröffentlicht sind auch alle vorgenannten Verbindungen der Formel 1, worin
- A₁: zwei peptidartig miteinander verbundene Aminosäurereste ausgewählt aus der Gruppe bestehend aus Valin und Leucin
ist, sowie deren physiologisch unbedenklichen Salze, Derivate, Solvate, Prodrugs und Stereoisomere, einschließlich deren Mischungen in allen Verhältnissen. Veröffentlicht sind auch alle vorgenannten Verbindungen der Formel 1, worin
- A₁: 0 bis 4 peptidartig miteinander verbundene Aminosäurereste, die in ihrer S-Konfiguration vorliegen,
ist, sowie deren physiologisch unbedenklichen Salze, Derivate, Solvate, Prodrugs und Stereoisomere, einschließlich deren Mischungen in allen Verhältnissen. Veröffentlicht sind auch alle vorgenannten Verbindungen der Formel 1, worin
- X: T
ist, sowie deren physiologisch unbedenklichen Salze, Derivate, Solvate, Prodrugs und Stereoisomere, einschließlich deren Mischungen in allen Verhältnissen. Veröffentlicht sind auch alle vorgenannten Verbindungen der Formel 1, worin
- A₁: 0 und
- X: T
ist, sowie deren physiologisch unbedenklichen Salze, Derivate, Solvate, Prodrugs und Stereoisomere, einschließlich deren Mischungen in allen Verhältnissen. Veröffentlicht sind auch alle vorgenannten Verbindungen der Formel 1, worin
- A₂: 0 bis 2 peptidartig miteinander verbundene Aminosäurereste, die in ihrer S-Konfiguration vorliegen, und
- X: T
ist, sowie deren physiologisch unbedenklichen Salze, Derivate, Solvate, Prodrugs und Stereoisomere, einschließlich deren Mischungen in allen Verhältnissen.

Ein besonders bevorzugter Gegenstand der vorwiegenden Veröffentlichung sind alle vorgenannten Verbindungen der Formel 1, worin
- R¹: Ethyl, n-Propyl, iso-Propyl, Cyclopropyl, Butyl, iso-Butyl, sec-Butyl, Cyclobutyl oder tert-Butyl,
ist, sowie deren physiologisch unbedenklichen Salze, Derivate, Solvate, Prodrugs und Stereoisomere, einschließlich deren Mischungen in allen Verhältnissen.

Ein ganz besonders bevorzugter Gegenstand der vorliegenden Veröffentlichung sind alle vorgenannten Verbindungen der Formel 1, worin
- R¹: Ethyl oder iso-Propyl
ist, sowie deren physiologisch unbedenklichen Salze, Derivate, Solvate, Prodrugs und Stereoisomere, einschließlich deren Mischungen in allen Verhältnissen.

Ganz besonders bevorzugte sind die nachfolgenden Verbindungen der Formel I:
a.) (S)-2-((2S,3S)-2-{(3S,4S)-3-Amino-4-[(S)-2-((S)-2-tert-butoxycarbonylamino-4-methyl-pentanoylamino)-butyrylamino]-5-phenyl-pentanoylamino}-3-methyl-pentanoylamino)-3-methyl-buttersäuremethylester
b.) (S)-2-((2S,3S)-2-{(3S,4S)-3-Amino-4-[(S)-2-((S)-2-amino-4-methyl-pentanoylamino)butyrylamino]-5-phenyl-pentanoylamino}-3-methyl-pentanoylamino)-3-methyl-buttersäuremethylester
c.) (S)-2-((2S,3S)-2-{(3S,4S)-3-Amino-4-[(S)-4-methyl-2-(3-methyl-butyrylamino)-pentanoylamino]-5-phenyl-pentanoylamino}-3-methyl-pentanoylamino)-3-methyl-buttersäuremethylester
d.) (S)-2-[(2S,3S)-2-((3S,4S)-3-Amino-4-{(S)-2-[(S)-4-methyl-2-(3-methyl-butyrylamino)-pentanoylamino]-1-oxo-butylamino}-5-phenyl-pentanoylamino)-3-methyl-pentanoylamino]-3-methyl-buttersäuremethylester
e.) (3S,4S)-3-Amino-4-{(S)-2-[(S)-4-methyl-2-(3-methyl-butyrylamino)-pentanoylamino]-butyrylamino}-5-phenyl-pentansäure-(2-benzo[1,3]dioxol-5-yl-ethyl)-amid
f.) (3S,4S)-3-Amino-4-{(S)-2-[(S)-4-methyl-2-(3-methyl-butyrylamino)-pentanoylamino]-butyrylamino}-5-phenyl-pentansäure-[2-(3,4-dichloro-phenyl)-ethyl]-amid
g.) (S)-2-[(2S,3S)-2-((3S,4S)-3-Amino-4-{(S)-3-methyl-2-[2-(3-trifluoromethoxy-phenyl)-acetylamino]-butyrylamino}-5-phenyl-pentanoylamino)-3-methyl-pentanoylamino]-3-methyl-buttersäuremethylester
h.) (S)-2-[(2S,3S)-2-((3S,4S)-3-Amino-4-{(S)-3-methyl-2-[2-(naphthalen-1-yloxy)-acetylamino]-butyrylamino}-5-phenyl-pentanoylamino)-3-methyl-pentanoylamino]-3-methyl-buttersäuremethylester
i.) (S)-2-[(2S,3S)-2-((3S,4S)-3-Amino-4-{(S)-2-[2-(3-methanesulfonylamino-phenyl)-acetylamino]-3-methyl-butyrylamino}-5-phenyl-pentanoylamino)-3-methyl-pentanoylamino]-3-methyl-buttersäuremethylester
j.) (S)-2-[(2S,3S)-2-((3S,4S)-3-Amino-4-{(S)-3-methyl-2-[(S)-4-methyl-2-(3-methyl-butyrylamino)-pentanoylamino]-1-oxo-butylamino}-5-phenyl-pentanoylamino)-3-methyl-pentanoylamino]-3-methyl-buttersäuremethylester
k.) (S)-2-[(2S,3S)-2-((3S,4S)-3-Amino-4-{(S)-2-[(S)-4-methyl-2-(3-methyl-butyrylamino)-pentanoylamino]-3-phenyl-propionylamino}-5-phenyl-pentanoylamino)-3-methyl-pentanoylamino]-3-methyl-buttersäuremethylester
l.) 1-[(2S,3S)-2-((S)-3-(S)-Amino-4-{(S)-3-methyl-2-[(S)-4-methyl-2-(3-methyl-butyrylamino)-pentanoylamino]-butyrylamino}-5-phenyl-pentanoylamino)-3-methyl-pentanoylamino]-cyclopropan-carbonsäureethylester
m.) (2S,3S)-2-[(S)-2-((S)-3-(S)-Amino-4-{(S)-3-methyl-2-[(S)-4-methyl-2-(3-methyl-butyrylamino)-pentanoylamino]-butyrylamino}-5-phenyl-pentanoylamino)-3-methyl-1-(S)-oxo-pentylamino]-3-methyl-pentansäuremethylester
n.) (S)-2-[(2S,3S)-2-((S)-3-(S)-Amino-4-{(S)-3-methyl-2-[(S)-4-methyl-2-(3-methyl-butyrylamino)-pentanoylamino]-butyrylamino}-5-phenyl-pentanoylamino)-3-methyl-pentanoylamino]-4-methyl-pentansäuremethylester
o.) (S)-2-[(S)-2-((3S,4S)-3-Amino-4-{(S)-3-methyl-2-[(S)-4-methyl-2-(3-methyl-butyrylamino)-pentanoylamino]-butyrylamino}-5-phenyl-pentanoylamino)-3-phenyl-propionylamino]-3-methyl-buttersäuremethylester
p.) (S)-2-((2S,3S)-2-{(3S,4S)-3-Amino-4-[(S)-2-((S)-2-tert-butoxycarbonylamino-4-methyl-pentanoylamino)-3-methyl-butyrylamino]-5-phenyl-pentanoylamino}-3-methyl-pentanoylamino)-3-methyl-buttersäuremethylester
q.) (S)-2-((2S,3S)-2-{(3S,4S)-3-Amino-4-[(S)-3-methyl-2-(3-phenyl-propionylamino)-butyrylamino]-5-phenyl-pentanoylamino}-3-methyl-pentanoylamino)-3-methyl-buttersäuremethylester
r.) (S)-2-[(2S,3S)-2-((3S,4S)-3-Amino-4-{(S)-4-methyl-2-[(S)-4-methyl-2-(3-methyl-butyrylamino)-pentanoylamino]-pentanoylamino}-5-phenyl-pentanoylamino)-3-methyl-pentanoylamino]-3-methyl-buttersäuremethylester
s.) (S)-2-[(2S,3S)-2-((3S,4S)-3-Amino-4-{(S)-3-methyl-2-[(S)-2-(3-methyl-butyrylamino)-3-phenyl-propionylamino]-butyrylamino}-5-phenyl-pentanoylamino)-3-methyl-pentanoylamino]-3-methyl-buttersäuremethylester
t.) (S)-2-((2S,3S)-2-{(3S,4S)-3-Amino-4-[(S)-2-((S)-2-tert-butoxycarbonylamino-3-phenyl-propionylamino)-3-methyl-butyrylamino]-5-phenyl-pentanoylamino}-3-methyl-pentanoylamino)-3-methyl-buttersäuremethylester
u.) (S)-2-((2S,3S)-2-{(3S,4S)-3-Amino-4-[(S)-2-((S)-2-amino-3-phenyl-propionylamino)-3-methyl-butyrylamino]-5-phenyl-pentanoylamino}-3-methyl-pentanoylamino)-3-methyl-buttersäuremethylester
v.) (S)-2-[(2S,3S)-2-((3S,4S)-3-Amino-4-{(S)-2-[2-(2,4-dichloro-phenoxy)-acetylamino]-3-methyl-butyrylamino}-5-phenyl-pentanoylamino)-3-methyl-pentanoylamino]-3-methyl-buttersäuremethylester
w.) (S)-2-[(2S,3S)-2-((3S,4S)-3-Amino-4-{(S)-3-methyl-2-[(S)-4-methyl-2-(3-methyl-butyrylamino)-pentanoylamino]-butyrylamino}-5-phenyl-pentanoylamino)-3-methyl-pentanoylamino]-3-methyl-buttersäure
x.) (S)-3-Amino-4-{(S)-(S)-3-methyl-2-[(S)-4-methyl-2-(3-methyl-butyrylamino)-pentanoylamino]-butyrylamino}-5-phenyl-pentansäure-((1S,2S)-2-methyl-1-phenethylcarbamoyl-butyl)-amid
y.) (S)-3-Amino-4-{(S)-(S)-3-methyl-2-[(S)-4-methyl-2-(3-methyl-butyrylamino)-pentanoylamino]-butyrylamino}-5-phenyl-pentansäure-[(1S,2S)-2-methyl-1-(pyridin-3-ylcarbamoyl)-butyl]-amid
z.) (S)-2-((2S,3S)-2-{(3S,4S)-3-Amino-4-[(R)-2-((S)-2-amino-3-phenyl-propionylamino)-3-methyl-butyrylamino]-5-phenyl-pentanoylamino}-3-methyl-pentanoylamino)-3-methyl-buttersäuremethylester
aa.) (S)-3-Amino-4-{(S)-(S)-3-methyl-2-[(S)-4-methyl-2-(3-methyl-butyrylamino)-pentanoylamino]-butyrylamino}-5-phenyl-pentansäure-[(1S,2S)-2-methyl-1-(1-methyl-1H-pyrazol-3-ylcarbamoyl)-butyl]-amid
bb.) (S)-2-[(S)-2-((3S,4S)-3-Amino-4-{(S)-3-methyl-2-[(S)-4-methyl-2-(3-methyl-butyrylamino)-pentanoylamino]-butyrylamino}-5-phenyl-pentanoylamino)-3-naphthalen-1-yl-propionylamino]-3-methyl-buttersäuremethylester
cc.) (S)-3-Amino-4-{(S)-(S)-3-methyl-2-[(S)-4-methyl-2-(3-methyl-butyrylamino)-pentanoylamino]-butyrylamino}-5-phenyl-pentansäure-((1S,2S)-1-benzylcarbamoyl-2-methyl-butyl)-amid
dd.) (S)-3-(S)-Amino-4-{(S)-3-methyl-2-[(S)-4-methyl-2-(3-methyl-butyrylamino)-pentanoylamino]-butyrylamino}-5-phenyl-pentansäure-[(1S,2S)-1-(cyclopropylmethyl-carbamoyl)-2-methyl-butyl]-amid
ee.) (S)-2-[(2S,3S)-2-((3S,4S)-3-Amino-4-{(S)-3,3-dimethyl-2-[(S)-4-methyl-2-(3-methyl-butyrylamino)-pentanoylamino]-butyrylamino}-5-phenyl-pentanoylamino)-3-methyl-pentanoylamino]-3-methyl-buttersäuremethylester
ff.) (S)-2-[(2S,3S)-2-((3S,4S)-3-Amino-4-{(S)-3-methyl-2-[2-(3-trifluoromethoxy-phenyl)-acetylamino]-butyrylamino}-5-phenyl-pentanoylamino)-3-methyl-pentanoylamino]-3-methyl-butyric acid
gg.) (S)-2-[(2S,3S)-2-((3S,4S)-3-Amino-4-{(S)-3-methyl-2-[(S)-2-(3-methyl-butyrylamino)-3-phenyl-propionylamino]-1-oxo-butylamino}-5-phenyl-pentanoylamino)-3-methyl-pentanoylamino]-3-methyl-buttersäure
hh.) (S)-3-(S)-Amino-4-{(S)-3-methyl-2-[(S)-4-methyl-2-(3-methyl-butyrylamino)-pentanoylamino]-butyrylamino}-5-phenyl-pentanoic acid [(1S,2S)-1-((S)-1-hydroxymethyl-2-methyl-propylcarbamoyl)-2-methylbutyl]-amid
ii.) (3S,4S)-3-Amino-4-{(S)-3-methyl-2-[2-(3-trifluoromethoxy-phenyl)-acetylamino]-butyrylamino}-5-phenyl-pentansäure-[(S)-1-(3-methyl-butylcarbamoyl)-ethyl]-amid
jj.) (S)-3-(S)-Amino-4-{(S)-3-methyl-2-[2-(3-trifluoromethoxy-phenyl)-acetylamino]-butyrylamino}-5-phenyl-pentansäure-[(1S,2S)-2-methyl-1-(3-methyl-butylcarbamoyl)-butyl]-amid
kk.) (S)-2-[(2S,3S)-2-((3S,4S)-3-Amino-4-{(1S,4S)-3-methyl-2-[2-(3-trifluoromethoxy-phenyl)-acetylamino]-pentanoylamino}-5-phenyl-pentanoylamino)-3-methyl-pentanoylamino]-3-methyl-buttersäure
ll.) (3S,4S)-3-Amino-4-{(S)-3-methyl-2-[(S)-4-methyl-2-(3-methyl-butyrylamino)-pentanoylamino]-butyrylamino}-5-phenyl-pentansäure-(2,6-diethyl-phenyl)-amid
mm.) (S)-3-(S)-Amino-4-{(S)-3-methyl-2-[(S)-4-methyl-2-(3-methyl-butyrylamino)-pentanoylamino]-butyrylamino}-5-phenyl-pentansäure-[(1S,2S)-1-(1-ethyl-propylcarbamoyl)-2-methyl-butyl]-amid
nn.) (3S,4S)-3-Amino-4-[(S)-3-methyl-2-(4-phenyl-butyrylamino)-butyrylamino]-5-phenyl-pentanoic acid (2,6-diethyl-phenyl)-amid
oo.) 3-(S)-Amino-4-{(S)-(2S,3S)-3-methyl-2-[2-(3-trifluoromethoxy-phenyl)-acetylamino]-pentanoylamino}-5-phenyl-pentansäure-{(2S,3S)-1-[1-((S)-hydroxymethyl)-2-methyl-propylcarbamoyl]-2-methyl-butyl}-amid
pp.) (S)-3-(S)-Amino-4-{(S)-3-methyl-2-[(S)-2-(3-methyl-butyrylamino)-3-phenyl-propionylamino]-butyrylamino}-5-phenyl-pentansäure-[(1S,2S)-1-((S)-1-hydroxymethyl-2-methyl-propylcarbamoyl)-2-methylbutyl]-amid
qq.) (3S,4S)-3-Amino-4-{(S)-3-methyl-2-[(S)-4-methyl-2-(3-methyl-butyrylamino)-pentanoylamino]-butyrylamino}-5-phenyl-pentansäure-[(S)-2-methyl-1-(pyridin-3-yloxymethyl)-propyl]-amid
rr.) (3S,4S)-3-Amino-4-{(S)-3-methyl-2-[(S)-4-methyl-2-(3-methyl-butyrylamino)-pentanoylamino]-butyrylamino}-5-phenyl-pentansäure-[(S)-1-(2,4-difluoro-phenoxymethyl)-2-methyl-propyl]-amid
ss.) (3S,4S)-3-Amino-4-{(S)-3-methyl-2-[(S)-4-methyl-2-(3-methyl-butyrylamino)-pentanoylamino]-butyrylamino}-5-phenyl-pentansäure-(S)-1-(2,4-difluoro-phenoxymethyl)-2-methyl-propyl]-amid
tt.) (3S,4S)-3-Amino-4-{(S)-3-methyl-2-[(S)-4-methyl-2-(3-methyl-butyrylamino)-pentanoylamino]-butyrylamino}-5-phenyl-pentansäure(1-benzyl-2-phenyl-ethyl)-amid
uu.) [(S)-1-((S)-1-{(1S,2S)-2-Amino-1-benzyl-3-[(S)-1-((S)-1-hydroxymethyl-2-methyl-propylcarbamoyl)-2-naphthalen-1-yl-ethylcarbamoyl]-propylcarbamoyl}-2-methyl-propylcarbamoyl)-2-phenyl-ethyl]-carbamoylsäure tert-butyl ester
w.) (S)-3-(S)-Amino-4-{(S)-3-methyl-2-[(S)-4-methyl-2-(3-phenyl-propionylamino)-pentanoylamino]-1-oxo-butylamino}-5-phenyl-pentansäure [(1S,2S)-1-(1-ethyl-propylcarbamoyl)-2-methyl-butyl]-amid
ww.) (S)-3-(S)-Amino-4-{(S)-2-[(S)-2-(3,3-dimethyl-butyrylamino)-4-methyl-pentanoylamino]-3-methyl-1-oxo-butylamino}-5-phenyl-pentansäure [(1S,2S)-1-(1-ethyl-propylcarbamoyl)-2-methyl-butyl]-amid
xx.) (S)-2-[(2S,3S)-2-((S)-3-(S)-Amino-4-{(S)-3-methyl-2-[(S)-4-methyl-2-(3-phenyl-propionylamino)-pentanoylamino]-1-oxo-butylamino}-5-phenyl-pentanoylamino)-3-methyl-pentanoylamino]-3-methyl-buttersäure
yy.) (S)-2-[(2S,3S)-2-((S)-3-(S)-Amino-4-{(S)-3-methyl-2-[(S)-4-methyl-2-(4-trifluoromethoxy-benzoylamino)-pentanoylamino]-1-oxo-butylamino}-5-phenyl-pentanoylamino)-3-methyl-pentanoylamino]-3-methyl-buttersäure
zz.) (S)-2-((2S,3S)-2-{(S)-3-(S)-Amino-4-[(S)-3-methyl-2-((S)-4-methyl-2-phenylacetylamino-pentanoylamino)-1-oxo-butylamino]-5-phenyl-pentanoylamino}-3-methyl-pentanoylamino)-3-methyl-buttersäure
aaa.) N-[(S)-1-(1-{(S)-(S)-2-(S)-Amino-1-benzyl-3-[(1S,2S)-1-(1-ethyl-propylcarbamoyl)-2-methyl-butylcarbamoyl]-propylcarbamoyl}-2-methyl-propylcarbamoyl)-3-methyl-butyl]-4-trifluoromethoxy-benzamid
bbb). (S)-2-[(2S,3S)-2-((S)-3-(S)-Amino-4-{(S)-2-[(S)-2-(3,3-demethylbutyrylamino)-4-methyl-pentanoylamino]-3-methyl-1-oxo-butylamino}-5-phenyl-pentanoylamino)-3-methyl-pentanoylamino]-3-methyl-buttersäure
ccc.) (S)-2-((2S,3S)-2-{(S)-3-(S)-Amino-4-[(S)-3-methyl-2-((S)-4-methyl-2-pentanoylamino-pentanoylamino)-1-oxo-butylamino]-5-phenyl-pentanoylamino}-3-methyl-pentanoylamino)-3-methyl-buttersäure
ddd.) (S)-3-(S)-Amino-4-[(S)-3-methyl-2-((S)-4-methyl-2-phenylacetylamino-pentanoylamino)-1-oxo-butylamino]-5-phenyl-pentansäure[(1S,2S)-1-(1-ethyl-propylcarbamoyl)-2-methyl-butyl]-amid
eee.) (S)-3-(S)-Amino-4-[(S)-3-methyl-2-((S)-4-methyl-2-pentanoylamino-pentanoylamino)-1-oxo-butylamino]-5-phenyl-pentansäure [(1S,2S)-1-(1-ethyl-propylcarbamoyl)-2-methyl-butyl]-amid sowie deren physiologisch unbedenklichen Salze, Solvate, und Stereoisomere, einschließlich deren Mischungen in allen Verhältnissen.

Die vor- und nachstehend aufgeführten Aminosäurereste stehen für die Reste folgender Aminosäuren:
- Natürliche Aminosäuren - Alanin, Arginin, Asparagin, Asparaginsäure, Cystein, Glutamin, Glutaminsäure, Glycin, Histidin, Isoleucin, Leucin, Lysin, Methionin, Ornithin, Phenylalanin, Prolin, Serin, Threonin, Tryptophan, Tyrosin, Valin
- Nicht-natürliche Aminosäuren wie beispielsweise Cyclopropylglycin, Cyclobutylglycin, Cyclopentylglycin, Cyclohexylglycin, 3-Oxetanylglycin, 3-Oxetanylglycin, Tetrahydro-furan-3-ylglycin, Tetrahydro-furan-2-ylglycin, Ethylsulfanylmethylglycin, 2-Methylsulfanylethylglycin, 1-Methylsulfanylethylglycin, Norvalin, Aminobuttersäure, tert-Leucin, Norleucin, Naphthylalanin, O-Methyl-Serin, O-Ethyl-Serin und dergleichen

Sofern die vorstehend genannten Aminosäuren in mehreren enantiomeren Formen auftreten können, so sind vor- und nachstehend alle diese Formen und auch ihre Gemische (z.B. DL-Formen) eingeschlossen.

**Ferner bedeutet nachstehend:**

| | |
|---|---|
| Boc | ter.-Butoxycarbonyl |
| CBZ | Benzyloxycarbonyl |
| DNP | 2,4-Dinitrophenyl |
| FMOC | 9-Fluorenylmethoxycarbonyl |
| imi-DNP | 2,4-Dinitrophenyl in 1-Stellung des Imidazolrings |
| OMe | Methylester |
| POA | Phenoxyacetyl |
| DCCI | Dicyclohexylcarbodiimid |
| HOBt | 1-Hydroxybenzotriazol |

Hal bedeutet Fluor, Chlor, Brom oder Iod, insbesondere Fluor oder Chlor.

Alkyl ist eine unverzweigte (lineare), verzweigte oder zyklische Kohlenwasserstoffkette und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, lineares oder verzweigtes Heptyl, Octyl, Nonyl oder Decyl bedeutet, weiter bevorzugt z.B. Trifluormethyl

Zyklisches Alkyl oder Cycloalkyl bedeutet vorzugsweise (Ist A cyclisch so bedeutet es) vorzugsweise Cyclopropyl, Cyclobutyl, Cylopentyl, Cyclohexyl oder Cycloheptyl.

Ein- oder zweikerniger gesättigter, ungesättigter oder aromatischer Heterozyklus bedeutet vorzugsweise unsubstituiertes oder ein-, zwei- oder dreifach substituiertes 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.

Die heterozyklischen Reste können auch teilweise oder vollständig hydriert sein und bedeuten z.B. auch 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder-4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder - 4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydro-benzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.
Heterozyklus bedeutet ferner z.B. 2-Oxo-piperidin-1-yl, 2-Oxo-pyrrolidin-1-yl, 2-Oxo-1*H*-pyridin-1-yl, 3-Oxo-morpholin-4-yl, 4-Oxo-1*H*-pyridin-1-yl, 2,6-Dioxo-piperidin1-yl, 2-Oxo-piperazin-1-yl, 2,6-Dioxo-piperazin-1-yl, 2,5-Dioxo-pyrrolidin-1-yl, 2-Oxo-1,3-oxazolidin-3-yl, 3-Oxo-2*H*-pyridazin-2-yl, 2-Caprolactam-1-yl (= 2-Oxo-azepan-1-yl), 2-Hydroxy-6-oxo-piperazin-1-yl, 2-Methoxy-6-oxo-piperazin-1-yl oder 2-Aza-bicyclo[2.2.2]-octan-3-on-2-yl.

Erfindungsgemäß sind auch alle physiologisch unbedenklichen Salze, Derivate, Solvate und Stereoisomere dieser Verbindungen, einschließlich deren Mischungen in allen Verhältnissen.

Gegenstand der Erfindung sind auch die optisch aktiven Formen (Stereoisomeren), die Enantiomeren, die Racemate, die Diastereomeren sowie Hydrate und Solvate dieser Verbindungen.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen. Da sich die pharmazeutische Wirksamkeit der Racemate bzw. der Stereoisomere der erfindungsgemäßen Verbindungen unterscheiden kann, kann es wünschenswert sein, die Enantiomere zu verwenden. In diesem Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Unter pharmazeutisch oder physiologisch unbedenklichen Derivaten versteht man z.B. Salze der erfindungsgemäßen Verbindungen, als auch sogenannte Prodrug-Verbindungen. Unter Prodrug-Verbindungen versteht man mit z.B. Alkyl- oder Acylgruppen (siehe auch nachstehende Amino- und Hydroxyschutzgruppen), Zuckern oder Oligopeptiden abgewandelte Verbindungen der Formel I, die im Organismus rasch zu den wirksamen erfindungsgemäßen Verbindungen gespalten oder freigesetzt werden. Hierzu gehören auch bioabbaubare Polymerderivate der erfindungsgemäßen Verbindungen, wie dies z.B. in Int. J. Pharm. 115 (1995), 61-67 beschrieben ist.

Als Säureadditionssalze kommen anorganische oder organische Salze aller physiologisch oder pharmakologisch unbedenklichen Säuren in Frage, beispielsweise Halogenide, insbesondere Hydrochloride oder Hydrobromide, Lactate, Sulfate, Citrate, Tartrate, Maleate, Fumarate, Oxalate, Acetate, Phosphate, Methylsulfonate oder p-Toluolsulfonate.

Unter Solvaten der Verbindungen der Formel I werden Anlagerungen von inerten Lösungsmittelmolekülen an die Verbindungen der Formel I verstanden, die sich aufgrund ihrer gegenseitigen Anziehungskraft ausbilden. Solvate sind beispielsweise Hydrate, wie Monohydrate oder Dihydrate oder Alkoholate, d.h. Additionsverbindungen mit Alkoholen wie beispielsweise mit Methanol oder Ethanol.

Gegenstand der Erfindung sind auch Mischungen der erfindungsgemäßen Verbindungen der Formel I, z.B. Gemische zweier Diastereomere z.B. im Verhältnis 1:1, 1:2, 1:3, 1:4, 1:5, 1:10, 1:100 oder 1:1000. Besonders bevorzugt handelt es sich um Mischungen zweier stereoisomerer Verbindungen.

Gegenstand der Erfindung ist außerdem ein Verfahren zur Herstellung der Verbindungen der Formel I, dadurch gekennzeichnet, dass man
a) eine Verbindung der Formel II, worin I₁, I₂, I₃, I₄ und I₅ die in Anspruch 1 angegebenen Bedeutungen haben und Q und Q' zueinander orthogonale Aminoschutzgruppen bedeuten, mit einer Verbindung der Formel III, worin Y, L₂, n und A₂ die in Anspruch 1 angegebenen Bedeutungen haben, unter Ausbildung einer Peptidbindung zwischen der Carboxylgruppe der Verbindung der Formel II und der Aminogruppe der Verbindung der Formel III zu einer Verbindung der Formel IVa umsetzt und die Verbindung der Formel IVa durch Abspaltung der Schutzgruppe Q zu einer Verbindung der Formel IV umsetzt, 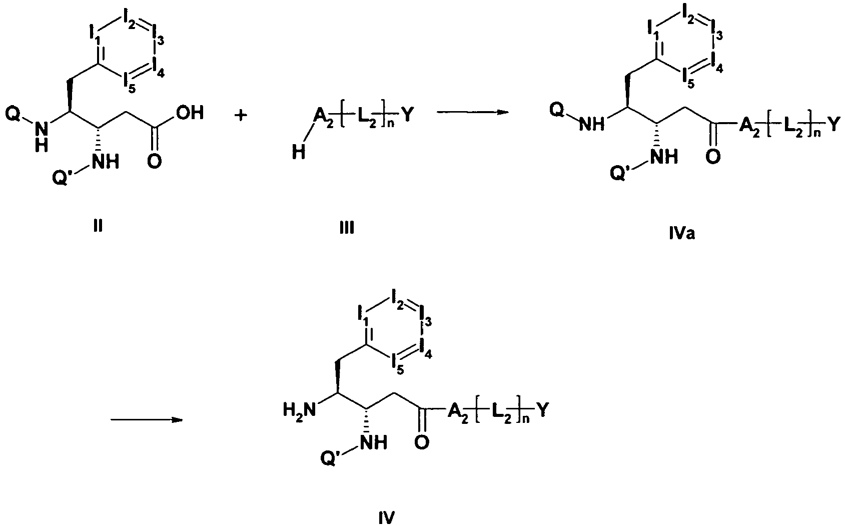 und eine Verbindung der Formel V, worin X, L₁, m und A₁ die in Anspruch 1 angegebenen Bedeutungen haben, mit einer Verbindung der Formel VI, worin R¹ die in Anspruch 1 angegebene Bedeutung hat und W eine Säureschutzgruppe bedeutet, unter Ausbildung einer Peptidbindung zwischen der Carboxylgruppe der Verbindung der Formel V und der Aminogruppe der Verbindung der Formel VI zu einer Verbindung der Formel VIIa umsetzt und die Verbindung der Formel VIIa durch Abspaltung der Schutzgruppe W zu einer Verbindung der Formel VII umsetzt, 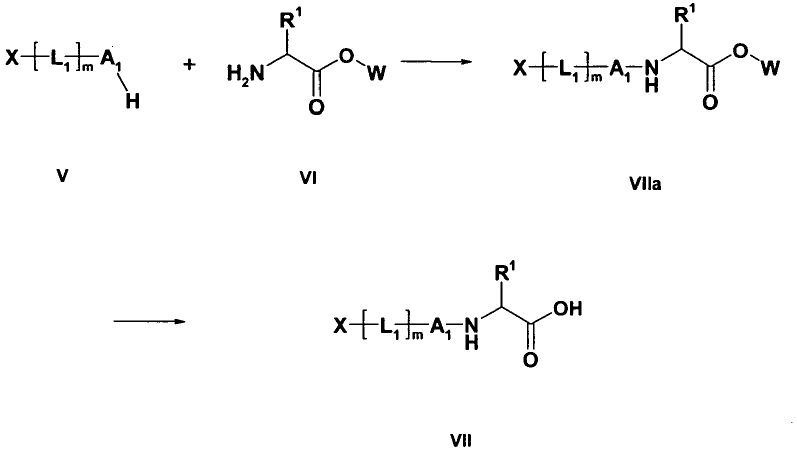 und eine Verbindung der Formel VII, worin X, L₁, m, A₁ und R¹ die in Anspruch 1 angegebenen Bedeutungen haben, mit einer Verbindung der Formel IV, worin I₁, I₂, I₃, I₄, I₅, Y, L₂, n und A₂ die in Anspruch 1 angegebenen Bedeutungen haben und Q' eine Aminoschutzgruppe ist, unter Ausbildung einer Peptidbindung zwischen der Carboxylgruppe der Verbindung der Formel VII und der Aminogruppe der Verbindung der Formel IV zu einer Verbindung der Formel la umsetzt und die Verbindung der Formel la durch Abspaltung der Schutzgruppe Q' zu einer Verbindung der Formel I umsetzt, 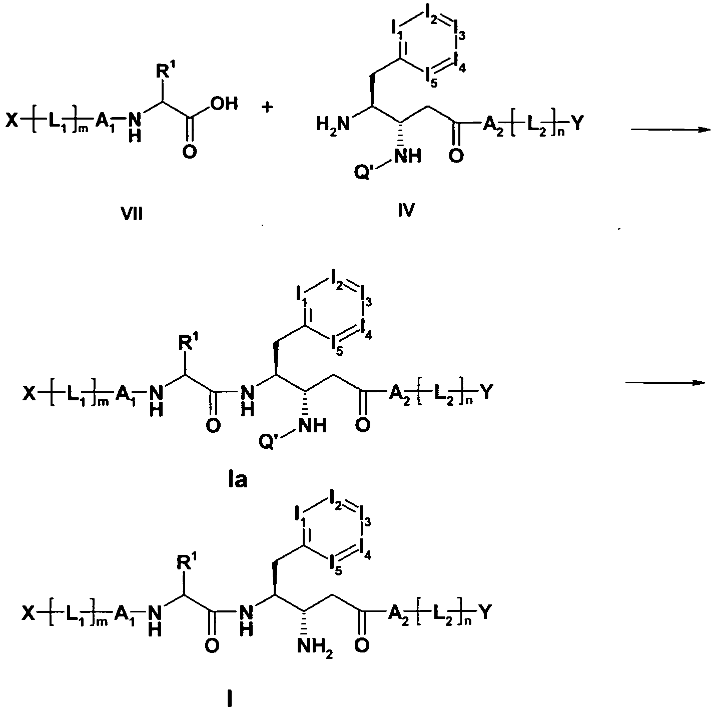
b) die Base einer Verbindung der Formel I durch Behandlung mit einer Säure in eines ihrer Salze überführt oder
c) eine Säure einer Verbindung der Formel I durch Behandlung mit einer Base in eines ihrer Salze überführt.

Die Peptid-Kupplungen zu Vlla und IVa werden beispielsweise mithilfe von DAPECI als Kupplungsreagenz durchgeführt, die Kupplung zu la wird beispielsweise mithilfe einer HATU-Kupplung durchgeführt. Weitere Kupplungsmethoden zur Ausbildung einer Peptidbindung sind dem Fachmann bekannt. Die Aminoschutzgruppe Q an den Verbindungen der Formel IVa, beispielsweise eine BOC-Schutzgruppe, wird von der Verbindung der Formel IVa beispielsweise durch HCL/Dioxan oder TFA/DCM unter geeigneten Bedingungen abgespalten. Ebenso wird die Aminoschutzgruppe Q' an den Verbindungen der Formel Ia, beispielsweise eine Z-Schutzgruppe von der Verbindung der Formel Ia durch Hydrogenolyse unter geeigneten Bedingungen z.B. in Gegenwart von Pd/C abgespalten. Weitere Schutzgruppen, beispielsweise an den Carboxylgruppen, z.B. Carbonsäurebenzylester in anderen Molekülteilen werden beispielsweise durch Hydrogenolyse in Gegenwart von Pd/c abgespalten und liefern beispielsweise im Falle des Benzylesters die freie Carbonsäure.

Es ist auch möglich die Reaktionen jeweils stufenweise durchzuführen und die Reihenfolge der Bausteinverknüpfungen unter Anpassung des Schutzgruppenkonzepts zu verändern.

Die Ausgangsstoffe oder Ausgangsverbindungen sind in der Regel bekannt. Sind sie neu, so können sie nach an sich bekannten Methoden hergestellt werden.

Die Ausgangsstoffe können, falls erwünscht, auch in situ gebildet werden, so dass man sie aus dem Reaktionsgemisch nicht isoliert, sondern sofort weiter zu den Verbindungen der Formel I umsetzt.

Vorzugsweise werden die Verbindungen der Formel I erhalten, indem man sie aus ihren funktionellen Derivaten durch Solvolyse, insbesondere durch Hydrolyse oder durch Hydrogenolyse freisetzt. Bevorzugte Ausgangsstoffe für die Solvolyse bzw. Hydrogenolyse sind solche, die an der Stelle einer oder mehrerer freier Amino-, Carboxyl- und/oder Hydroxygruppen entsprechend geschützte Amino-, Carboxyl- und/oder Hydroxygruppen enthalten, vorzugsweise solche, die an der Stelle eines H-Atoms, das mit einem N-Atom verbunden ist, eine Aminoschutzgruppe tragen. Ferner sind Ausgangsstoffe bevorzugt, die an der Stelle des H-Atoms einer Hydroxygruppe eine Hydroxyschutzgruppe tragen. Außerdem sind Ausgangsstoffe bevorzugt, die an der Stelle einer freien Carboxylgruppe eine geschützte Carboxygruppe tragen. Es können auch mehrere, gleiche oder verschiedene geschützte Amino-, Carboxyl- und/oder Hydroxygruppen im Molekül des Ausgangsstoffs vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen de Moleküls durchgeführt worden ist. Typisch für solche Gruppen sind insbesondere unsubstituierte oder substituierte Acylgruppen, ferner unsubstituierte oder substituierte Aryl- (z.B. 2,4-Dinitophenyl) oder Aralkylgruppen (z.B. Benzyl, 4-Nitrobenzyl, Triphenylmethyl). Da die Aminoschutzgruppen nach der gewünschten Reaktion oder Reaktionsfolge entfernt werden, ist ihre Art und Größe im Übrigen nicht kritisch, bevorzugt sind jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterozyklischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acteyl, Propionyl, Buturyl, Aralkanoyl wie Phenylacetyl, Aroyl wie Benzoyl oder Toluyl, Aryoxyaklkanoyl wie Phenoxyacetyl, Alkyoxycarbonyyl wie Methoxycarbonyl, Ethoxycarbonyl, 2,2,2-Trichlorethoxycarbonyl, BOC, 2-Jodethoxycaronyl, Aralkyloxycarbonyl wie CBZ, 4-Methoxybenzyloxycarbonyl oder FMOC. Bevorzugte Acylgruppen sind CBZ, FMOC, Benzyl und Acetyl.

Der Ausdruck "Säureschutzgruppe" oder "Carboxylschutzgruppe" ist ebenfalls allgemein bekannt und bezieht sich auf Gruppen, die geeignet sind, eine -COOH-gruppe vor chemischen Umsetzungen zu schützen, die aber leicht entfernbar sind, nachdem die gewünschte chemische Reaktion an anderen Stellen des Moleküls durchgeführt worden sind. Typisch ist die Verwendung von Estern anstelle der freien Säuren z.B. von substutierten und unsubstituierten Alkylestern (wie Methyl, Ethyl, tert-Butyl und deren substituierte Derivate), von substutierten und unsubstituierten Benzylestern oder Silylestern. Die Art und Größe der Säureschutzgruppen ist nicht kritisch, bevorzugt sind jedoch solche mit 1-20, insbesondere 1-10 C-Atomen.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden sind. Typisch für solche Gruppen sind die oben genannten unsubstituierten oder substituierten Aryl-, Aralkyl- odder Acylgruppen, ferner auch Alkylgruppen. Die ihre Art und Größe der Hydroxyschutzgruppen ist nicht kritisch, bevorzugt sind jedoch solche mit 1-20, insbesondere 1-10 C-Atomen. Beispiele für Hyrdoxyschutzgruppen sind u.a. Benzyl, p-Nitrobenzoyl, p-Toluolsulfonyl und Acetyl, wobei Benzyl und Acetyl bevorzugt sind.

Weitere typische Beispiele für Amino-, Säure-, und Hydroxyschutzgruppen finden sich beispielsweise in "Greene's Protective Groups in Organic Synthesis", vierte Auflage, Wiley-Interscience, 2007

Die als Ausgangstoffe zu verwendenden funktionellen Derivate der Verbindungen der Formel I können nach bekannten Methoden der Aminosäure- und Peptidsynthese hergestellt werden, wie sie z.B. in den genannten Standardwerken und Patentanmeldungen beschrieben sind.

Die Freisetzung der Verbindungen der Formel I aus ihren funktionellen Derivaten gelingt, je nach verwendeter Schutzgruppe, z.B. mithilfe starker Säuren, zweckmäßig mit Trifluoressigsäure oder Perchlorsäure, jedoch auch mit anderen starken anorganischen Säuren wie Salz- oder Schwefelsäure, starken organischen Säuren wie Trichloressigsäure oder Sulfonsäuren wie Benzoyl- oder p-Toluol-Sulfonsäure. Die Anwesenheit eines zusätzlichen inerten Lösungsmittels und/oder eines Katalysators ist möglich, jedoch nicht immer erforderlich.

In Abhängigkeit des jeweiligen Synthesewegs kann man die Ausgangsstoffe, gegebenenfalls in Anwesenheit eines inerten Lösungsmittels reagieren lassen.

Als inerte Lösungsmittel eignen sich z.B. Heptan, Hexan, Petrolether, DMSO, Benzol, Toluol, Xylol, Trichlorethylen-, 1,2- Dichlorethantetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether (bevorzugt für die Substitution am Indolstickstoff), Tetrahydrofuran (THF) oder Dioxan; Glykolether wie Ethylenglykolmonomethyl- oder monoethylether (Methylglykol oder Ethylglykol), Ethylenglykoldimethy-lether (Diglyme); Ketone wie Aceton oder Butanon; Amide wie Acetamid, Dimethylacetamid, N-Methylpyrrolidon (NMP) oder Dimethylformamid (DMF); Nitrile wie Acetonitril; Ester wie Ethylacetat, Carbonsäuren oder Säureanhydride, wie z. B. wie Essigsäure oder Acetanhydrid, Nitroverbindungen wie Nitromethan oder Nitrobenzol, gegebenenfalls auch Gemische der genannten Lösungsmittel untereinander oder Gemische mit Wasser.

Die Menge des Lösungsmittels ist nicht kritisch, vorzugsweise können 10 g bis 500 g Lösungsmittel je g der umzusetzenden Verbindung der Formel I zugesetzt werden.

Vorteilhaft kann die Zugabe eines säurebindenden Mittels sein, z.B. ein Alkalimetall oder Erdalkalimetallhydroxid, -carbonat oder -bicarbonat oder andere Alkalimetall- oder Erdalkalimetallsalze schwacher Säuren, vorzugsweise ein Kalium-, Natrium- oder Calciumsalz, oder die Zugabe einer organischen Base, wie beispielsweise an Triethylamin, Dimethylamin, Pyridin oder Chinolin, oder ein Überschuss der Aminkomponente.

Die erhaltenen erfindungsgemäßen Verbindungen können von der entsprechenden Lösung, in der sie hergestellt werden, getrennt werden (z.B. durch Zentrifugation und Waschen) und können nach der Trennung in einer anderen Zusammensetzung gelagert werden, oder sie können direkt in der Herstellungslösung verbleiben. Die erhaltenen erfindungsgemäßen Verbindungen können auch zur jeweiligen Verwendung in gewünschte Lösungsmittel aufgenommen werden.

Geeignete Reaktionstemperaturen liegen bei Temperaturen von 0 bis 40°C, vorzugsweise bei 5 bis 25°C.

Die Dauer der Umsetzung hängt von den gewählten Reaktionsbedingungen ab. In der Regel beträgt die Reaktionsdauer 0.5 Stunden bis 10 Tage, vorzugsweise 1 bis 24 Stunden. Bei Verwendung einer Mikrowelle kann die Reaktionszeit auf Werte von 1 bis 60 Minuten reduziert werden.

Die Verbindungen der Formel I und auch die Ausgangsstoffe zu ihrer Herstellung werden im übrigen nach bekannten Methoden hergestellt, wie sie in der Literatur beschrieben sind (z. B. in Standardwerken wie Houben-Weyl, Methoden der organischen Chemie, Georg-Thieme-Verlag, Stuttgart) so z.B. unter Reaktionsbedingungen, die für die genannten Umsetzungen bekannt und geeignet sind. Dabei kann man auch von an sich bekannten, hier nicht näher beschriebenen Varianten Gebrauch machen.

Durch übliche Aufarbeitungsschritte wie z. B. Wasserzugabe zum Reaktionsgemisch und Extraktion können die Verbindungen nach Entfernung des Lösungsmittels erhalten werden. Es kann vorteilhaft sein, zur weiteren Reinigung des Produktes eine Destillation oder Kristallisation anzuschließen oder eine chromatographische Reinigung durchzuführen.

Eine Säure der Formel I kann mit einer Base in das dazugehörige Additionssalz überführt werden, beispielsweise durch Umsetzung äquivalenter Mengen der Säure und der Base in einem inerten Lösungsmittel wie Ethanol und einschließendes Eindampfen. Für diese Umsetzung kommen insbesondere Basen in Frage, die physiologisch unbedenkliche Salze liefern. So kann die Säure der Formel I mit einer Base (z.B. Natrium- oder Kaliumhydroxid oder -carbonat) in das entsprechende Metall-, insbesondere Alkali- oder Erdalkalimetall- oder in das entsprechende Ammoniumsalz umgewandelt werden. Für diese Umsetzung kommen auch organische Basen in Frage, die physiologisch unbedenkliche Salze liefern, wie z.B. Ethanolamin.

Andererseits kann eine Base der Formel I mit einer Säure in das zugehörige Säureadditionssalz überführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wir Orthophosphorsäure, Sulfaminsäure, ferner organische Säuren, insbesondere aliphatische, alicyclische, araliphatische, aromatische oder heterocyclische, ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxysulfonsäure, Benzolsulfonsäure, p-Toluolsulfonsäure, Naphthalin-mom- und disulfonsäuren oder Laurylschwefelsäure. Salze mit physiologisch nicht unbedenklichen Säuren, z.B. Pikrate, können zur Isolierung und/oder Aufreinigung der Verbindungen der Formel I verwendet werden.

Es wurde gefunden, dass die Verbindungen der Formel I gut verträglich sind und wertvolle pharmakologische Eigenschaften besitzen, da sie Aspartylproteasen und insbesondere Cathepsin D selektiv inhibieren. Veröffentlicht ist auch die Verwendung von erfindungsgemäßen Verbindungen zur Herstellung eines Medikaments zur Behandlung und/oder Prophylaxe von Krankheiten, die durch Cathepsin D und/oder durch Cathepsin D-vermittelte Signaltransduktion verursacht, vermittelt und/oder propagiert werden.

Gegenstand der Erfindung ist somit insbesondere auch ein Arzneimittel enthaltend wenigstens eine erfindungsgemäße Verbindung und/oder eines ihrer physiologisch unbedenklichen Salze, Solvate und Stereoisomere, einschließlich deren Mischungen in allen Verhältnissen zur Verwendung bei der Behandlung und/oder Prophylaxe von physiologischen und/oder pathophysiologischen Zuständen.

Besonders bevorzugt sind insbesondere physiologische und/oder pathophysiologische Zustände, die mit Cathepsin D in Zusammenhang stehen.

Unter physiologischen und/oder pathophysiologischen Zuständen werden physiologische und/oder pathophysiologische Zustände verstanden, die medizinisch relevant sind, wie beispielsweise Krankheiten bzw. Erkrankungen und medizinische Störungen, Beschwerden, Symptome oder Komplikationen und dergleichen, insbesondere Krankheiten.

Ein weiterer Gegenstand der Erfindung ist ein Arzneimittel enthaltend wenigstens eine erfindungsgemäße Verbindung und/oder eines ihrer physiologisch unbedenklichen Salze, Solvate und Stereoisomere, einschließlich deren Mischungen in allen Verhältnissen zur Verwendung bei der Behandlung und/oder Prophylaxe von physiologischen und/oder pathophysiologischen Zuständen, ausgewählt aus der Gruppe bestehend Arthrose, traumatische Knorpelverletzungen und Arthritis, insbesondere rheumatoide Arthritis.

Ein weiterer Gegenstand der Erfindung ist ein Arzneimittel enthaltend wenigstens eine erfindungsgemäße Verbindung und/oder eines ihrer physiologisch unbedenklichen Salze, Solvate und Stereoisomere, einschließlich deren Mischungen in allen Verhältnissen zur Verwendung bei der Behandlung und/oder Prophylaxe von physiologischen und/oder pathophysiologischen Zuständen, ausgewählt aus der Gruppe bestehend aus Alzheimer Krankheit, Chorea Huntington, Mucolipidose, Krebs, insbesondere Brustkrebs, Kontaktdermatitis, Spät-Typ der Überempfindlichkeitsreaktion, Entzündungen, Endometriose, Vernarbung, gutartige Prostatahyperplasie, Osteosarkom, Rachitis, Hauterkrankungen wie beispielsweise Psoriasis, immunologische Krankheiten, Autoimmunkrankheiten und Immunschwächekrankheiten.

In diesem Zusammenhang sind Hirnkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischer Krebs, Schilddrüsenkrebs, Lymphome, chronische Leukämie und akute Leukämie als krebsartige Erkrankungen anzusehen, die alle gewöhnlich zur Gruppe der hyperproliferative Erkrankungen gezählt werden.

Schmerz ist eine komplexe Sinneswahrnehmung, die als akutes Geschehen den Charakter eines Warn- und Leitsignals aufweist, als chronischer

Schmerz diesen aber verloren hat und in diesem Fall (als *Chronisches Schmerzsyndrom*) heute als eigenständiges Krankheitsbild gesehen und behandelt werden soll. Als Hyperalgesie wird in der Medizin eine übermäßige Schmerzempfindlichkeit und Reaktion auf einen üblicherweise schmerzhaften Reiz hin bezeichnet. Reize, die Schmerzen auslösen können, sind beispielsweise Druck, Wärme, Kälte oder Entzündungen. Die Hyperalgesie ist eine Form der Hyperästhesie, dem Oberbegriff für eine übermäßige Empfindlichkeit auf einen Reiz hin. Als Allodynie wird in der Medizin eine Schmerzempfindung bezeichnet, die durch Reize ausgelöst wird, welche üblicherweise keinen Schmerz verursachen.

Es ist beabsichtigt, dass mit obenstehend offenbarten Arzneimitteln eine entsprechende Verwendung der erfindungsgemäßen Verbindungen zur Herstellung eines Medikaments zur Behandlung und/oder Prophylaxe von vorstehenden physiologischen und/oder pathophysiologischen Zuständen umfasst ist.

Es ist außerdem beabsichtigt, dass mit obenstehend offenbarten Arzneimitteln ein entsprechendes Verfahren zur Behandlung und/oder Prophylaxe von vorstehenden physiologischen und/oder pathophysiologischen Zuständen, bei dem man wenigstens eine erfindungsgemäße Verbindung an einen Patienten verabreicht, der eine solche Behandlung benötigt, umfasst ist.

Die erfindungsgemäßen Verbindungen zeigen bevorzugt eine vorteilhafte biologische Aktivität, die in Enzym-Assays und Tierversuchen, wie in den Beispielen beschrieben, leicht nachweisbar ist. In derartigen auf Enzymen basierenden Assays zeigen und bewirken die erfindungsgemäßen Antikörper bevorzugt einen inhibierenden Effekt, der gewöhnlich durch IC₅₀-Werte in einem geeigneten Bereich, bevorzugt im mikromolaren Bereich und bevorzugter im nanomolaren Bereich dokumentiert wird.

Die erfindungsgemäßen Verbindungen können an Menschen oder Tiere, insbesondere Säugetiere, wie Affen, Hunde, Katzen, Ratten oder Mäuse verabreicht werden und bei der therapeutischen Behandlung des menschlichen oder des tierischen Körpers sowie bei der Bekämpfung der oben aufgeführten Krankheiten verwendet werden. Sie können weiterhin als Diagnostika oder als Reagenzien Verwendung finden.

Weiterhin können erfindungsgemäße Verbindungen zur Isolierung und zur Untersuchung der Aktivität oder Expression von Cathepsin D verwendet werden. Außerdem eignen sie sich insbesondere zur Verwendung in diagnostischen Verfahren zu Erkrankungen im Zusammenhang mit gestörter Cathepsin D-Aktivität. Ein weiterer Gegenstand der Erfindung ist deshalb die Verwendung der erfindungsgemäßen Verbindungen zur Isolierung und zur Untersuchung der Aktivität oder Expression von Cathepsin D bzw. als Binder und Inhibitoren von Cathepsin D.

Für diagnostische Zwecke können die erfindungsgemäßen Verbindungen beispielsweise radioaktiv markiert werden. Beispiele für radioaktive Markierungen sind ³H, ¹⁴C, ²³¹I und ¹²⁵I. Ein bevorzugtes Markierungsverfahren ist die Iodogen-Methode (Fraker et al., 1978). Darüber hinaus können die erfindungsgemäßen Verbindungen durch Enzyme, Fluorophore und Chemophore markiert werden. Beispiele für Enzyme sind alkalische Phosphatase, β-Galaktosidase und Glucoseoxidase, ein Beispiel für eine Fluorophor ist Fluorescin, ein Beispiel für ein Chemophor ist Luminol und Automatisierte Detektionssysteme beispielsweise für fluoreszente Färbungen werden beispielweise in US 4,125,828 und US 4,207,554 beschrieben.

Die Verbindungen der Formel I können zur Herstellung pharmazeutischer Zubereitungen verwendet werden, insbesondere auf nicht-chemischem Wege. Hierbei werden sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht.

Ein weiterer Gegenstand der Erfindung sind deshalb pharmazeutische Zubereitungen, enthaltend wenigstens eine erfindungsgemäße Verbindung der Formel I und/oder ihre physiologisch unbedenklichen Salze, Derivate, Solvate, und Stereoisomere, einschließlich deren Mischungen in allen Verhältnissen. Gegenstand der Erfindung sind insbesondere auch solche pharmazeutische Zubereitungen, die weitere Träger- und/oder Hilfsstoffe enthalten, sowie auch solche pharmazeutische Zubereitungen, die wenigstens einen weiteren Arzneimittelwirkstoff enthalten. Veröffentlicht ist auch ein Verfahren zur Herstellung einer pharmazeutischen Zubereitung, dadurch gekennzeichnet, dass man eine Verbindung der Formel I und/oder eines ihrer physiologisch unbedenklichen Salze, Derivate, Solvate, und Stereoisomere, einschließlich deren Mischungen in allen Verhältnissen zusammen mit einem festen, flüssigen oder halbflüssigen Träger- oder Hilfsstoff und gegebenenfalls mit einem weiteren Arzneimittelwirkstoff in eine geeignete Dosierungsform bringt.

Die erfindungsgemäßen pharmazeutischen Zubereitungen können als Arzneimittel in der Human- oder Veterinärmedizin verwendet werden. Der Patient oder Wirt kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle (wie Sonnenblumenöl oder Lebertran), Benzylalkohole, Polyethylenglykole, Gelatine, Kohlehydrate wie Laktose oder Stärke, Magnesiumstearat, Talk, Lanolin oder Vaseline. Welche Hilfsstoffe für die gewünschte Arzneimittelformulierung geeignet sind, ist dem Fachmann auf Grund seines Fachwissens geläufig. Neben Lösungsmitteln z.B. Wasser, physiologische Kochsalzlösung oder Alkohole wie z.B. Ethanol, Propanol oder Glycerin, Zuckerlösungen wie Glucose oder Mannitlösungen oder eine Mischung der genannten Lösungsmittel, Gelbildnern, Tablettenhilfsstoffen und anderen Wirkstoffträgern können beispielsweise Gleitmittel, Stabilisatoren und/oder Netzmittel, Emulgatoren, Salze zur Beeinflussung des osmotischen Druckes, Antioxidantien, Dispergiermittel, Entschäumer, Puffersubstanzen, Geschmacks- und/oder Aromastoffe bzw. Geschmackskorrigentien, Konservierungsmittel, Lösungsvermittler oder Farbstoffe verwendet werden. Falls erwünscht, können erfindungsgemäße Zubereitungen oder Medikamente einen oder mehrere weitere Wirkstoffe enthalten, beispielsweise ein oder mehrere Vitamine.

Die Begriffe "pharmazeutische Formulierung" und "pharmazeutische Zubereitung" werden im Rahmen der vorliegenden Erfindung als Synonyme verwendet.

Wie hier verwendet bezieht sich "pharmazeutisch verträglich" auf Arzneimittel, Präzipitationsreagezien, Trägerstoffe, Hilfsstoffe, Stabilisatoren, Lösungsmittel und sonstige Agenzien, die die Verabreichung der daraus erhaltenen pharmazeutischen Zubereitungen ohne unerwünschte physiologische Nebenwirkungen, wie beispielsweise Übelkeit, Schwindel, Verdauungsprobleme o.ä. an ein Säugetier ermöglichen .

Bei pharmazeutischen Zubereitungen zur parenteralen Verabreichung besteht die Forderung nach Isotonie, Euhydrie sowie nach Verträglichkeit und Sicherheit der Formulierung (geringe Toxizität), der eingesetzten Hilfsstoffe und des Primärpackmittels. Überraschenderweise haben die erfindungsgemäßen Verbindungen vorzugsweise den Vorteil, dass eine direkte Verwendung möglich ist und vor Verwendung der erfindungsgemäßen Verbindungen in pharmazeutischen Formulierungen keine weiteren Reinigungsschritte zum Entfernen toxikologisch bedenklicher Agenzien, wie beispielsweise hohe Konzentrationen organischer Lösungsmittel oder anderer toxikologisch bedenklicher Hilfsstoffe, erforderlich sind.

Ein besonders bevorzugter Gegenstand der Erfindung sind auch pharmazeutische Zubereitungen enthaltend wenigstens eine erfindungsgemäße Verbindung in präzipitierter nicht-kristalliner, präzipitierter kristalliner oder in gelöster oder suspendierter Form, sowie gegebenenfalls Träger- und/oder Hilfsstoffe und/oder weitere pharmazeutische Wirkstoffe.

Vorzugsweise ermöglichen es die erfindungsgemäßen festen Verbindungen, hochkonzentrierte Formulierungen herzustellen, ohne dass es zu ungünstigen unerwünschten Aggregationen der erfindungsgemäßen Verbindungen kommt. So lassen sich mithilfe erfindungsgemäßer Verbindungen mit wässrigen Lösungsmitteln oder in wässrigen Medien applikationsfertige Lösungen mit hohem Wirkstoffanteil herstellen.

Die Verbindungen und/oder deren physiologisch unbedenklichen Salze und Solvate können auch lyophilisiert und die erhaltenden Lyophilisate beispielsweise zur Herstellung von Injektionspräparaten verwendet werden.

Wässrige Zubereitungen können hergestellt werden, indem man erfindungsgemäße Verbindungen in einer wässrigen Lösung löst oder suspendiert und gegebenenfalls Hilfsstoffe zufügt. Zweckmäßigerweise wird hierzu einer Lösung oder Suspension mit einer definierten Konzentration an erfindungsgemäßen Verbindungen mit definierten Volumina an Stammlösungen, die die genannten weiteren Hilfsstoffe in definierter Konzentration enthalten, versetzt und ggf. mit Wasser auf die vorberechnete Konzentration verdünnt. Alternativ können die Hilfsstoffe in fester Form zugesetzt werden. Die erhaltene wässrige Lösung oder Suspension kann anschließend mit den jeweils erforderlichen Mengen an Stammlösungen und/oder Wasser versetzt werden. Zweckmäßigerweise können erfindungsgemäße Verbindungen auch direkt in einer alle weiteren Hilfsstoffe enthaltenden Lösung gelöst oder suspendiert werden.

Vorteilhaft können die erfindungsgemäßen Verbindungen enthaltende Lösungen oder Suspensionen mit einem pH-Wert von 4 bis 10, bevorzugt mit einem pH-Wert von 5 bis 9, und einer Osmolalität von 250 bis 350 mOsmol/kg hergestellt werden. Die pharmazeutische Zubereitung kann somit weitgehend schmerzfrei intravenös, intraarteriell, intraartikulär, subkutan oder perkutan direkt verabreicht werden. Daneben kann die Zubereitung auch Infusionslösungen, wie z.B. Glukoselösung, isotonische Kochsalzlösung oder Ringerlösung, die auch weitere Wirkstoffe enthalten können, zugesetzt werden, so dass auch größere Wirkstoffmengen appliziert werden können.

Erfindungsgemäße pharmazeutische Zubereitungen können auch Mischungen mehrerer erfindungsgemäßer Verbindungen enthalten.

Die erfindungsgemäßen Zubereitungen sind physiologisch gut verträglich, leicht herstellbar, exakt dosierbar und vorzugsweise über die Dauer der Lagerung sowie des Transports und bei mehrfachen Einfrier- und Auftauvorgängen hinsichtlich Gehalt, Zersetzungsprodukten und Aggregaten stabil. Sie können bei Kühlschranktemperatur (2-8°C) und bei Raumtemperatur (23-27°C) und 60 % relativer Luftfeuchtigkeit (r.F.) vorzugsweise über einen Zeitraum von mindestens drei Monaten bis zu zwei Jahren stabil gelagert werden.

Beispielsweise können die erfindungsgemäßen Verbindungen durch Trocknung stabil gelagert und bei Bedarf durch Lösung oder Suspension in eine gebrauchsfertige pharmazeutische Zubereitung überführt werden. Mögliche Methoden zur Trocknung sind zum Beispiel, ohne auf diese Beispiele beschränkt zu sein, Stickstoffgastrocknung, Vakuumofen-Trocknung, Lyophilization, Waschen mit organischem Lösungsmittel und anschließende Lufttrocknung, Flüssigbett-Trocknung, Wirbelschichttrocknung, Sprühtrocknung, Walzentrocknung, Lagentrocknung; Lufttrocknung bei Raumtemperatur und weitere Methoden.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder angestrebt wird.

Darüber hinaus bedeutet der Ausdruck "therapeutisch wirksame Menge" eine Menge, die, verglichen zu einem entsprechenden Subjekt, das diese Menge nicht erhalten hat, folgendes zur Folge hat: verbesserte Heilbehandlung, Heilung, Prävention oder Beseitigung einer Krankheit, eines Krankheitsbildes, eines Krankheitszustandes, eines Leidens, einer Störung oder Verhinderung von Nebenwirkungen oder auch die Verminderung des Fortschreitens einer Krankheit, eines Leidens oder einer Störung. Die Bezeichnung "therapeutisch wirksame Menge" umfasst auch die Mengen, die wirkungsvoll sind, die normale physiologische Funktion zu erhöhen.

Bei Verwendung von erfindungsgemäßen Zubereitungen oder Medikamenten werden die erfindungsgemäßen Verbindungen und/oder dessen physiologisch unbedenklichen Salze und Solvate in der Regel analog zu bekannten, käuflich erhältlichen Zubereitungen oder Präparaten verwendet, vorzugsweise in Dosierungen zwischen 0,1 und 500 mg, insbesondere 5 und 300 mg pro Anwendungseinheit. Die tägliche Dosierung liegt vorzugsweise zwischen 0,001 und 250 mg/kg, insbesondere 0,01 und 100 mg/kg Köpergewicht. Die Zubereitung kann ein- oder mehrmals pro Tag verabreicht werden, z.B. zwei-, drei- oder viermal am Tag. Jedoch hängt die individuelle Dosierung für einen Patienten von einer großen Zahl individueller Faktoren ab, wie beispielsweise von der Wirksamkeit der jeweils verwendeten Verbindung, vom Alter, Körpergewicht, allgemeinen Gesundheitszustand, Geschlecht, Ernährung, vom Verabreichungszeitpunkt und -weg, von der Ausscheidungsrate, von der Kombination mit anderen Arzneimitteln und von der Schwere und Dauer der jeweiligen Erkrankung.

Ein Maß für die Aufnahme eines Arzneimittelwirkstoffs in einen Organismus ist seine Bioverfügbarkeit. Wird der Arzneimittelwirkstoff in Form einer Injektionslösung dem Organismus intravenös zugeführt, so liegt seine absolute Bioverfügbarkeit, d.h. der Anteil des Pharmakons, der unverändert im systemischen Blut, d.h. in den großen Kreislauf gelangt, bei 100%. Bei oraler Gabe eines therapeutischen Wirkstoffs liegt der Wirkstoff in der Regel als Feststoff in der Formulierung vor und muss sich daher zuerst auflösen, damit er die Eintrittsbarrieren, beispielsweise den Gastrointestinaltrakt, die Mundschleimhaut, nasale Membranen oder die Haut, insbesondere das Stratum corneum, überwinden kann bzw. vom Körper resorbiert werden kann. Daten zur Pharmakokinetik, d.h. zur Bioverfügbarkeit können analog zu der Methode von J. Shaffer et al., J. Pharm. Sciences, 88 (1999), 313-318 erhalten werden.

Weiterhin lassen sich solche Arzneimittel mit einem der im pharmazeutischen Fachgebiet allgemein bekannten Verfahren herstellen.

Arzneimittel lassen sich zur Verabreichung über einen beliebigen geeigneten Weg, beispielsweise auf oralem (einschließlich buccalem bzw. sublingualem), rektalem, pumonalen, nasalem, topischem (einschließlich buccalem, sublingualem oder transdermalem), vaginalem oder parenteralem (einschließlich subkutanem, intramuskulärem, intravenösem, intradermalem und insbesondere intraartikulärem) Wege, anpassen. Solche Arzneimittel können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

Zur Verabreichung der erfindungsgemäßen Arzneimittel eignet sich vorzugsweise die parenterale Applikation. Im Falle der parenteralen Applikation ist die intraartikuläre Applikation besonders bevorzugt. Veröffentlicht ist so auch die Verwendung einer erfindungsgemäßen pharmazeutischen Zubereitung zur intraartikulären Applikation bei der Behandlung und/oder Prophylaxe von physiologischen und/oder pathophysiologischen Zuständen, ausgewählt aus der Gruppe bestehend aus Arthrose, traumatischen Knorpelverletzungen, Arthritis, Schmerz, Allodynie oder Hyperalgesie.

Die intraartikuläre Applikation hat den Vorteil, dass die erfindungsgemäße Verbindung direkt in die Nähe des Gelenkknorpels in die Synovialflüssigkeit appliziert werden und von dort auch in das Knorpelgewebe hineindiffundieren kann. Erfindungsgemäße pharmazeutische Zubereitungen können somit auch direkt in den Gelenkspalt injiziert werden und so direkt am bestimmungsgemäßen Wirkort ihre Wirkung entfalten. Die erfindungsgemäßen Verbindungen eignen sich auch zur Herstellung von parenteral zu applizierenden Arzneimitteln mit kontrollierter, gleichmäßiger und/oder verzögerter Wirkstofffreisetzung (slow-release, sustained-release, controlled release). Somit eignen sie sich auch zur Herstellung von Depot-Formulierungen, die für den Patienten vorteilhaft sind, da eine Applikation nur in größeren Zeitintervallen notwendig ist.

Zu den an die parenterale Verabreichung angepassten Arzneimitteln gehören wässrige und nichtwässrige sterile Injektionslösungen, die Antioxidantien, Puffer, Bakteriostatika und Solute, durch die die Formulierung isotonisch mit dem Blut oder der Synovialflüssigkeit des zu behandelnden Empfängers gemacht wird, enthalten; sowie wässrige und nichtwässrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so dass nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Die erfindungsgemäßen Verbindungen lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die erfindungsgemäßen Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die erfindungsgemäßen Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate, Polymilchcoglykolsäure, Polymere wie Konjugate zwischen Dextran und Methacrylate, Polyphosphoester, verschiedene Polysaccharide und Polyamine sowie Poly-ε-caprolacton, Albumin, Chitosan, Collagen oder modifizierte Gelatine und quervernetzte oder amphipatische Blockcopolymere von Hydrogelen, gekoppelt sein.

Zur enteralen Applikation (oral oder rektal) dienen insbesondere Tabletten, Dragees, Kapseln, Sirupe, Säfte, Tropfen oder Suppositorien, für die topische Anwendung Salben, Creme, Pasten, Lotionen, Gele, Sprays, Schäume, Aerosole, Lösungen (z.B. Lösungen in Alkoholen wie Ethanol oder Isopropanol, Acetonitril, DMF, Dimethylacetamid, 1,2-Propandiol oder deren Gemischen untereinander und/oder mit Wasser) oder Puder. Insbesondere für topische Anwendungen kommen auch liposomale Zubereitungen in Betracht.

Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

An die transdermale Verabreichung angepasste Arzneimittel können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

Es versteht sich, dass die erfindungsgemäßen Arzneimittel neben den obigen besonders erwähnten Bestandteilen andere im Fachgebiet übliche Mittel mit Bezug auf die jeweilige Art der pharmazeutischen Formulierung enthalten können.

Gegenstand der Erfindung ist auch ein Set (Kit) bestehend aus getrennten Packungen von
a) einer wirksamen Menge einer erfindungsgemäßer Verbindung der Formel I und/oder ihrer physiologisch unbedenklichen Salze, Derivate, Solvate und Stereoisomere, einschließlich deren Mischungen in allen Verhältnissen und
b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge einer Verbindung der Formel I und/oder ihrer pharmazeutische unbedenklichen Derivate, Solvate, Stereoisomere, einschließlich deren Mischungen in allen Verhältnissen, und eine wirksame Menge eines weiteren Arzneimittelwirkstoffs gelöst oder in lyophilisierter Form vorliegt.

Weiterhin können die erfindungsgemäße Arzneimittel verwendet werden, um bei gewissen bekannten Therapien additive oder synergistische Effekte bereitzustellen, und/oder können dazu verwendet werden, um die Wirksamkeit gewisser existierender Therapien wiederherzustellen.

Die erfindungsgemäßen pharmazeutischen Zubereitungen können neben den erfindungsgemäßen Verbindungen noch weitere Arzneimittelwirkstoffe enthalten, z.B. zur Verwendung bei der Behandlung von Arthrose andere Cathepsin D-Inhibtioren, NSAIDS, Cox-2-Inhibitoren, Glucocorticoide, Hyaluronsäure, Azathioprin, Methotrexat, anti-CAM Antikörper, wie beispielweise anti-ICAM-1 Antikörper, FGF-18. Zur Behandlung der anderen genannten Erkrankungen können die erfindungsgemäßen pharmazeutischen Zubereitungen können neben den erfindungsgemäßen Verbindungen noch weitere Arzneimittelwirkstoffe, die dem Fachmann bei deren Behandlung bekannt sind, enthalten.

Die folgenden Beispiele sollen somit die Erfindung erläutern. Sofern nichts anderes angegeben ist, bedeuten Prozentangaben Gewichtsprozent. Alle Temperaturen sind in Grad Celsius angegeben. "Übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, filtriert, dampft ein und reinigt durch Chromatographie an Kieselgel und /oder durch Kristallisation. Rf-Werte an Kieselgel; Massenspektrometrie: El (Elektronenstoßionisation): M⁺, FAB (Fast Atom Bombardment): (M+H)⁺, THF (Tetrahydrofuran), NMP (N-Methlpyrrolidon), DMSO (Dimethlysulfoxid), EE (Ethylacetat), MeOH (Methanol), DC (Dünnschichtchromatographie)

Die folgenden Substanzen wurden synthetisiert und charakterisiert. Die Herstellung und Charakterisierung der Substanzen ist jedoch auch auf anderen Wegen für den Fachmann durchführbar.

### Beispiel 1: Beispielhafte Verbindungen der Formel I

| Nr. | Verbindung | Molekulargewicht | M+H | Ret [min] | Reninselektivität IC₅₀ s. Beispiel 6 | Stabilität in Synovialflüssigkeit s. Beispiel 5 | CatD-Assay IC₅₀-(M) s. Beispiel 3 | GAG-Assay IC₅₀-(M) s. Beispiel 4 |
|---|---|---|---|---|---|---|---|---|
| 1 | 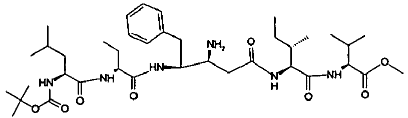 | | | | | | | |
| | (S)-2-((2S,3S)-2-{(3S,4S)-3-Amino-4-[(S)-2-((S)-2-tert-butoxycarbonylamino-4-methyl-pentanoylamino)-butyrylamino]-5-phenyl-pentanoylamino}-3-methyl-pentanoylamino)-3-methyl-buttersäuremethylester | 732,96 | 733,96 | 2,00 | | | 4,00E-08 | 3,13E-09 |
| 2 | 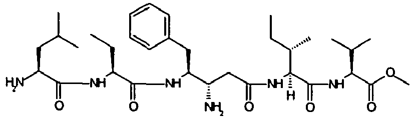 | | | | | | | |
| | (S)-2-((2S,3S)-2-{(3S,4S)-3-Amino-4-[(S)-2-((S)-2-amino-4-methyl-pentanoylamino)butyrylamino]-5-phenyl-pentanoylamino}-3-methyl-pentanoylamino)-3-methyl-buttersäuremethylester | 632,84 | 633,84 | 1,45 | >30µM | | 2,00E-06 | |
| 3 | 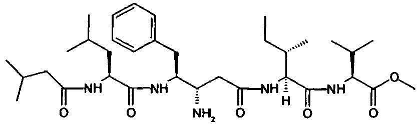 | | | | | | | |
| | (S)-2-((2S,3S)-2-{(3S,4S)-3-Amino-4-[(S)-4-methyl-2-(3-methyl-butyrylamino)-pentanoylamino]-5-phenyl-pentanoylamino}-3-methyl-pentanoylamino)-3-methyl-buttersäuremethylester | 631,85 | 632,85 | 2,00 | | | 5,50E-06 | |
| 4 | 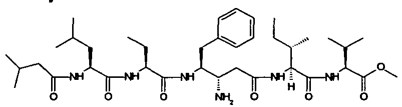 | | | | | | | |
| | (S)-2-[(2S,3S)-2-((3S,4S)-3-Amino-4 -{(S)-2-[(S)-4-methyl-2-(3-methyl-butyrylamino)-pentanoylamino]-1-oxo-butylamino}-5-phenyl-pentanoylamino)-3-methyl-pentanoylamino]-3-methyl-buttersäuremethylester | 716,96 | 717,96 | 1,93 | | | 9,70E-08 | |
| 5 | 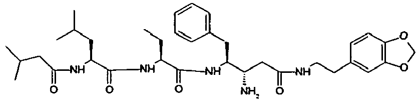 | | | | | | | |
| | (3S,4S)-3-Amino-4-{(S)-2-[(S)-4-methyl-2-(3-methyl-butyrylamino)-pentanoylamino]-butyrylamino}-5-phenyl-pentansäure-(2-benzo[1,3]dioxol-5-yl-ethyl)-amid | 637,82 | 638,82 | 1,81 | >30µM | | 1,80E-06 | 7,73E-09 |
| 6 | 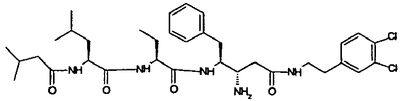 | | | | | | | |
| | (3S,4S)-3-Amino-4-{(S)-2-[(S)-4-methyl-2-(3-methyl-butyrylamino)-pentanoylamino]-butyrylamino}-5-phenyl-pentansäure-[2-(3,4-dichloro-phenyl)-ethyl]-amid | 662,70 | 663,70 | 2,08 | >30µM | | 1,20E-06 | |
| 7 | 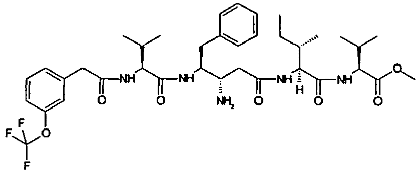 | | | | | | | |
| | (S)-2-[(2S,3S)-2-((3S,4S)-3-Amino-4-{(S)-3-methyl-2-[2-(3-trifluoromethoxy-phenyl)-acetylamino]-butyrylamino}-5-phenyl-pentanoylamino)-3-methyl-pentanoylamino]-3-methyl-buttersäuremethylester | 735,84 | 736,84 | 2,09 | >30µM | | 1,80E- 08 | 2,14E-09 |
| 8 | 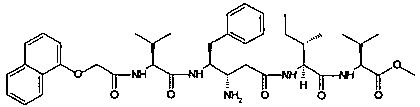 | | | | | | | |
| | (S)-2-[(2S,3S)-2-((3S,4S)-3-Amino-4-{(S)-3-methyl-2-[2-(naphthalen-1-yloxy)-acetylamino]-butyrylamino}-5-phenyl-pentanoylamino)-3-methyl-pentanoylamino]-3-methyl-buttersäure-methylester | 717,90 | 718,90 | 2,11 | >30µM | | 2,00E-07 | |
| 9 | 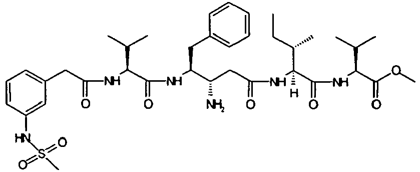 | | | | | | | |
| | (S)-2-[(2S,3S)-2-((3S,4S)-3-Amino-4-{(S)-2-[2-(3-methanesulfonylamino-phenyl)-acetylamino]-3-methyl-butyrylamino}-5-phenyl-pentanoylamino)-3-methyl-pentanoylamino]-3-methyl-buttersäure-methylester | 744,95 | 745,95 | 1,85 | >30µm | | 8,10E-08 | |
| 10 | 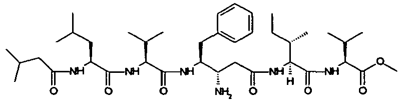 | | | | | | | |
| | (S)-2-[(2S,3S)-2-((3S,4S)-3-Amino-4-{(S)-3-methyl-2-[(S)-4-methyl-2-(3-methyl-butyrylamino)-pentanoylamino]-1-oxo-butylamino}-5-phenyl-pentanoylamino)-3-methyl-pentanoylamino]-3-methyl-buttersäure-methylester | 730,99 | 731,99 | 1,92 | | | 5,10E-09 | |
| 11 | 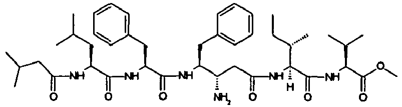 | | | | | | | |
| | (S)-2-[(2S,3S)-2-((3S,4S)-3-Amino-4-{(S)-2-[(S)-4-methyl-2-(3-methyl-butyrylamino)-pentanoylamino]-3-phenyl-propionylamino}-5-phenyl-pentanoylamino)-3-methyl-pentanoylamino]-3-methyl-buttersäuremethylester | 779,03 | 780,03 | 2,19 | >30µM | | 6,10E-07 | |
| 12 | 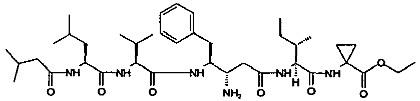 | | | | | | | |
| | 1-[(2S,3S)-2-((S)-3-(S)-Amino-4-{(S)-3-methyl2-[(S)-4-methyl-2-(3-methyl-butyrylamino)-pentanoylamino]-butyrylamino}-5-phenyl-pentanoylamino)-3-methyl-pentanoylamino]-cyclopropanecarbonsäureethylester | 728,97 | 729,97 | 1,93 | >30µM | | 1,10E-07 | |
| 13 | 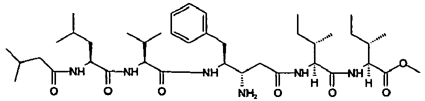 | | | | | | | |
| | (2S,3S)-2-[(S)-2-((S)-3-(S)-Amino-4-{(S)-3-methyl-2-[(S)-4-methyl-2-(3-methyl-butyrylamino)-pentanoylamino]-butyrylamino}-5-phenyl-pentanoylamino)-3-methyl-1-(S)-oxo-pentylamino]-3-methyl-pentansäuremethylester | 745,01 | 746,01 | 2,10 | | | 4,70E-08 | |
| 14 | 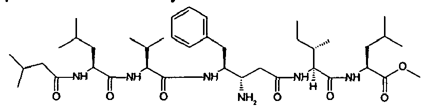 | | | | | | | |
| | (S)-2-[(2S,3S)-2-((S)-3-(S)-Amino-4-{(S)-3-methyl-2-[(S)-4-methyl-2-(3-methyl-butyrylamino)-pentanoylamino]-butyrylamino}-5-phenyl-pentanoylamino)-3-methyl-pentanoylamino]-4-methyl-pentansäure-methylester | 745,01 | 746,01 | 2,09 | >30µM | | 1,60E-07 | |
| 15 | 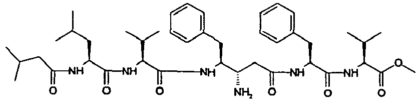 | | | | | | | |
| | (S)-2-[(S)-2-((3S,4S)-3-Amino-4-{(S)-3-methyl-2-[(S)-4-methyl-2-(3-methyl-butyrylamino)-pentanoylamino]-butyrylamino}-5-phenyl-pentanoylamino)-3-phenyl-propionylamino]-3-methyl-buttersäuremethylester | 765,00 | 766,00 | 2,05 | >30µm | | 5,70E-09 | |
| 16 | 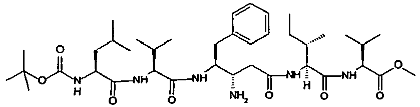 | | | | | | | |
| | (S)-2-((2S,3S)-2-{(3S,4S)-3-Amino-4-[(S)-2-((S)-2-tert-butoxycarbonylamino-4-methyl-pentanoylamino)-3-methyl-butyrylamino]-5-phenyl-pentanoylamino}-3-methyl-pentanoylamino)-3-methyl-buttersäuremethylester | 746,98 | 747,98 | 2,20 | | | 3,60E-08 | |
| 17 | 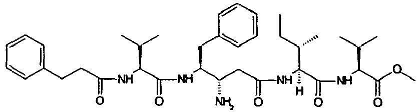 | | | | | | | |
| | (S)-2-((2S,3S)-2-{(3S,4S)-3-Amino-4-[(S)-3-methyl-2-(3-phenyl-propionylamino)-butyrylamino]-5-phenyl-pentanoylamino}-3-methyt-pentanoylamino)-3-methyl-buttersäure-methylester | 665,87 | 666,87 | 1,93 | | | n.d. | 5,52E-08 |
| 18 | 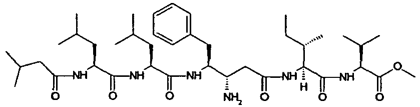 | | | | | | | |
| | (S)-2-[(2S,3S)-2-((3S,4S)-3-Amino-4-{(S)-4-methyl-2-[(S)-4-methyl-2-(3-methyl-butyrylamino)-pentanoylamino]-pentanoylamino}-5-phenyl-pentanoylamino)-3-methyl-pentanoylamino]-3-methyl-buttersäuremethylester | 745,01 | 746,01 | 2,30 | | | n.d. | |
| 19 | 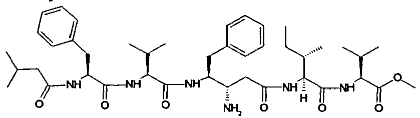 | | | | | | | |
| | (S)-2-[(2S,3S)-2-((3S,4S)-3-Amino-4-{(S)-3-methyl-2-[(S)-2-(3-methyl-butyrylamino)-3-phenyl-propionylamino]-butyrylamino}-5-phenyl-pentanoylamino)-3-methyl-pentanoylamino]-3-methyl-buttersäuremethylester | 765,00 | 766,00 | 2,14 | | | 3,90E-09 | |
| 20 | 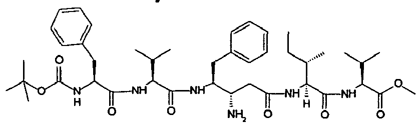 | | | | | | | |
| | (S)-2-((2S,3S)-2-{(3S,4S)-3-Amino-4-[(S)-2-((S)-2-tert-butoxycarbonylamino-3-phenyl-propionylamino)-3-methyl-butyrylamino]-5-phenyl-pentanoylamino}-3-methyl-pentanoylamino)-3-methyl-buttersäuremethylester | 781,00 | 782,00 | 2,20 | | | 1,00E-09 | |
| 21 | 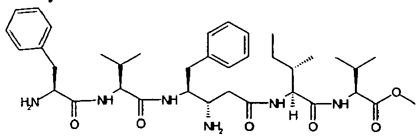 | | | | | | | |
| | (S)-2-((2S,3S)-2-{(3S,4S)-3-Amino-4-[(S)-2-((S)-2-amino-3-phenyl-propionylamino)-3-methyl-butyrylamino]-5-phenyl-pentanoylamino}-3-methyl-pentanoylamino)-3-methyl-buttersäuremethylester | 680,89 | 681,89 | 1,60 | | | 8,10E-08 | |
| 22 | 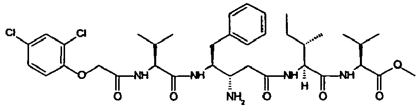 | | | | | | | |
| | (S)-2-[(2S,3S)-2-((3S,4S)-3-Amino-4-{(S)-2-[2-(2,4-dichloro-phenoxy)-acetylamino]-3-methyl-butyrylamino}-5-phenyl-pentanoylamino)-3-methyl-pentanoylamino]-3-methyl-buttersäuremethylester | 736,73 | 737,73 | 2,14 | | | 2,70E-08 | |
| 23 | 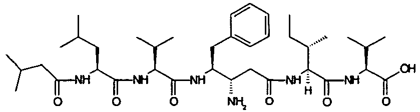 | | | | | | | |
| | (S)-2-[(2S,3S)-2-((3S,4S)-3-Amino-4-{(S)-3-methyl-2-[(S)-4-methyl-2-(3-methyl-butyrylamino)-pentanoylamino]-butyrylamino}-5-phenyl-pentanoylamino)-3-methyl-pentanoylamino]-3-methyl-buttersäure | 716,96 | 717,96 | 1,82 | | | 5,00E-08 | |
| 24 | 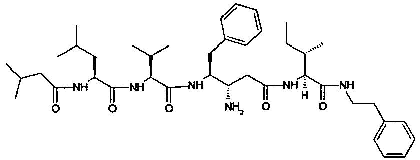 | | | | | | | |
| | (S)-3-Amino-4-{(S)-(S)-3-methyl-2-[(S)-4-methyl-2-(3-methyl-butyrylamino)-pentanoylamino]-butyrylamino}-5-phenyl-pentansäure-((1S,2S)-2-methyl-1-phenethylcarbamoyl-butyl)-amid | 720,99 | 721,99 | 2,18 | >30µM | stabil | 2,40E-07 | |
| 25 | 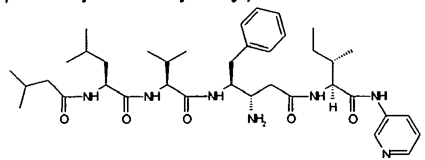 | | | | | | | |
| | (S)-3-Amino-4-{(S)-(S)-3-methyl-2-[(S)-4-methyl-2-(3-methyl-butyrylamino)-pentanoylamino]-butyrylamino}-5-phenyl-pentansäure-[(1S,2S)-2-methyl-1-(pyridin-3-ylcarbamoyl)-butyl]-amid | 693,93 | 694,93 | 1,82 | >30µM | | 5,20E-07 | 2,75E-08 |
| 26 | 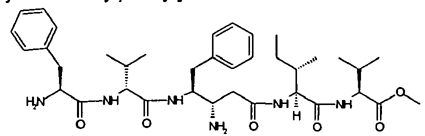 | | | | | | | |
| | (S)-2-((2S,3S)-2-{(3S,4S)-3-Amino-4-[(R)-2-((S)-2-amino-3-phenyl-propionylamino)-3-methyl-butyrylamino]-5-phenyl-pentanoylamino}-3-methyl-pentanoylamino)-3-methyl-buttersäuremethylester | 680,89 | 681,89 | 1,60 | | | n.d. | |
| 27 | 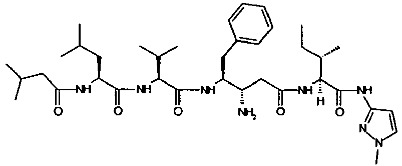 | | | | | | | |
| | (S)-3-Amino-4-{(S)-(S)-3-methyl-2-[(S)-4-methyl-2-(3-methyl-butyrylamino)-pentanoylamino]-butyrylamino}-5-phenyl-pentansäure-[(1S,2S)-2-methyl-1-(1-methyl-1H-pyrazol-3-ylcarbamoyl)-butyl]-amid | 696,93 | 697,93 | 1,92 | | stabil | 4,60E-08 | |
| 28 | 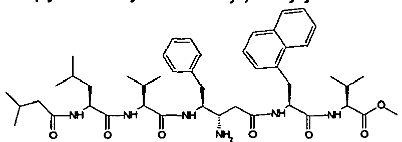 | | | | | | | |
| | (S)-2-[(S)-2-((3S,4S)-3-Amino-4-{(S)-3-methyl-2-[(S)-4-methyl-2-(3-methyl-butyrylamino)-pentanoylamino]-butyrylamino}-5-phenyl-pentanoylamino)-3-naphthalen-1-yl-propionylamino]-3-methyl-buttersäure-methylester | 815,06 | 816,06 | 2,11 | >30µM | | 3,70E-09 | |
| 29 | 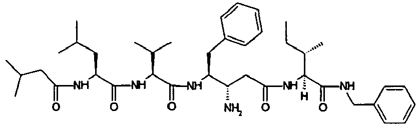 | | | | | | | |
| | (S)-3-Amino-4-{(S)-(S)-3-methyl-2-[(S)-4-methyl-2-(3-methyl-butyrylamino)-pentanoylamino]-butyrylamino}-5-phenyl-pentansäure-((1S,2S)-1-benzylcarbamoyl-2-methyl-butyl)-amid | 706,97 | 707,97 | 2,06 | >30µM | | 1,80E-07 | |
| 30 | 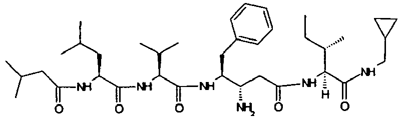 | | | | | | | |
| | (S)-3-(S)-Amino-4-{(S)-3-methyl-2-[(S)-4-methyl-2-(3-methyl-butyrylamino)-pentanoylamino]-butyrylamino}-5-phenyl-pentansäure-[(1S,2S)-1-(cyclopropylmethyl-carbamoyl)-2-methyl-butyl]-amid | 670,93 | 671,93 | 2,05 | >30µM | | 4.00E-07 | |
| 31 | 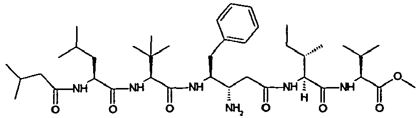 | | | | | | | |
| | (S)-2-[(2S,3S)-2-((3S,4S)-3-Amino-4-{(S)-3,3-dimethyl-2-[(S)-4-methyl-2-(3-methyl-butyrylamino)-pentanoylamino]-butyrylamino}-5-phenyl-pentanoylamino)-3-methyl-pentanoylamino]-3-methyl-buttersäure-methylester | 745,01 | 746,01 | 2,02 | >30µM | | 1,20E-07 | |
| 32 | 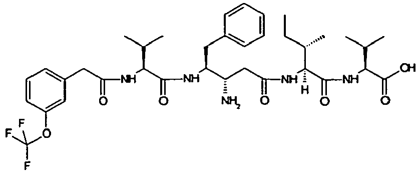 | | | | | | | |
| | (S)-2-[(2S,3S)-2-((3S,4S)-3-Amino-4-{(S)-3-methyl-2-[2-(3-trifluoromethoxy-phenyl)-acetylamino]-butyrylamino}-5-phenyl-pentanoylamino)-3-methyl-pentanoylamino]-3-methyl-buttersäure | 721,81 | 722,81 | 1,97 | >30µM | | 1,20E-08 | |
| 33 | 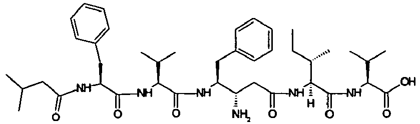 | | | | | | | |
| | (S)-2-[(2S,3S)-2-((3S,4S)-3-Amino-4-{(S)-3-methyl-2-[(S)-2-(3-methyl-butyrylamino)-3-phenyl-propionylamino]-1-oxo-butylamino}-5-phenyl-pentanoylamino)-3-methyl-pentanoylamino]-3-methyl-buttersäure | 750,98 | 751,98 | 1,92 | | stabil | 4,10E-09 | 2,64E-09 |
| 34 | 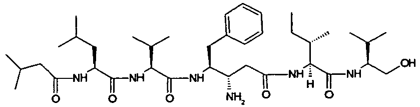 | | | | | | | |
| | (S)-3-(S)-Amino-4-{(S)-3-methyl-2-[(S)-4-methyl-2-(3-methyl-butyrylamino)-pentanoylamino]-butyrylamino}-5-phenyl-pentansäure-[(1S,2S)-1-((S)-1-hydroxymethyl-2-methyl-propylcarbamoyl)-2-methyl-butyl]-amid | 702,98 | 703,98 | 1,91 | | | 6,20E-09 | |
| 35 | 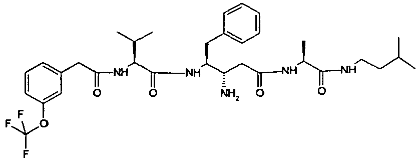 | | | | | | | |
| | (3S,4S)-3-Amino-4-{(S)-3-methyl-2-[2-(3-trifluoromethoxy-phenyl)-acetylamino]-butyrylamino}-5-phenyl-pentansäure-[(S)-1-(3-methyl-butylcarbamoyl)-ethyl]-amid | 649,75 | 650,75 | 1,94 | >30µM | | 1,90E-07 | |
| 36 | 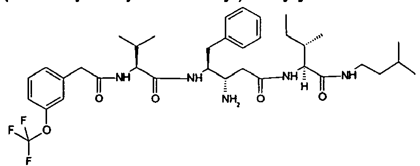 | | | | | | | |
| | (S)-3-(S)-Amino-4-{(S)-3-methyl-2-[2-(3-trifluoromethoxy-phenyl)-acetylamino]-butyrylamino}-5-phenyl-pentansäure-[(1S,2S)-2-methyl-1-(3-methyl-butylcarbamoyl)-butyl]-amid | 691,83 | 692,83 | 2,20 | >30µM | | 2,80E-08 | 1,38E-08 |
| 37 | 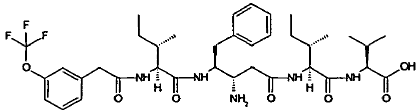 | | | | | | | |
| | (S)-2-[(2S,3S)-2-((3S,4S)-3-Amino-4-{(1S,4S)-3-methyl-2-[2-(3-trifluoromethoxy-phenyl)-acetylamino]-pentanoylamino}-5-phenyl-pentanoylamino)-3-methyl-pentanoylamino]-3-methyl-buttersäure | 735,84 | 736,84 | 2,01 | >30µM | | 3,20E-09 | 8,20E-09 |
| 38 | 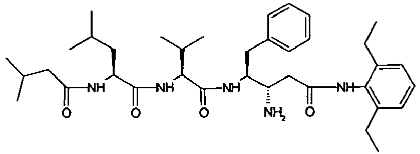 | | | | | | | |
| | (3S,4S)-3-Amino-4-{(S)-3-methyl-2-[(S)-4-methyl-2-(3-methyl-butyrylamino)-pentanoylamino]-butyrylamino}-5-phenyl-pentansäure-(2,6-diethyl-phenyl)-amid | 635,89 | 636,89 | 2,12 | >30µm | | 2.30E-07 | |
| 39 | 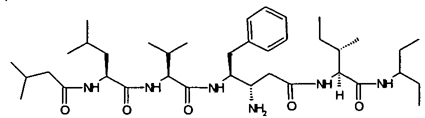 | | | | | | | |
| | (S)-3-(S)-Amino-4-{(S)-3-methyl-2-[(S)-4-methyl-2-(3-methyl-butyrylamino)-pentanoylamino]-butyrylamino}-5-phenyl-pentansäure-[(1S,2S)-1-(1-ethyl-propylcarbamoyl)-2-methyl-butyl]-amid | 686,98 | 687,98 | 2,06 | >30µM | stabil | 9,90E-09 | 2,33E-10 |
| 40 | 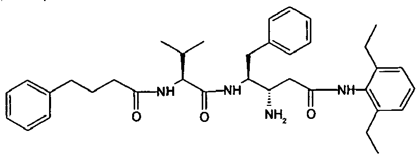 | | | | | | | |
| | (3S,4S)-3-Amino-4-[(S)-3-methyt-2-(4-phenyl-butyrylamino)-butyrylamino]-5-phenyl-pentansäure-(2,6-diethyl-phenyl)-amid | 584,80 | 585,80 | 2,07 | >30µM | | 8.00E-07 | |
| 41 | 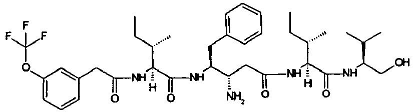 | | | | | | | |
| | 3-(S)-Amino-4-{(S)-(2S,3S)-3-methyl-2-[2-(3-trifluoromethoxy-phenyl)-acetylamino]-pentanoylamino}-5-phenyl-pentansäure{(2S,3S)-1-[1-((S)-hydroxymethyl)-2-methyl-propylcarbamoyl]-2-methyl-butyl}-amid | 721,86 | 722,86 | 2,15 | >30µM | | 4,80E-08 | |
| 42 | 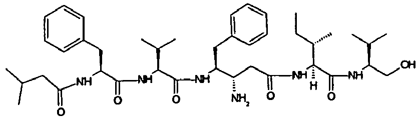 | | | | | | | |
| | (S)-3-(S)-Amino-4-{(S)-3-methyl-2-[(S)-2-(3-methyl-butyrylamino)-3-phenyl-propionylamino]-butyrylamino}-5-phenyl-pentansäure-[(1S,2S)-1-((S)-1-hydroxymethyl-2-methyl-propylcarbamoyl)-2-methyl-butyl]-amid | 736,99 | 737,99 | 2,10 | | | 3,60E-09 | |
| 43 | 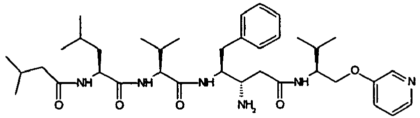 | | | | | | | |
| | (3S,4S)-3-Amino-4-{(S)-3-methyl-2-[(S)-4-methyl-2-(3-methyl-butyrylamino)-pentanoylamino]-butyrylamino}-5-phenyl-pentansäure-[(S)-2-methyl-1-(pyridin-3-yloxymethyl)-propyl]-amid | 666,90 | 667,90 | 1,72 | >30µM | | 2,40E-07 | |
| 44 | 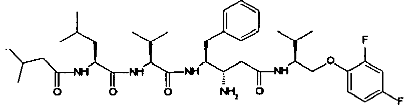 | | | | | | | |
| | (3S,4S)-3-Amino-4-{(S)-3-methyl-2-[(S)-4-methyl-2-(3-methyl-butyrylamino)-pentanoylamino]-butyrylamino}-5-phenyl-pentansäure-[(S)-1-(2,4-difluoro-phenoxymethyl)-2-methyl-propyl]-amid | 701,89 | 702,89 | 2,21 | >30µM | | 3,90E-08 | |
| 45 | 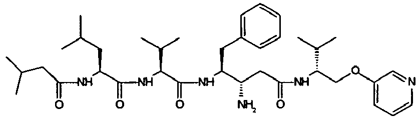 | | | | | | | |
| | (3S,4S)-3-Amino-4-{(S)-3-methyl-2-[(S)-4-methyl-2-(3-methyl-butyrylamino)-pentanoylamino]-butyrylamino}-5-phenyl-pentansäure-[(S)-1-(2,4-difluoro-phenoxymethyl)-2-methyl-propyl]-amid | 666,90 | 667,90 | 1,73 | >30µM | | 2,80E-07 | |
| 46 | 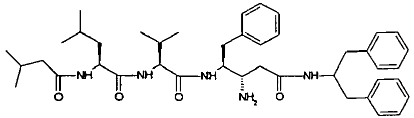 | | | | | | | |
| | (3S,4S)-3-Amino-4-{(S)-3-methyl-2-[(S)-4-methyl-2-(3-methyl-butyrylamino)-pentanoylamino]-butyrylamino}-5-phenyl-pentansäure(1-benzyl-2-phenyl-ethyl)-amid | 697,96 | 698,96 | 2,2 | | | | |
| 47 | 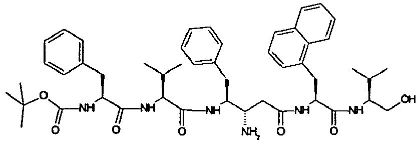 | | | | | | | |
| | [(S)-1-((S)-1-{(1S,2S)-2-Amino-1-benzyl-3-[(S)-1-((S)-1-hydroxymethyl-2-methyl-propylcarbamoyl)-2-naphthalen-1-yl-ethylcarbamoyl]-propylcarbamoyl}-2-methyl-propylcarbamoyl)-2-phenyl-ethyl]-carbamoylsäure tert-butyl ester | 837,07 | 838,07 | 2,16 | | | | |
| 48 | 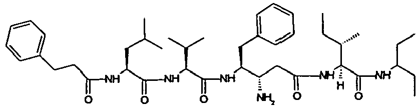 | | | | | | | |
| | (S)-3-(S)-Amino-4-{(S)-3-methyl-2-[(S)-4-methyl-2-(3-phenyl-propionylamino)-pentanoylamino]-1-oxo-butylamino}-5-phenyl-pentansäure [(1S,2S)-1-(1-ethyl-propylcarbamoyl)-2-methyl-butyl]-amid | 735,02 | 736,02 | 2,1 | | | | |
| 49 | 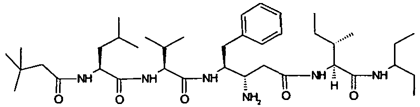 | | | | | | | |
| | (S)-3-(S)-Amino-4-{(S)-2-[(S)-2-(3,3-dimethyl-butyrylamino)-4-methyl-pentanoylamino]-3-methyl-1-oxo-butylamino)-5-phenyl-pentansäure [(1S,2S)-1-(1-ethyl-propylcarbamoyl)-2-methyl-butyl]-amid | 701,00 | 702,00 | 2,07 | | | | |
| 50 | 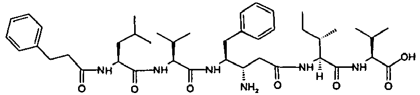 | | | | | | | |
| | (S)-2-[(2S,3S)-2-((S)-3-(S)-Amino-4-{(S)-3-methyl-2-[(S)-4-methyl-2-(3-phenyl-propionylamino)-pentanoylamino]-1-oxo-butylamino}-5-phenyl-pentanoylamino)-3-methyl-pentanoylamino]-3-methyl-buttersäure | 765,00 | 766,00 | 1,97 | | | | |
| 51 | 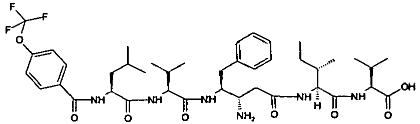 | | | | | | | |
| | (S)-2-[(2S,3S)-2-((S)-3-(S)-Amino-4-{(S)-3-methyl-2-[(S)-4-methyl-2-(4-trifluoromethoxy-benzoylamino)-pentanoylamino]-1-oxo-butylamino}-5-phenyl-pentanoylamino)-3-methyl-pentanoylamino]-3-methyl-buttersäure | 820,95 | 821,95 | 2,04 | | | | |
| 52 | 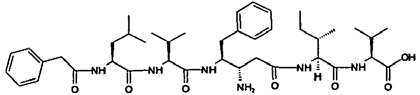 | | | | | | | |
| | (S)-2-((2S,3S)-2-{(S)-3-(S)-Amino-4-[(S)-3-methyl-2-((S)-4-methyl-2-phenylacetylamino-pentanoylamino)-1-oxo-butylamino]-5-phenyl-pentanoylamino}-3-methyl-pentanoylamino)-3-methyl-buttersäure | 750,98 | 751,98 | 1,94 | | | | |
| 53 | 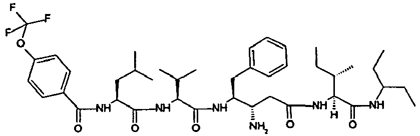 | | | | | | | |
| | N-[(S)-1-(1-{(S)-(S)-2-(S)-Amino-1-benzyl-3-[(1S,2S)-1-(1-ethyl-propylcarbamoyl)-2-methyl-butylcarbamoyl]-propylcarbamoyl}-2-methyl-propylcarbamoyl)-3-methyl-butyl]-4-trifluoromethoxy-benzamid | 790,96 | 791,96 | 2,15 | | | | |
| 54 | 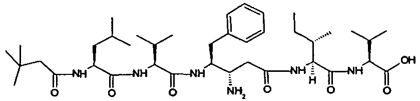 | | | | | | | |
| | (S)-2-[(2S,3S)-2-((S)-3-(S)-Amino-4-{(S)-2-[(S)-2-(3,3-dimethyl-butyrylamino)-4-methyl-pentanoylamino]-3-methyl-1-oxo-butylamino}-5-phenyl-pentanoylamino)-3-methyl-pentanoylamino]-3-methyl-buttersäure | 730,99 | 731,99 | 1,99 | | | | |
| 55 | 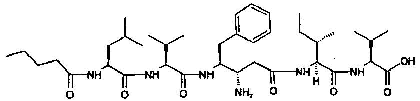 | | | | | | | |
| | (S)-2-((2S,3S)-2-{(S)-3-(S)-Amino-4-[(S)-3-methyl-2-((S)-4-methyl-2-pentanoylamino-pentanoylamino)-1-oxo-butylamino]-5-phenyl-pentanoylamino}-3-methyl-pentanoylamino)-3-methyl-buttersäure | 716,96 | 717,96 | 2,03 | | | | |
| 56 | 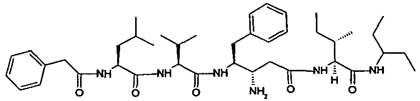 | | | | | | | |
| | (S)-3-(S)-Amino-4-[(S)-3-methyl-2-((S)-4-methyl-2-phenytacetylamino-pentanoylamino)-1-oxo-butylamino]-5-phenyl-pentansäure[(1S,2S)-1-(1-ethyl-propylcarbamoyl)-2-methyl-butyl]-amid | 720,99 | 721,99 | 2,09 | | | | |
| 57 | 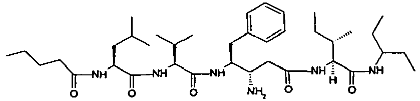 | | | | | | | |
| | (S)-3-(S)-Amino-4-[(S)-3-methyl-2-((S)-4-methyl-2-pentanoylamino-pentanoylamino)-1-oxo-butylamino]-5-phenyl-pentansäure [(1S,2S)-1-(1-ethyl-propylcarbamoyl)-2-methyl-butyl]-amid | 686,98 | 687,98 | 2,17 | | | | |

Um möglichen Zweifeln vorzubeugen wird überall, wo zu einer erfindungsgemäßen Verbindung die chemische Bezeichnung der Verbindung und die Abbildung der chemischen Struktur der Verbindung fälschlicherweise nicht übereinstimmen, die erfindungsgemäße Verbindung eindeutig durch die Abbildung der chemischen Struktur definiert.

### Beispiel 2: Herstellung der erfindungsgemäßen Verbindungen

Die erfindungsgemäßen Verbindungen sind z. B. nach dem Fachmann bekannten Methoden durch folgende Synthesesequenzen darstellbar. Die angegebenen Beispiele beschreiben die Synthese.

### Synthesesequenz:

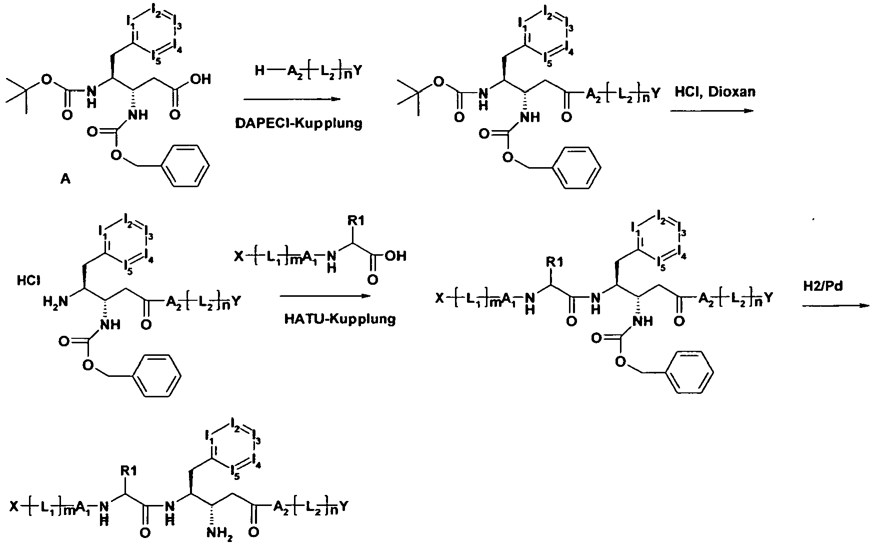

Ausgehend von dem geschützten Aminostatinbaustein A erfolgt der Aufbau der neuen Peptidbindung mit entsprechenden Aminen, Aminosäurederivaten, Dipeptiden, Tripeptiden oder Tetrapeptiden (typischerweise am C-Terminus geschützt) mit Hilfe einer dem Fachmann bekannten Kupplungsmethode, wie zum Beispiel einer DAPECI-Kupplung. Im zweiten Schritt wird die BOC-Schutzgruppe unter geeigneten Bedingungen abgespalten (z.B. durch HCl/Dioxan oder TFA/DCM) und der erhaltene Baustein mit einer entsprechende Säure, Aminosäurederivaten, Dipeptiden, Tripeptiden oder Tetrapeptiden (typischerweise jeweils am N-Terminus geschützt) gekuppelt. Besonders bevorzugt sind bei diesem Schritt Kupplungsreagenzien, die geeignet sind eine Racemisierung zu unterdrücken wie z.B. HATU oder ähnliche Reagenzien. Im letzten Schritt erfolgt die Abspaltung der Schutzgruppe an der Aminogruppe des Statins durch geeignete Methoden - im Falle einer Z-Schutzgruppe z.B. durch Hydrogenolyse in Gegenwart von Pd/C. Weitere Z-Schutzgruppen, z.B. Carbonsäurebenzylester in anderen Molekülteilen werden unter diesen Bedingungen ebenfalls abgespalten und liefern z.B. im Falle eines Benzylesters die freie Carbonsäure.

Herstellung von (A31): (S)-2-[(2S,3S)-2-((3S,4S)-3-Amino-4-{(S)-3,3-dimethyl-2-[(S)-4-methyl-2-(3-methyl-butyrylamino)-pentanoylamino]-butyrylamino}-5-phenyl-pentanoylamino)-3-methyl-pentanoylamino]-3-methyl-buttersäuremethylester

### Schritt 1

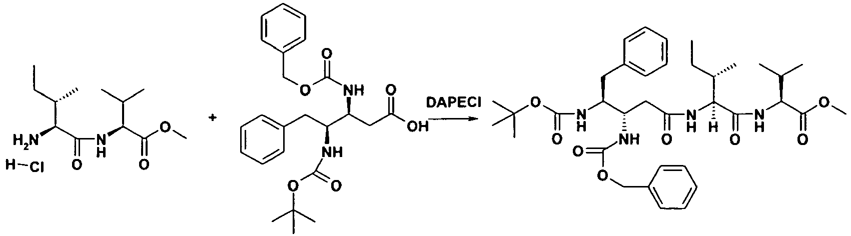

In einem Kolben wurden unter Eiskühlung 2,00 g (S)-2-((2S,3S)-2-Amino-3-methyl-pentanoylamino)-3-methyl-buttersäuremethylester (Hydrochloride), 2,53 g (3S,4S)-3-Benzyloxycarbonylamino-4-tert-butoxycarbonylamino-5-phenyl-pentansäure, 0,38 g 1-Hydroxybenzotriazolhydrat, 1,24 ml 4-Methylmorpholin und 1,19 g N-(3-Dimethylaminopropyl)-N'-ethylcarbodiimidhydrochlorid (DAPECI) in ca 25 ml DMF gelöst und über Nacht gerührt. Die Reaktionsmischung wurde auf gesättigte Natriumhydrogencarbonatlösung gegossen und 15 Min gerührt. Der entstandene Niederschlag wurde abgesaugt, mit verdünnter Zitronensäurelösung und Wasser gewaschen und getrocknet. Man erhält 3,80 g (S)-2-[(2S,3S)-2-((4S,5S)-3-Benzyloxycarbonylamino-4-tert-butoxycarbonylamino-5-phenyl-pentanoylamino)-3-methyl-pentanoylamino]-3-methyl-butersäuremethylester als weißen Feststoff. (Ausbeute 94,4%, Gehalt 94%). MS-FAB (M + H⁺- BOC) = 569,7 R_{f} (Methode polar): 2,67 Min. Analog zu diesem Schritt können z.B. die folgenden Verbindungen hergestellt werden.

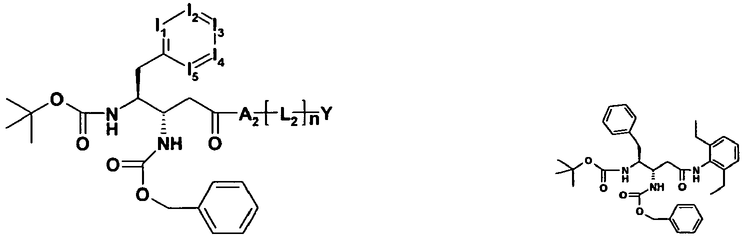

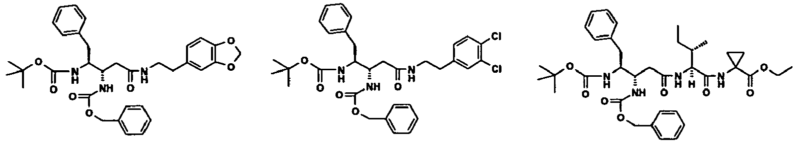

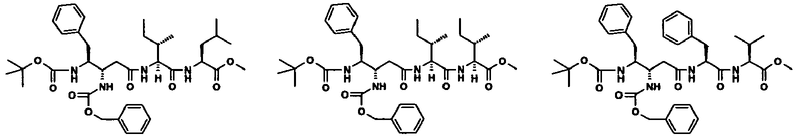

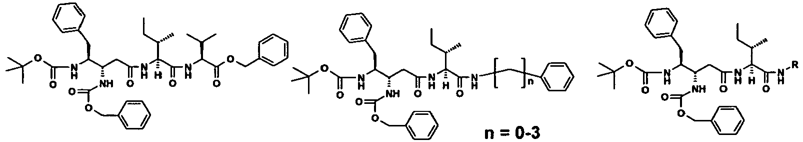

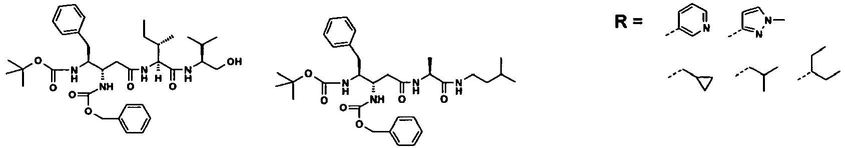

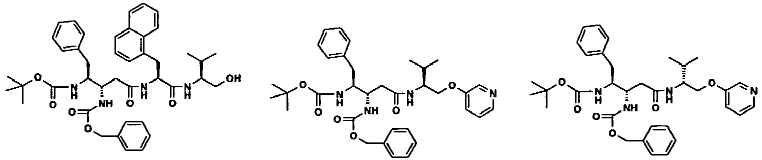

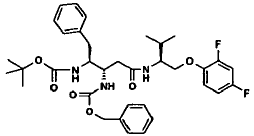

### Schritt 2

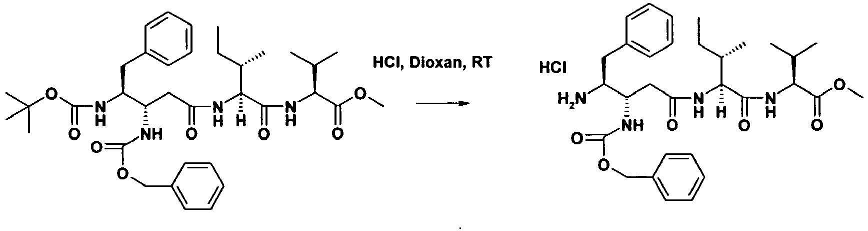

In einem Kolben wurden (S)-2-[(2S,3S)-2-((3S,4S)-3-Benzyloxycarbonylamino-4-tert-butoxycarbonylamino-5-phenyl-pentanoylamino)-3-methyl-pentanoylamino]-3-methyl-butansäuremethylester (2,76 g; 4,04 mmol) in 10 ml HCl-Lösung in Dioxan (4 M) gelöst und 5h bei RT gerührt. Überschüssige HCl wurde am Hausvakuum entfernt, das Lösungsmittel im Vakuum entfernt und der Rückstand über Nacht lyophilisiert.

Man erhält 2,4 g (S)-2-[(2S,3S)-2-((3S,4S)-4-Amino-3-benzyloxycarbonylamino-5-phenyl-pentanoylamino)-3-methyl-pentanoylamino]- 3-methyl-butersäuremethylester als weißen Feststoff. (Ausbeute 92,2%, Gehalt 94%). MS-FAB (M+H⁺) = 569,3 R_{f} (Methode polar): 1,67 Min.

### Schritt 3

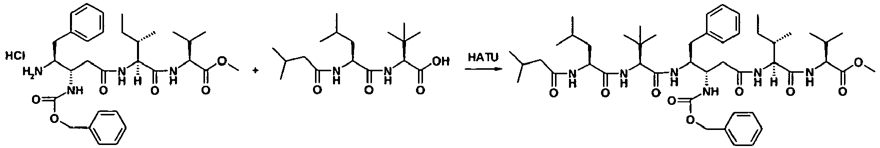

In einem Kolben wurden unter Eiskühlung 300 mg (S)-2-[(2S,3S)-2-((3S,4S)-4-Amino-3-benzyloxycarbonylamino-5-phenyl-pentanoylamino)-3-methyl-pentanoylamino]-3-methyl-buttersäuremethylester (Hydrochloride), 170,1 mg (S)-3,3-Dimethyl-2-[(S)-4-methyl-2-(3-methyl-butyrylamino)-pentanoylamino]-buttersäure und 163,4 µl Ethyldiisopropylamin in ca. 10 ml DMF gelöst, mit 197,0 mg HATU (C10H15N6O*PF6) versetzt und über Nacht bei RT gerührt.

Die gelbe Reaktionsmischung wurde auf gesättigte Natriumhydrogencarbonatlösung gegossen und 15 Min gerührt. Der entstandene Niederschlag wurde abgesaugt, mit verdünnter Ameisensäure verrieben, erneut abgesaugt, mit Wasser gewaschen und getrocknet. Man erhält 340 mg (S)-2-[(2S,3S)-2-((3S,4S)-3-Benzyloxycarbonylamino-4-{(S)-3,3-dimethyl-2-[(S)-4-methyl-2-(3-methyl-butyrylamino)-pentanoylamino]-butyrylamino}-5-phenyl-pentanoylamino -3-methyl-pentanoylamino]-3-methyl-buttersäuremethylester als weißen Feststoff. (Ausbeute 82%). MS-FAB (M + H⁺)= 880,1 R_{f} (Methode polar): 2,91 Min.

Analog zu diesem Schritt können die in Schritt 1 angegebenen Verbindungen z.B. mit folgenden Bausteinen umgesetzt werden.

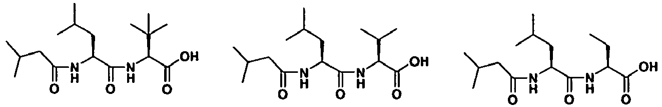

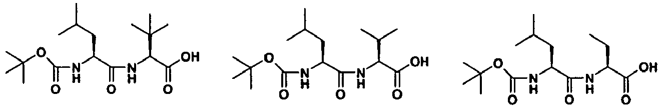

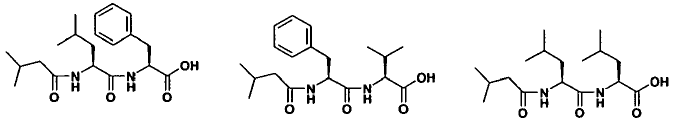

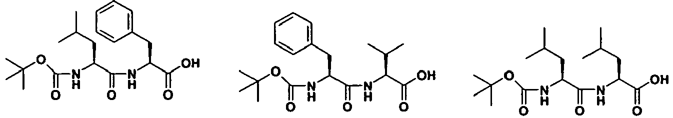

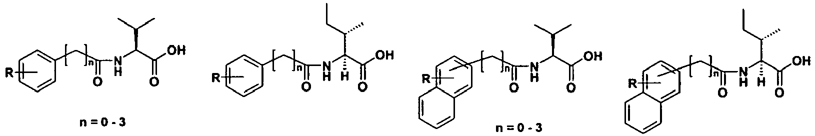

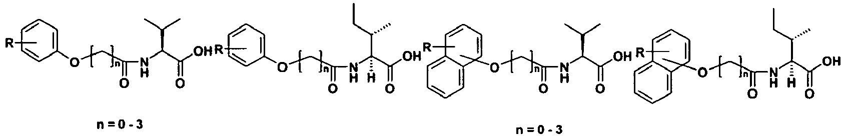

An diesen Schritt können sich weitere Folgereaktionen wie z.B. Abspaltungen von BOC-Schutzgruppen anschließen, bevor der nächste Schritt durchgeführt wird.

### Schritt 4

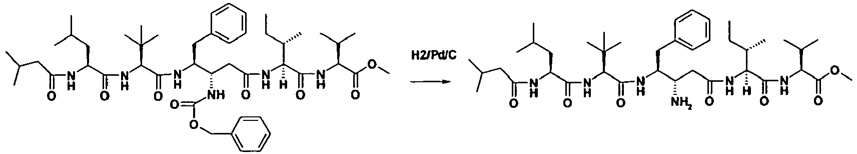

In einem geeigneten Gefäß wurden 300 mg (S)-2-[(2S,3S)-2-((3S,4S)-3-Benzyloxycarbonylamino-4-{(S)-3,3-dimethyl-2-[(S)-4-methyl-2-(3-methyl-butyrylamino)-pentanoylamino]-butyrylamino}-5-phenyl-pentanoylamino)-3-methyl-pentanoylamino]-3-methyl-buttersäuremethylester in Gegenwart von 300 mg Pd/C (5%Pd) in 30 ml THF bei RT so lange bei Normaldruck hydriert, bis das Edukt vollständig umgesetzt war (über Nacht). Das Lösungsmittel wurde im Vakuum entfernt, der Rückstand mit MTBE verrieben und abgesaugt. Reinigung durch präparative HPLC (Chromolith®prep, RP18e, 100-25; Gradient Wasser:Acetonitril 1-60% in 12 Min) und anschließende Lyophilisierung ergab 89 mg (S)-2-[(2S,3S)-2-((3S,4S)-3-Amino-4-{(S)-3,3-dimethy)-2-[(S)-4-methy)-2-(3-methy)-butyrylamino)-pentanoylamino]-butyrylamino}-5-phenyl-pentanoylamino)-3-methyl-pentanoylamino]-3-methyl-buttersäuremethylester als weißes Pulver (Ausbeute 31%). MS-FAB (M + H⁺)= 746,1 R_{f} (Methode polar): 2,02 Min.

Über diese Synthesesequenz lassen sich z.B. die Verbindungen Nr. 1-45 (siehe Tabelle in Beispiel 1) herstellen.

Sind mehrere orthogonale Schutzgruppen vorhanden (z.B. BOC und Z) lässt sich die Reihenfolge der Schutzgruppenabspaltungen auch umkehren, so dass z.B. die Z-Abspaltung vor der BOC-Abspaltung durchgeführt wird.

### Methode (HPLC-MS):

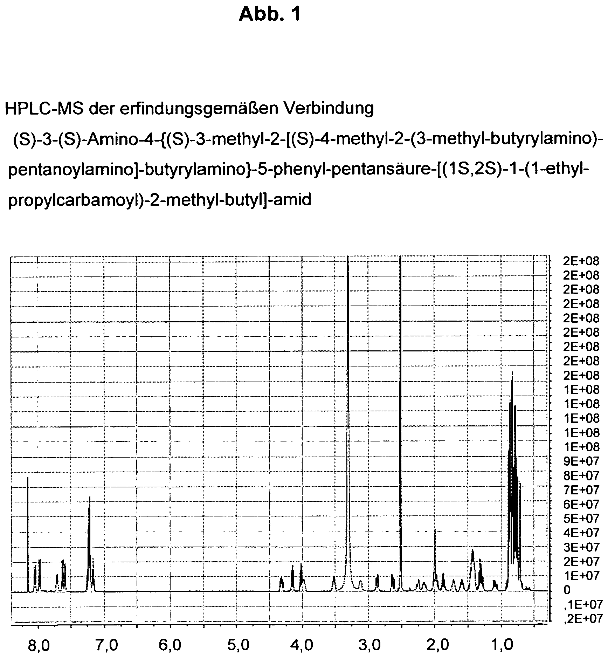

Chromolith Speed Rod RP 18e 50-4,6 mm LCMS; Polar.m, 2,4ml/min, 220nm, Puffer A 0,05% HCOOH/H2O, Puffer B 0,04% HCOOH/ACN, 0,0-2,8min 4%-100% Puffer B; 2,8-3,3min 100% Puffer B 3,3-3,4min 100%-4% Puffer B (siehe Abbildung 1)

### Abkürzungen:

DCM = Dichlormethan
DMA = Dimethylacetamid
DMF = Dimethylformamid
EE = Essigsäureethylester
MTBE = Methyl-t*ert*-butylether
PE = Petrolether
RT = Raumtemperatur
TFA = Trifluoressigsäure

### Beispiel 3: In-vitro Fluoreszenz-Assay zur Identifizierung von Cathepsin D Inhibitoren

Zur Identifizierung von Modulatoren der Cathepsin D Aktivität wurde ein kontinuierlicher enzymatischer Test mit einem synthetischen Peptid, das eine fluoreszierende Gruppe (MCA=(7-methoxycoumarin-4-yl)acetyl) trägt, die durch Energietransfer von einer Dpn (2,4 dinitrophenyl)-Gruppe am selben Molekül gequencht ist, in 384-well-nb-Mikrotiterplatten von Greiner durchgeführt. Die Spaltung des peptidischen Substrats durch Cathepsin D bewirkt einen Anstieg in der Fluoreszenzintensität. Zur Bestimmung der Wirksamkeit von Substanzen wurde der zeitabhängige Anstieg in der Fluoreszenzintensität in Gegenwart der Substanz mit der zeitabhängigen Fluoreszenzzunahme in Abwesenheit von Substanzen verglichen. Als Referenzsubstanz diente Pepstatin A (Sigma-Aldrich). Als Substrat wurde MCA-GKPILFFRLK(Dnp)d-R-NH₂ (Enzo Life Sciences, Lörrach) verwendet. Als Enzym wurde aus der humanen Leber isoliertes Cathepsin D (Sigma-Aldrich) in einer finalen Konzentration von 1.4 nM eingesetzt. Der Test wurde in 100 mM Natrium-Acetat-Puffer, 1.25 % (v/v) DMSO, 0.25 % (w/v) Chaps, pH 5.5 durchgeführt. Zu je 4 µl Cathepsin D-Lösung wurden je 2 µl Substanzlösung mit seriell verdünnter Substanzkonzentration zugegeben und 10 min bei Raumtemperatur inkubiert. Die Reaktion wurde gestartet durch Zugabe von 2 µl Substrat-Lösung (finale Konzentration 5 µM). Nach Durchführung einer Startpunkt-Fluoreszenzmessung (Anregungswellenlänge 340 nm/Emissionswellenlänge 450 nm) mit einem Envision Multilabel reader (Perkin Elmer) wurde die Reaktion 60 min bei Raumtemperatur inkubiert. Anschließend wurde die Menge des während der Reaktionszeit abgespaltenen Peptidfragments durch Bestimmung der Zunahme der Fluoreszenzintensität bei 450 nm (Anregungswellenlänge 340 nm) gemessen.

Alle Verbindungen aus der Tabelle aus Beispiel 1 wiesen einen IC₅₀-Wert kleiner als 100nM auf (siehe Tabelle aus Beispiel 1, vorletzte Spalte).

### Beispiel 4: Knorpel-Explant-Assay

Um den Effekt von potentiellen Cathepsin D Inhibitoren auf Knorpelabbau zu untersuchen wird ein pH-induziertes Modell basierend auf bovinen Explantaten verwendet. Dabei wird der pH-Wert des Mediums, in dem die Explantate kultiviert werden, dem pathophysiologischen pH-Wert eines arthrotischen Knies angepasst. Dieser pH-Wert liegt bei pH 5,5. In diesem *ex vivo* Modell werden anschließend potentielle Cathepsin D Inhibitoren auf ihre Wirkung hinsichtlich einer Arretierung des Knorpelabbauprozesses untersucht. Wird der Knorpel zerstört, werden Glycosaminoglycane (GAGs) in den Zellkulturüberstand abgegeben. Die Menge der freigesetzten GAGs lässt sich quantitativ mit Hilfe des DMMB (Dimethylmethylenblau-Hydrochlorid) bestimmen. Beim Nachweis sulfatierter GAGs mit Dimethylmethylenblau-Hydrochlorid macht man sich die Abnahme der Absorption bei 633 nm zu Nutze. Da auch bei sehr niedrigen GAG-Konzentrationen gearbeitet werden kann, fällt auch nach langer Inkubation von DMMB mit GAG kein Farbstoff/GAG-Komplex aus, wie das bei anderen Messmethoden mitunter schon nach kurzer Zeit passiert. Zur Bestimmung der Konzentration wird eine Eichgerade mit Chondroitinsulfat mitgeführt. Anhand der GAG-Werte lässt sich ein IC₅₀-Wert errechnen, d.h. eine Konzentration bei der eine Substanz 50% ihrer Wirkung zeigt.

### Lösungen:

### Inkubationsmedium, pH 7,4:

DMEM ohne FBS, Zugabe von 1% Pen/Strep und 30 µg/ml Ascorbinsäure, das Medium wird nicht gelagert.

### Inkubationsmedium, pH 5,5:

DMEM ohne FBS, der pH-Wert wird durch Zugabe von MES eingestellt und am pH-Meter kontrolliert, Zugabe von 1% Pen/Strep und 30 µg/ml Ascorbinsäure.

### Lösungen für die GAG-Messung:

### DMMB-Färbelösung (V = 500 ml):

8 mg DMMB (Dimethylmethylenblau) in 2,5 ml Ethanol lösen + 1 g Natriumformiat + 1 ml Ameisensäure, mit bidest. Wasser ad 500 ml auffüllen
Inkubationsmedium: FBS (Medium ohne FBS)

### Chondroitinsulfat-Lösungen (Standardkurve)

Ansatz von Standardlösungen mit folgenden Konzentrationen: 50µg/ml; 25µg/ml; 12,5µg/ml; 6,25µg/ml; 3,125µg/ml; 1,56µg/ml; 0,78µg/ml sowie eine Leerkontrolle des Mediums. Der Ansatz der Standardlösung erfolgt in dem Medium, mit welchem auch der Versuch durchgeführt wurde.

### 1.) Durchführung: pH induzierter Knorpelabbau von bovinen Explantaten

Die bovinen Explantate werden zunächst präpariert. Die Induktion des Knorpelabbaus wird in 96 -Multiwellplatten durchgeführt. Dabei wird ein Explantat pro well kultiviert. Es erfolgt die Zugabe von je 200 µl DMEM (Inkubationsmedium pH 5,5) ohne FBS + 30 µg/ml Ascorbinsäure. Also Negativkontrolle werden Explantate (n= 4) bei pH 7,4 (ohne FBS) inkubiert. Diese Kontrolle geht nicht in die Berechnung der Daten mit ein, sondern stellt sicher dass die pH-Wert Änderung den gewünschten Effekt auf die Freisetzung von GAG hat. An dieser Stelle erfolgt die Zugabe der zu testenden Substanzen. Es findet keine Vorinkubation der Explantate statt.Die Explantate werden mit den entsprechenden Substanzen 3 Tage im Brutschrank bei 37°C und 7,5% CO₂ kultiviert.

### 2.) Inkubationsablauf

Um den Effekt von Cathepsin D Inhibitoren auf die Freisetzung von GAG (Glycosaminoglycan) zu untersuchen, werden die Substanzen in der gewünschten Konzentration eingesetzt und für 3 Tage kultiviert. Dabei werden die zu testenden Verbindungen in einem ersten Experiment in einer Konzentration von 1 µM und 1% DMSO getestet. Substanzen die einen Effekt von >50% auf die Freisetzung von GAG haben (das entspricht <50% der Kontrolle im Assay Explorer), werden in einem nächsten Experiment bei 100 nM und 1% DMSO gestestet. Substanzen, die unter diesen Bedingungen einen Effekt von >50% auf die Freisetzung von GAG haben (das entspricht <50% der Kontrolle im Assay Explorer), werden in einer Konzentrations-Wirkungs-Beziehung getestet. Dabei werden die Verbindungen in den folgenden Konzentrationen untersucht: 30 µM, 10 µM, 3 µM, 1 µM, 0.3 µM, 0.1 µM, 0.03 µM, 0.01 µM.

Als Positivkontrolle wird Pepstatin A mit einer Konzentration von 0,01 µM verwendet. Das Wirkungsfenster (assay window) ist definiert durch die Kontrolle (pH 5,5), definiert als 0% Effekt und die Kontrolle pH 5,5 + 0,01 µM Pepstatin A, definiert als 100% Effekt. Nach 3 Tagen Inkubation werden die Zellkulturüberstände gesammelt und bei -20°C gelagert oder direkt vermessen. Dabei wird photometrisch die Menge an freigesetztem GAG gemessen.

Berichtet werden für Konzentrationen von 1 µM und 100 nM der Effekt (1 Wert) der jeweiligen Substanz in % bezogen auf die Positivkontrolle (pH 5,5 + 0,01 µM Pepstatin A) und die Negativkontrolle (pH 5,5). Der Wert stellt den Mittelwert aus 4 Replikaten da. Bei der Ermittlung einer Konzentrations-Wirkungs-Beziehung wird ein IC₅₀-Wert an die Datenbank berichtet (Assay Explorer).

### 4.) Messung

Die Zellkulturüberstände (200 µl) werden entweder direkt vermessen oder werden bei -20°C gelagert. Eine genaue Bestimmung der Konzentration (µg/ml GAG im Überstand) von GAG zu gewährleisten müssen sich die Messwerte im linearen Bereich der Standardkurve befinden. Um dies zu gewährleisten werden routinemäßig verschiedene Verdünnungen vorgelegt (1/5, 1/10, 1/20, 1/40). Die Verdünnungen werden mit Medium hergestellt und automatisiert (Hamilton) in einer 384-Multiwellplatte vorgelegt (15 µl). Ebenfalls automatisiert (oder mit Mehrkanalpipette) werden 60 µl DMMB-Lösung zugegeben. Es erfolgt eine schnelle Farbreaktion die anschließend bei 633 nm mit einem Platten-Reader (z.B. Envision) gemessen wird. Je nach vorhandener Probenmenge wird mindestens eine Doppelbestimmung durchgeführt.

Die Daten werden vom MTP-Reader als csv oder xls-Files zur Verfügung gestellt und basierend auf diesem Format (xls) als Rohdaten gespeichert bzw. zur Berechnung des prozentualen Effekts der jeweiligen Verbindung herangezogen.

### 5.) Qualitätskontrollen

Als Kontrolle für die Induktion des pH-induzierten Knorpelabbaus werden 4 Explantate bei pH 7,4 inkubiert. Dies entspricht dem physiologischen pH-Wert des Knorpels und somit ist hier kein Effekt auf die Freisetzung von GAG zu erwarten. Diese GAG Werte (µg/ml Überstand) sind somit immer signifikant niedriger als die GAG-Werte einer Inkukation mit pH 5,5.

Eine weitere Kontrolle, die sowohl der Überprüfung des Experimentes dient aber auch wichtig für die Definition des Wirkungsfensters ist, ist die Pepstatin-Kontrolle (pH 5,5 + 0,01 µM Pepstatin A). Diese Substanz blockiert unspezifisch die Aktivität der meisten Proteasen und legt somit den maximal möglichen Effekt einer Verbindung fest.
(1) Klompmakers, A. & Hendriks, T. (1986) Anal. Biochem. 153, 80-84, Spektrophotometrischer Nachweis für sulfatierte Glycosaminoglycane.
(2) Groves, P.J. et al. (1997) Anal. Biochem. 245, 247-248 Polyvinylalkohol-stabilisierte Bindung von sulfatierten GAGs an Dimethylmethylenblau.

### 6.) Ergebnisse

zu einigen Verbindungen aus der Tabelle in Beispiel 1 wurden mit diesem Assay IC₅₀-Werte ermittelt, diese sind in der Tabelle in Beispiel 1 in der letzten Spalte aufgeführt.

### Beispiel 5: Untersuchung anti-hyperalgetischer Wirkung im Tier

Zur Induktion einer Entzündungsreaktion wurde eine Carrageenan-Lösung (CAR, 1%, 50 µl) einseitig intraartikulär in ein Rattenkniegelenk injiziert. Die nichtinjizierte Seite wurde zu Kontrollzwecken herangezogen. Es wurden sechs Tiere pro Gruppe verwendet. Die Schwellung wurde mittels einer Mikrometerschraube bestimmt (medial-lateral am Kniegelenk) und die thermische Hyperalgesie mittels einer gerichteten Infrarotlichtquelle nach der Hargreaves-Methode (Hargreaves et al. 1988) an der Fußunterseite bestimmt. Da der Ort der Entzündung (Kniegelenk) von dem Ort der Messung (Pfotenunterseite) abweicht, spricht man hier von sekundärer thermischer Hyperalgesie, deren Mechanismen für die Auffindung wirksamer Analgetika von Bedeutung ist.

Versuchsbeschreibung Thermische Hyperalgesie (Hargreaves Test): Das Versuchstier wird in einer Plastikkammer auf eine Quarzglasscheibe gesetzt. Vor der Testung wird dem Versuchstier zunächst etwa 5 - 15 Minuten Zeit gegeben, sich an die Umgebung zu gewöhnen. Sobald sich das Versuchstier nach der Erkundungsphase nicht mehr so oft bewegt (Ende der Explorationsphase), wird die Infrarotlichtquelle, deren Fokus in der Ebene des Glasbodens liegt, direkt unterhalb der zu stimulierenden Hinterpfote positioniert. Ein Versuchsdurchlauf wird nun durch Knopfdruck gestartet: Infrarotlicht führt zur Erhöhung der Hauttemperatur der Hinterpfote. Der Versuchablauf wird entweder bei Anheben der Hinterpfote durch das Versuchstier (als Ausdruck des Erreichens der Schmerzschwelle) oder bei Erreichen einer vorgegeben Höchsttemperatur durch automatisches Ausschalten der Infrarotlichtquelle beendet. Solange das Versuchstier still sitzt, wird Licht, das von der Pfote reflektiert wird, registriert. Ein Wegziehen der Pfote unterbricht diese Reflektion, woraufhin die Infrarotlichtquelle abgeschaltet und die Zeit von An- bis Abschalten registriert wird. Das Gerät ist so geeicht, dass die Infrarotlichtquelle in 10s die Hauttemperatur auf ca. 45 Grad Celsius erhöht (Hargreaves et al. 1988). Zur Testung wird ein von der Firma Ugo Basile zu diesem Zweck produziertes Gerät benutzt.

CAR wurde von Sigma-Aldrich bezogen. Die Applikation des spezifischen Cathepsin D-Inhibitors, der Verbindung Nr. 23 (aus Beispiel 1, Tabelle 1, (S)-2-[(2S,3S)-2-((3S,4S)-3-Amino-4-{(S)-3-methyl-2-[(S)-4-methyl-2-(3-methyl-butyrylamino)-pentanoylamino]-butyrylamino}-5-phenyl-pentanoylamino)-3-methyl-pentanoylamino]-3-methyl-buttersäure) wurde 30 Minuten vor der CAR intraartikulär vorgenommen. Als Positivkontrolle wurde Triamcinolone (TAC) 10 µg/Gelenk und als Negativkontrolle wurde das Lösungsmittel (Vehikel) verwendet. Die Hyperalgesie wird als Differenz der Wegziehzeiten zwischen der entzündeten und der nicht-entzündeten Pfote angegeben.

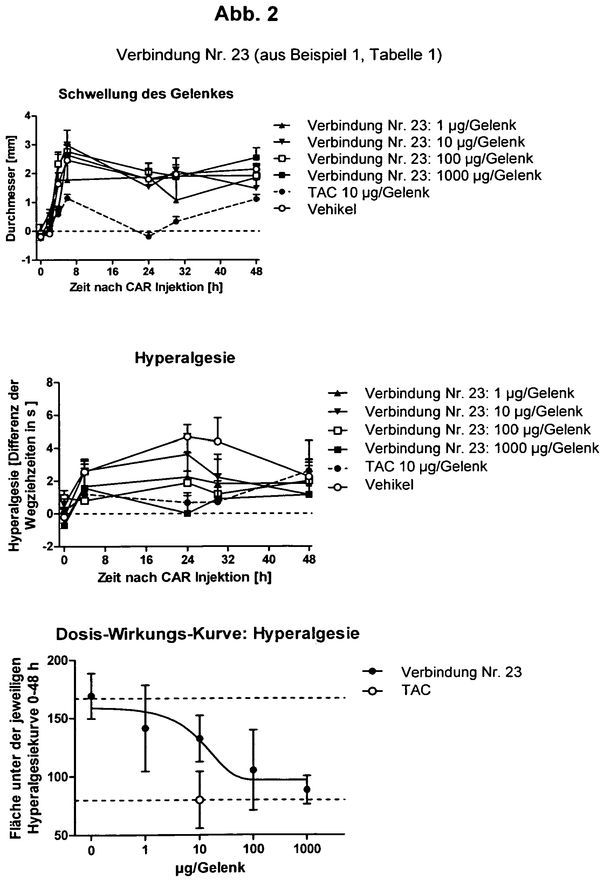

Ergebnis: TAC war in der Lage, die CAR-induzierte Schwellung zu reduzieren, nicht aber der spezifische Cathepsin D-Inhibitor. Im Gegensatz dazu konnte der spezifische Cathepsin D-Inhibitor das Ausmaß der thermischen Hyperalgesie dosisabhängig reduzieren (siehe Abbildung 2) Beurteilung: Es konnte gezeigt werden, dass die Verbindung Nr. 23 eine anti-hyperalgetische Wirkung ausübt. Dies kann postuliert werden, da die Verbindung Nr. 23 keinen Einfluss auf die entzündliche Schwellung und damit auf den Auslöser der Hyperalgesie zeigte. Es kann damit angenommen werden, dass die Verbindung Nr. 23 beim Menschen eine Schmerz reduzierende Wirkung entfaltet.

### Beispiel 6: Stabilität der erfindungsgemäßen Verbindungen in boviner Synovialflüssigkeit

### 1.) Gewinnung von boviner Synovialflüssigkeit

Bei der Präparation von bovinen Explantaten (für die Diffusionskammer oder andere Assays) werden entweder Rinderhufe (metacarpale Gelenke) oder Rinderknie verwendet. Die Synovialflüssigkeit lässt sich aus beiden Gelenken gewinnen. Dazu wird bei der Öffnung des Gelenkes die Synovialflüssigkeit mit einer 10 ml Spritze und einer Kanüle vorsichtig aus dem Gelenk entfernt und in bereit gestellte 2 ml Eppendorfgefäße gefüllt. Die Eppendorfgefäße sind je nach Tier beschriftet (Rinderpass liegt vor). Dabei ist darauf zu achten, dass bei der Präparation der Gelenke kein Blut in den Gelenkspalt eindringt. Ist dies der Fall, verfärbt sich die Synovialflüssigkeit rötlich und muss somit verworfen werden. Die Synovialflüssigkeit ist grundsätzlich hochviskos und klar bis gelblich gefärbt. Die Entnahme zusammen mit einer makroskopischen Analyse der Synovialflüssigkeit wird dokumentiert.

### 2.) Ansatz der Stabilitätsprüfung von Substanzen in SF

Um die Stabilität einzelner Verbindungen zu überprüfen wird ein Pool aus 4 verschiedenen bovinen Synovialflüssigkeiten gemischt. Dazu werden ca. 1 ml je SF verwendet. Das Gemisch wird direkt in einem 5 ml Glasgefäß angesetzt. Die SFs werden gründlich aber vorsichtig gemischt. Dabei sollten keine Luftblasen oder Schaum entstehen. Dazu wird ein Vortex-Gerät auf niedrigster Stufe verwendet. Die zu testenden Verbindungen werden in einer Anfangskonzentration (sofern nicht anders angefordert) von 1 µM getestet. Nach Zugabe der Substanz erfolgt eine erneute gründliche und vorsichte Durchmischung des Ansatzes. Zur optischen Kontrolle werden alle SF-Ansätze fotografiert und die Bilder im eLabBio-Ordner des entsprechenden Versuchs abgelegt. Abbildung 1 zeit exemplarisch eine solche Fotodokumentation. Die Ansätze werden für 48 h bei 37°C und 7,5% CO₂ im Brutschrank inkubiert.

### 3.) Probenentnahme

Die Probenentnahme erfolgt nach den vorher abgestimmten Zeiten (sofern nicht anders angefordert, siehe unten). Dabei werden pro Zeitpunkt 200 µl der SF aus dem Gemisch entnommen und direkt in ein 0,5 ml "Low-binding" Eppendorf Gefäß überführt. "Low-binding" Eppendorf Gefäße werden verwendet um eine Wechselwirkung der Substanzen mit dem Kunststoff der Gefäße zu minimieren. In dem Eppendorfgefäß wurde bereits 200 µl Acetonitril vorgelegt, so dass danach eine 1 + 1 Mischung der SF entsteht. Dies erleichtert die folgende Analytik, es kann jedoch direkt nach Zugabe der SF zum Ausfallen vom Protein kommen. Dies ist im Protokoll zu vermerken. Direkt nach Zugabe der Substanz wird die 0 h Probe entnommen. Dies entspricht dem 100% Wert in der Stabilitätsberechnung. Idealerweise sollte sich hier die eingesetzte Konzentration wiederfinden. Die Proben können bei -20°C eingefroren werden.
- 0 h
- 6 h
- 24 h
- 48 h

Als Negativkontrolle wird SF ohne Substanz verwendet. Als Positivkontrolle wird SF mit 1µM Substanz verwendet. Dies entspricht dem 0h Wert und somit 100% Stabilität.

Die Lagerung der Proben erfolgt in "low-Binding" Eppendorf-Gefäßen bei - 20°C. Anschließend werden die Proben quantitativ vermessen.

### 4.) Datenverarbeitung

Die gemessenen Konzentrationen (ng/ml) werden in einer Graphik (GraphPad Prism®) als zeitlicher Verlauf dargestellt. Dabei wird die prozentuale Stabilität der Substanz ermittelt. Als 100% Wert wird der Ausgangswert in SF zum Zeitpunkt 0 h verwendet. Die Daten werden unter der jeweiligen Versuchsnummer in eLabBio gespeichert und in die MSR Datenbank (als Prozent Stabilität nach den entsprechenden Inkubationszeiten) berichtet.

### 5.) Ergebnisse

Alle gemessenen Verbindungen blieben stabil. (siehe Tabelle in Beispiel 1).

### Beispiel 7: In-vitro Fluoreszenz-Assay zur Identifizierung von Renin inhibitorischer Aktivität

Zur Identifizierung von Modulatoren der Renin Aktivität wurde ein kontinuierlicher enzymatischer Test mit einem synthetischen Peptid, das eine fluor7szierende Gruppe Edans (=(5-(aminoethyl)aminonaphthalene sulfonate) trägt, die durch Energietransfer von einer Dabcyl (4'-dimethylaminoazo-benzene-4-carboxylate)-Gruppe am selben Molekül gequencht ist, in 384-well-Mikrotiterplatten von Greiner durchgeführt. Die Spaltung des peptidischen Substrats durch Renin bewirkt einen Anstieg in der Fluoreszenzintensität. Zur Bestimmung der Wirksamkeit von Substanzen wurde der zeitabhängige Anstieg in der Fluoreszenzintensität in Gegenwart der Substanz mit der zeitabhängigen Fluoreszenzzunahme in Abwesenheit von Substanzen verglichen. Als Referenzsubstanz diente Renin inhibitor 2 (Z-Arg-Arg-Pro-Phe-His-Sta-Ile-His N-Boc-Lys methyl ester Z) (Sigma-Aldrich). Als Substrat wurde Renin FRET Substrate I (DABCYL - g - Abu - Ile - His - Pro - Phe - His - Leu - Val - Ile - His - Thr - EDANS) (Anaspec, Fremont CA) verwendet. Als Enzym wurde rekombinantes humanes Renin (Proteos, Kalamazoo, MI) in einer finalen Konzentration von 10 nM eingesetzt. Der Test wurde in 50 mM Mops-Puffer, 1.5 % (v/v) DMSO, 0.1 % (w/v) Igepal®, pH 7.2, 0.5 % (w/v) BSA durchgeführt. Zu je 4 µl Renin-Lösung wurden je 2 µl Substanzlösung mit seriell verdünnter Substanzkonzentration zugegeben und 15 min bei Raumtemperatur inkubiert. Die Reaktion wurde gestartet durch Zugabe von 4 µl Substrat-Lösung (finale Konzentration 5 µM). Nach Durchführung einer Startpunkt-Fluoreszenzmessung (Anregungswellenlänge 340 nm/Emissionswellenlänge 495 nm) mit einem Envision Multilabel reader (Perkin Elmer) wurde die Reaktion 60 min bei 37°C inkubiert. Anschließend wurde die Menge des während der Reaktionzeit abgespaltenen Peptidfragments durch Bestimmung der Zunahme der Fluoreszenzintensität bei 495 nm (Anregungswellenlänge 340 nm) gemessen.
Ergebnis: Alle gemessenen Verbindungen haben eine ICC50 der
Reninselektiviät >30µM (siehe Tabelle in Beispiel 1)

### Beispiel 8: Injektionsgläser

Eine Lösung von 100 g einer Verbindung der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg einer Verbindung der Formel I.

### Beispiel 9: Lösung

Man bereitet eine Lösung aus 1 g einer Verbindung der Formel I, 9,38 g NaH₂PO₄ 2 H₂O, 28,48 g Na₂HPO₄· 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel 10: Salbe

Man mischt 500 mg einer Verbindung der Formel I mit 99,5 g Vaseline unter aseptischen Bedingungen.

### Beispiel 11: Ampullen

Eine Lösung von 1 kg einer Verbindung der Formel I in 60 I zweifach destilliertem Wasser wird steril filtriert, in Ampullen abgefüllt, unter sterilen Bedingungen lyophilisiert und steril verschlossen. Jede Ampulle enthält 10 mg einer Verbindung der Formel I.

## Patentansprüche

1. Verbindungen der Formel I, 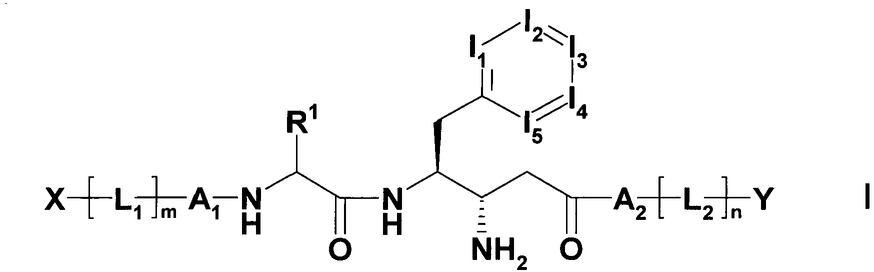 worin
I₁, I₂, I₃, I₄, I₅ unabhängig voneinander N oder CR',
T ein unsubstituiertes oder ein-, zwei- drei- oder vierfach durch R substituiertes Phenyl oder Naphthyl, oder ein ein- oder zweikerniger gesättigter, ungesättigter oder aromatischer Heterozyklus mit 1 bis 4 N-, O- und/oder S-Atomen, der ein-, zwei- oder dreifach durch R, =S, =NR' und/oder =O substituiert sein kann,
R¹ Ethyl, n-Propyl, iso-Propyl, Cyclopropyl, Butyl, iso-Butyl, sec-Butyl, Cyclobutyl, tert-Butyl, Phenyl, Benzyl, 2-Oxetanyl, 3-Oxetanyl, Tetrahydro-furan-3-yl, Tetrahydro-furan-2-yl, Cyclopentyl, Pentyl, Methylsulfanylmethyl, Ethylsulfanylmethyl, 2-Methylsulfanylethyl oder 1-Methylsulfanylethyl,
A₁ 0 bis 4 peptidartig miteinander verbundene Aminosäurereste, ausgewählt aus der Gruppe bestehend aus Alanin, Glycin, Cyclopropylglycin, Cyclobutylglycin, Cyclopentylglycin, Cyclohexylglycin, 3-Oxetanylglycin, 3-Oxetanylglycin, Tetrahydro-furan-3-ylglycin, Tetrahydro-furan-2-ylglycin, Ethylsulfanylmethylglycin, 2-Methylsulfanylethylglycin, 1-Methylsulfanylethylglycin, Valin, Norvalin, Aminobuttersäure, Leucin, Isoleucin, Prolin, tert-Leucin, Norleucin, Methionin, Phenylalanin, Naphthylalanin, O-Methyl-Serin, O-Ethyl-Serin, -OCO-,-NRCO-, -SO₂- und -NRSO₂- und
A₂ 0 bis 4 peptidartig miteinander verbundene Aminosäurereste, ausgewählt aus der Gruppe bestehend aus Alanin, Glycin, Cyclopropylglycin, Cyclobutylglycin, Cyclopentylglycin, Cyclohexylglycin, 3-Oxetanylglycin, 3-Oxetanylglycin, Tetrahydro-furan-3-ylglycin, Tetrahydro-furan-2-ylglycin, Ethylsulfanylmethylglycin, 2-Methylsulfanylethylglycin, 1-Methylsulfanylethylglycin, Valin, Norvalin, Aminobuttersäure, Leucin, Isoleucin, Prolin, tert-Leucin, Norleucin, Methionin, Phenylalanin, Naphthylalanin, O-Methyl-Serin und O-Ethyl-Serin
L₁ eine Einfachbindung oder -CRR'-,
L₂ eine Einfachbindung, -CRR'-, -NR-, -NRCR'R- oder-NRCRR'CRR'-,
X, Y H, T, unsubstituiertes oder ein-, zwei- oder dreifach durch =S, =NR, =O, R, T, OR, NRR', SOR, SO₂R, SO₂NRR', CN, COOR, CONRR', NRCOR', NRCONR'R", NRSO₂R' und/oder NRCOR' substituiertes, lineares oder verzweigtes Alkyl mit 1-10 C-Atomen, worin eine, zwei oder drei CH₂-Gruppen unabhängig voneinander durch O, S, SO, SO₂, NR, -OCO-, -NRCONR'-, -NRCO-,-NRSO₂R'-, -COO-, -CONR-, -C≡C-Gruppen und/oder durch -CH=CH-Gruppen und/oder auch 1-20 H-Atome durch F und/oder Cl ersetzt sein können, oder unsubstituiertes oder ein-, zwei- oder dreifach durch =S, =NR, =O, R, T, OR, NRR', SOR, SO₂R, SO₂NRR', CN, COOR, CONRR', NRCOR', NRCONR'R", NRSO₂R' und/oder NRCOR' substituiertes, lineares oder verzweigtes zyklisches Alkyl mit 3-7 C-Atomen, worin eine, zwei oder drei CH₂-Gruppen unabhängig voneinander durch O, S, SO, SO₂, NR, -OCO-,-NRCONR'-, -NRCO-, - NRSO₂R'-, -COO-, -CONR-,-C≡C-Gruppen und/oder durch -CH=CH-Gruppen und/oder auch 1-11 H-Atome durch F und/oder Cl ersetzt sein können,
R,R' unabhängig voneinander H, unsubstituiertes oder ein-, zwei- oder dreifach durch =S, =NR, =O, Hal, OH, NH₂, SO₂CH₃, SO₂NH₂, CN, CONH₂, NHCOCH₃, und/oder NHCONH₂ substituiertes, lineares oder verzweigtes Alkyl mit 1-10 C-Atomen, worin eine, zwei oder drei CH₂-Gruppen unabhängig voneinander durch O, S, SO, SO₂, NH, NCH₃, -OCO-, -NHCONH-, -NHCO-, - NRSO₂R'-, -COO-, -CONH-, -NCH₃CO-, -CONCH₃-, -C≡C-Gruppen und/oder durch -CH=CH-Gruppen und/oder auch 1-20 H-Atome durch F und/oder Cl ersetzt sein können, oder unsubstituiertes oder ein-, zwei- oder dreifach durch =S, =NR, =O, OH, NH₂, SO₂CH₃, SO₂NH₂, CN, CONH₂, NHCOCH₃, und/oder NHCONH₂ substituiertes zyklisches Alkyl mit 3-7 C-Atomen, worin eine, zwei oder drei CH₂-Gruppen unabhängig voneinander durch O, S, SO, SO₂, NH, NCH₃, -OCO-,-NHCONH-, -NHCO-, - NRSO₂R'-, -COO-, -CONH-,-NCH₃CO-, -CONCH₃- und/oder durch -CH=CH-Gruppen und/oder auch 1-11 H-Atome durch F und/oder Cl ersetzt sein können,
m 0 - 4,
n 0 - 2, und
Hal F, Cl, Br oder I
ist, sowie deren physiologisch unbedenklichen Salze, Solvate und Stereoisomere, einschließlich deren Mischungen in allen Verhältnissen.

2. Verbindungen nach Anspruch 1,
a.) (S)-2-((2S,3S)-2-{(3S,4S)-3-Amino-4-[(S)-2-((S)-2-tert-butoxycarbonylamino-4-methyl-pentanoylamino)-butyrylamino]-5-phenyl-pentanoylamino}-3-methyl-pentanoylamino)-3-methyl-buttersäuremethylester
b.) (S)-2-((2S,3S)-2-{(3S,4S)-3-Amino-4-[(S)-2-((S)-2-amino-4-methyl-pentanoylamino)butyrylamino]-5-phenyl-pentanoylamino}-3-methyl-pentanoylamino)-3-methyl-buttersäuremethylester
c.) (S)-2-((2S,3S)-2-{(3S,4S)-3-Amino-4-[(S)-4-methyl-2-(3-methyl-butyrylamino)-pentanoylamino]-5-phenyl-pentanoylamino}-3-methyl-pentanoylamino)-3-methyl-buttersäuremethylester
d.) (S)-2-[(2S,3S)-2-((3S,4S)-3-Amino-4-{(S)-2-[(S)-4-methyl-2-(3-methyl-butyrylamino)-pentanoylamino]-1-oxo-butylamino}-5-phenyl-pentanoylamino)-3-methyl-pentanoylamino]-3-methyl-buttersäuremethylester
e.) (3S,4S)-3-Amino-4-{(S)-2-[(S)-4-methyl-2-(3-methyl-butyrylamino)-pentanoylamino]-butyrylamino}-5-phenyl-pentansäure-(2-benzo[1,3]dioxol-5-yl-ethyl)-amid
f.) (3S,4S)-3-Amino-4-{(S)-2-[(S)-4-methyl-2-(3-methyl-butyrylamino)-pentanoylamino]-butyrylamino}-5-phenyl-pentansäure-[2-(3,4-dichloro-phenyl)-ethyl]-amid
g.) (S)-2-[(2S,3S)-2-((3S,4S)-3-Amino-4-{(S)-3-methyl-2-[2-(3-trifluoromethoxy-phenyl)-acetylamino]-butyrylamino}-5-phenyl-pentanoylamino)-3-methyl-pentanoylamino]-3-methyl-buttersäuremethylester
h.) (S)-2-[(2S,3S)-2-((3S,4S)-3-Amino-4-{(S)-3-methyl-2-[2-(naphthalen-1-yloxy)-acetylamino]-butyrylamino}-5-phenyl-pentanoylamino)-3-methyl-pentanoylamino]-3-methyl-buttersäuremethylester
i.) (S)-2-[(2S,3S)-2-((3S,4S)-3-Amino-4-{(S)-2-[2-(3-methanesulfonylamino-phenyl)-acetylamino]-3-methyl-butyrylamino}-5-phenyl-pentanoylamino)-3-methyl-pentanoylamino]-3-methyl-buttersäuremethylester
j.) (S)-2-[(2S,3S)-2-((3S,4S)-3-Amino-4-{(S)-3-methyl-2-[(S)-4-methyl-2-(3-methyl-butyrylamino)-pentanoylamino]-1-oxo-butytamino}-5-phenyl-pentanoy)amino)-3-methyl-pentanoylamino]-3-methyl-buttersäuremethylester
k.) (S)-2-[(2S,3S)-2-((3S,4S)-3-Amino-4-{(S)-2-[(S)-4-methyl-2-(3-methyl-butyrylamino)-pentanoylamino]-3-phenyl-propionylamino}-5-phenyl-pentanoylamino)-3-methyl-pentanoylamino]-3-methyl-buttersäuremethylester
I.) 1-[(2S,3S)-2-((S)-3-(S)-Amino-4-{(S)-3-methyl-2-[(S)-4-methyl-2-(3-methyl-butyrylamino)-pentanoylamino]-butyrylamino}-5-phenyl-pentanoylamino)-3-methyl-pentanoylamino]-cyclopropan-carbonsäureethylester
m.) (2S,3S)-2-[(S)-2-((S)-3-(S)-Amino-4-{(S)-3-methyl-2-[(S)-4-methyl-2-(3-methyl-butyrylamino)-pentanoylamino]-butyrylamino}-5-phenyl-pentanoylamino)-3-methyl-1-(S)-oxo-pentylamino]-3-methyl-pentansäuremethylester
n.) (S)-2-[(2S,3S)-2-((S)-3-(S)-Amino-4-{(S)-3-methyl-2-[(S)-4-methyl-2-(3-methyl-butyrylamino)-pentanoylamino]-butyrylamino}-5-phenyl-pentanoylamino)-3-methyl-pentanoylamino]-4-methyl-pentansäuremethylester
o.) (S)-2-[(S)-2-((3S,4S)-3-Amino-4-{(S)-3-methyl-2-[(S)-4-methyl-2-(3-methyl-butyrylamino)-pentanoylamino]-butyrylamino}-5-phenyl-pentanoylamino)-3-phenyl-propionylamino]-3-methyl-buttersäuremethylester
p.) (S)-2-((2S,3S)-2-{(3S,4S)-3-Amino-4-[(S)-2-((S)-2-tert-butoxycarbonylamino-4-methyl-pentanoylamino)-3-methyl-butyrylamino]-5-phenyl-pentanoylamino}-3-methyl-pentanoylamino)-3-methyl-buttersäuremethylester
q.) (S)-2-((2S,3S)-2-{(3S,4S)-3-Amino-4-[(S)-3-methyl-2-(3-phenyl-propionylamino)-butyrylamino]-5-phenyl-pentanoylamino}-3-methyl-pentanoylamino)-3-methyl-buttersäuremethylester
r.) (S)-2-[(2S,3S)-2-((3S,4S)-3-Amino-4-{(S)-4-methyl-2-[(S)-4-methyl-2-(3-methyl-butyrylamino)-pentanoylamino]-pentanoylamino}-5-phenyl-pentanoylamino)-3-methyl-pentanoylamino]-3-methyl-buttersäuremethylester
s.) (S)-2-[(2S,3S)-2-((3S,4S)-3-Amino-4-{(S)-3-methyl-2-[(S)-2-(3-methyl-butyrylamino)-3-phenyl-propionylamino]-butyrylamino}-5-phenyl-pentanoylamino)-3-methyl-pentanoylamino]-3-methyl-buttersäuremethylester
t.) (S)-2-((2S,3S)-2-{(3S,4S)-3-Amino-4-[(S)-2-((S)-2-tert-butoxycarbonylamino-3-phenyl-propionylamino)-3-methyl-butyrylamino]-5-phenyl-pentanoylamino}-3-methyl-pentanoylamino)-3-methyl-buttersäuremethylester
u.) (S)-2-((2S,3S)-2-((3S,4S)-3-Amino-4-[(S)-2-((S)-2-amino-3-phenyl-propionylamino)-3-methyl-butyrylamino]-5-phenyl-pentanoylamino}-3-methyl-pentanoylamino)-3-methyl-buttersäuremethylester
v.) (S)-2-[(2S,3S)-2-((3S,4S)-3-Amino-4-{(S)-2-[2-(2,4-dichloro-phenoxy)-acetylamino]-3-methyl-butyrylamino}-5-phenyl-pentanoylamino)-3-methyl-pentanoylamino]-3-methyl-buttersäuremethylester
w.) (S)-2-[(2S,3S)-2-((3S,4S)-3-Amino-4-((S)-3-methyl-2-[(S)-4-methyl-2-(3-methyl-butyrylamino)-pentanoylamino]-butyrylamino}-5-phenyl-pentanoylamino)-3-methyl-pentanoylamino]-3-methyl-buttersäure
x.) (S)-3-Amino-4-{(S)-(S)-3-methyl-2-[(S)-4-methyl-2-(3-methyl-butyrylamino)-pentanoylamino]-butyrylamino}-5-phenyl-pentansäure-((1S,2S)-2-methyl-1-phenethylcarbamoyl-butyl)-amid
y.) (S)-3-Amino-4-{(S)-(S)-3-methyl-2-[(S)-4-methyl-2-(3-methyl-butyrylamino)-pentanoylamino]-butyrylamino}-5-phenyl-pentansäure-[(1S,2S)-2-methyl-1-(pyridin-3-ylcarbamoyl)-butyl]-amid
z.) (S)-2-((2S,3S)-2-{(3S,4S)-3-Amino-4-[(R)-2-((S)-2-amino-3-phenyl-propionylamino)-3-methyl-butyrylamino]-5-phenyl-pentanoylamino}-3-methyl-pentanoylamino)-3-methyl-buttersäuremethylester
aa.) (S)-3-Amino-4-{(S)-(S)-3-methyl-2-[(S)-4-methyl-2-(3-methyl-butyrylamino)-pentanoylamino]-butyrylamino}-5-phenyl-pentansäure-[(1S,2S)-2-methyl-1-(1-methyl-1H-pyrazol-3-ylcarbamoyl)-butyl]-amid
bb.) (S)-2-[(S)-2-((3S,4S)-3-Amino-4-{(S)-3-methyl-2-[(S)-4-methyl-2-(3-methyl-butyrylamino)-pentanoylamino]-butyrylamino}-5-phenyl-pentanoylamino)-3-naphthalen-1-yl-propionylamino]-3-methyl-buttersäuremethylester
cc.) (S)-3-Amino-4-{(S)-(S)-3-methyl-2-[(S)-4-methyl-2-(3-methyl-butyrylamino)-pentanoylamino]-butyrylamino}-5-phenyl-pentansäure-((1S,2S)-1-benzylcarbamoyl-2-methyl-butyl)-amid
dd.) (S)-3-(S)-Amino-4-{(S)-3-methyl-2-[(S)-4-methyl-2-(3-methyl-butyrylamino)-pentanoylamino]-butyrylamino}-5-phenyl-pentansäure-[(1S,2S)-1-(cyclopropylmethyl-carbamoyl)-2-methylbutyl]-amid
ee.) (S)-2-[(2S,3S)-2-((3S,4S)-3-Amino-4-{(S)-3,3-dimethyl-2-[(S)-4-methyl-2-(3-methyl-butyrylamino)-pentanoylamino]-butyrylamino}-5-phenyl-pentanoylamino)-3-methyl-pentanoylamino]-3-methyl-buttersäuremethylester
ff.) (S)-2-[(2S,3S)-2-((3S,4S)-3-Amino-4-{(S)-3-methyl-2-[2-(3-trifluoromethoxy-phenyl)-acetylamino]-butyrylamino}-5-phenyl-pentanoylamino)-3-methyl-pentanoylamino]-3-methyl-butyric acid
gg.) (S)-2-[(2S,3S)-2-((3S,4S)-3-Amino-4-{(S)-3-methyl-2-[(S)-2-(3-methyl-butyrylamino)-3-phenyl-propionylamino]-1-oxo-butylamino}-5-phenyl-pentanoylamino)-3-methyl-pentanoylamino]-3-methyl-buttersäure
hh.) (S)-3-(S)-Amino-4-{(S)-3-methyl-2-[(S)-4-methyl-2-(3-methyl-butyrylamino)-pentanoylamino]-butyrylamino}-5-phenyl-pentanoic acid [(1S,2S)-1-((S)-1-hydroxymethyl-2-methyl-propylcarbamoyl)-2-methyl-butyl]-amid
ii.) (3S,4S)-3-Amino-4-{(S)-3-methyl-2-[2-(3-trifluoromethoxy-phenyl)-acetylamino]-butyrylamino}-5-phenyl-pentansäure-[(S)-1-(3-methyl-butylcarbamoyl)-ethyl]-amid
jj.) (S)-3-(S)-Amino-4-{(S)-3-methyl-2-[2-(3-trifluoromethoxy-phenyl)-acetylamino]-butyrylamino}-5-phenyl-pentansäure-[(1S,2S)-2-methyl-1-(3-methyl-butylcarbamoyl)-butyl]-amid
kk.) (S)-2-[(2S,3S)-2-((3S,4S)-3-Amino-4-{(1S,4S)-3-methyl-2-[2-(3-trifluoromethoxy-phenyl)-acetylamino]-pentanoylamino}-5-phenyl-pentanoylamino)-3-methyl-pentanoylamino]-3-methyl-buttersäure
II.) (3S,4S)-3-Amino-4-{(S)-3-methyl-2-[(S)-4-methyl-2-(3-methyl-butyrylamino)-pentanoylamino]-butyrylamino}-5-phenyl-pentansäure-(2,6-diethyl-phenyl)-amid
mm.) (S)-3-(S)-Amino-4-{(S)-3-methyl-2-[(S)-4-methyl-2-(3-methyl-butyrylamino)-pentanoylamino]-butyrylamino}-5-phenyl-pentansäure-[(1S,2S)-1-(1-ethyl-propylcarbamoyl)-2-methyl-butyl]-amid
nn.) (3S,4S)-3-Amino-4-[(S)-3-methyl-2-(4-phenyl-butyrylamino)-butyrylamino]-5-phenyl-pentanoic acid (2,6-diethyl-phenyl)-amid
oo.) 3-(S)-Amino-4-{(S)-(2S,3S)-3-methyl-2-[2-(3-trifluoromethoxy-phenyl)-acetylamino]-pentanoylamino}-5-phenyl-pentansäure-{(2S,3S)-1-[1-((S)-hydroxymethyl)-2-methyl-propylcarbamoyl]-2-methyl-butyl}-amid
pp.) (S)-3-(S)-Amino-4-{(S)-3-methyl-2-[(S)-2-(3-methyl-butyrylamino)-3-phenyl-propionylamino]-butyrylamino}-5-phenyl-pentansäure-[(1S,2S)-1-((S)-1-hydroxymethyl-2-methyl-propylcarbamoyl)-2-methyl-butyl]-amid
qq.) (3S,4S)-3-Amino-4-{(S)-3-methyl-2-[(S)-4-methyl-2-(3-methyl-butyrylamino)-pentanoylamino]-butyrylamino}-5-phenyl-pentansäure-[(S)-2-methyl-1-(pyridin-3-yloxymethyl)-propyl]-amid
rr.) (3S,4S)-3-Amino-4-((S)-3-methyl-2-[(S)-4-methyl-2-(3-methyl-butyrylamino)-pentanoylamino]-butyrylamino}-5-phenyl-pentansäure-[(S)-1-(2,4-difluoro-phenoxymethyl)-2-methyl-propyl]-amid
ss.) (3S,4S)-3-Amino-4-{(S)-3-methyl-2-[(S)-4-methyl-2-(3-methyl-butyrylamino)-pentanoylamino]-butyrylamino}-5-phenyl-pentansäure-(S)-1-(2,4-difluoro-phenoxymethyl)-2-methyl-propyl]-amid
tt.) (3S,4S)-3-Amino-4-{(S)-3-methyl-2-[(S)-4-methyl-2-(3-methyl-butyrylamino)-pentanoylamino]-butyrylamino}-5-phenyl-pentansäure(1-benzyl-2-phenyl-ethyl)-amid
uu.) [(S)-1-((S)-1-{(1S,2S)-2-Amino-1-benzyl-3-[(S)-1-((S)-1-hydroxymethyl-2-methyl-propylcarbamoyl)-2-naphthalen-1-yl-ethylcarbamoyl]-propylcarbamoyl}-2-methyl-propylcarbamoyl)-2-phenyl-ethyl]-carbamoylsäure tert-butyl ester
vv.) (S)-3-(S)-Amino-4-{(S)-3-methyl-2-[(S)-4-methyl-2-(3-phenyl-propionylamino)-pentanoylamino]-1-oxo-butylamino}-5-phenyl-pentansäure [(1S,2S)-1-(1-ethyl-propylcarbamoyl)-2-methyl-butyl]-amid
ww.) (S)-3-(S)-Amino-4-{(S)-2-[(S)-2-(3,3-dimethyl-butyrylamino)-4-methyl-pentanoylamino]-3-methyl-1-oxo-butylamino}-5-phenyl-pentansäure [(1S,2S)-1-(1-ethyl-propylcarbamoyl)-2-methyl-butyl]-amid
xx.) (S)-2-[(2S,3S)-2-((S)-3-(S)-Amino-4-{(S)-3-methyl-2-[(S)-4-methyl-2-(3-phenyl-propionylamino)-pentanoylamino]-1-oxo-butylamino}-5-phenyl-pentanoylamino)-3-methyl-pentanoylamino]-3-methyl-buttersäure
yy.) (S)-2-[(2S,3S)-2-((S)-3-(S)-Amino-4-{(S)-3-methyl-2-[(S)-4-methyl-2-(4-trifluoromethoxy-benzoylamino)-pentanoylamino]-1-oxo-butylamino}-5-phenyl-pentanoylamino)-3-methyl-pentanoylamino]-3-methyl-buttersäure
zz.) (S)-2-((2S,3S)-2-{(S)-3-(S)-Amino-4-[(S)-3-methyl-2-((S)-4-methyl-2-phenylacetylamino-pentanoylamino)-1-oxo-butylamino]-5-phenyl-pentanoylamino}-3-methyl-pentanoylamino)-3-methyl-buttersäure
aaa.) N-[(S)-1-(1-{(S)-(S)-2-(S)-Amino-1-benzyl-3-[(1S,2S)-1-(1-ethyl-propylcarbamoyl)-2-methyl-butylcarbamoyl]-propylcarbamoyl}-2-methyl-propylcarbamoyl)-3-methyl-butyl]-4-trifluoromethoxy-benzamid
bbb.) (S)-2-[(2S,3S)-2-((S)-3-(S)-Amino-4-{(S)-2-[(S)-2-(3,3-dimethyl-butyrylamino)-4-methyl-pentanoylamino]-3-methyl-1-oxo-butylamino}-5-phenyl-pentanoylamino)-3-methyl-pentanoylamino]-3-methyl-buttersäure
ccc.) (S)-2-((2S,3S)-2-{(S)-3-(S)-Amino-4-[(S)-3-methyl-2-((S)-4-methyl-2-pentanoylamino-pentanoylamino)-1-oxo-butylamino]-5-phenyl-pentanoylamino}-3-methyl-pentanoylamino)-3-methyl-buttersäure
ddd.) (S)-3-(S)-Amino-4-[(S)=3-methyl-2-((S)-4-methyl-2-phenylacetylamino-pentanoylamino)-1-oxo-butylamino]-5-phenyl-pentansäure[(1S,2S)-1-(1-ethyl-propylcarbamoyl)-2-methyl-butyl]-amid
eee.) (S)-3-(S)-Amino-4-[(S)-3-methyl-2-((S)-4-methyl-2-pentanoylamino-pentanoylamino)-1-oxo-butylamino]-5-phenyl-pentansäure [(1S,2S)-1-(1-ethyl-propylcarbamoyl)-2-methyl-butyl]-amid
sowie deren physiologisch unbedenklichen Salze, Solvate und
Stereoisomere, einschließlich deren Mischungen in allen Verhältnissen.

3. Verfahren zur Herstellung der Verbindungen nach Anspruch 1 oder 2,
**dadurch gekennzeichnet, dass** man
a) eine Verbindung der Formel II, worin I₁, I₂, I₃, I₄ und I₅ die in Anspruch 1 angegebenen Bedeutungen haben und Q und Q' zueinander orthogonale Aminoschutzgruppen bedeuten, mit einer Verbindung der Formel III, worin Y, L₂, n und A₂ die in Anspruch 1 angegebenen Bedeutungen haben, unter Ausbildung einer Peptidbindung zwischen der Carboxylgruppe der Verbindung der Formel II und der Aminogruppe der Verbindung der Formel III zu einer Verbindung der Formel IVa umsetzt und die Verbindung der Formel IVa durch Abspaltung der Schutzgruppe Q zu einer Verbindung der Formel IV umsetzt, 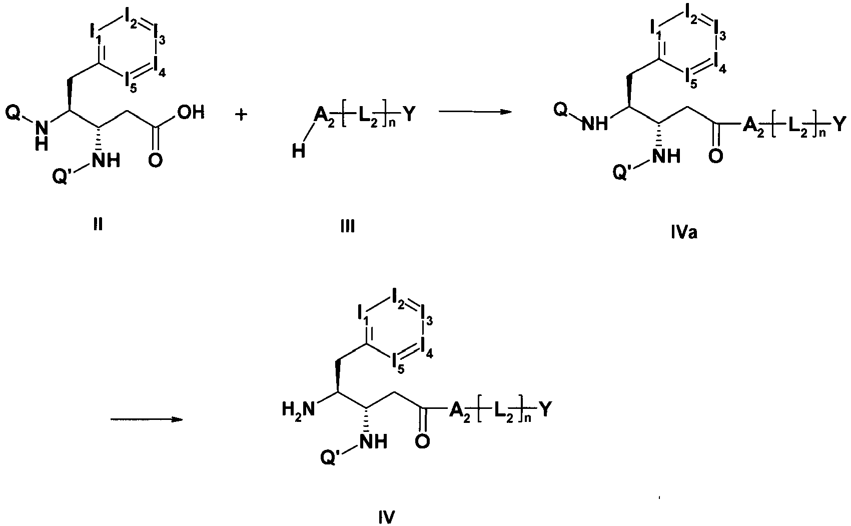 und eine Verbindung der Formel V, worin X, L₁, m und A₁ die in Anspruch 1 angegebenen Bedeutungen haben mit einer Verbindung der Formel VI, worin R¹ die in Anspruch 1 angegebene Bedeutung hat und W eine Säureschutzgruppe bedeutet, unter Ausbildung einer Peptidbindung zwischen der Carboxylgruppe der Verbindung der Formel V und der Aminogruppe der Verbindung der Formel VI zu einer Verbindung der Formel Vlla umsetzt und die Verbindung der Formel Vlla durch Abspaltung der Schutzgruppe W zu einer Verbindung der Formel VII umsetzt, 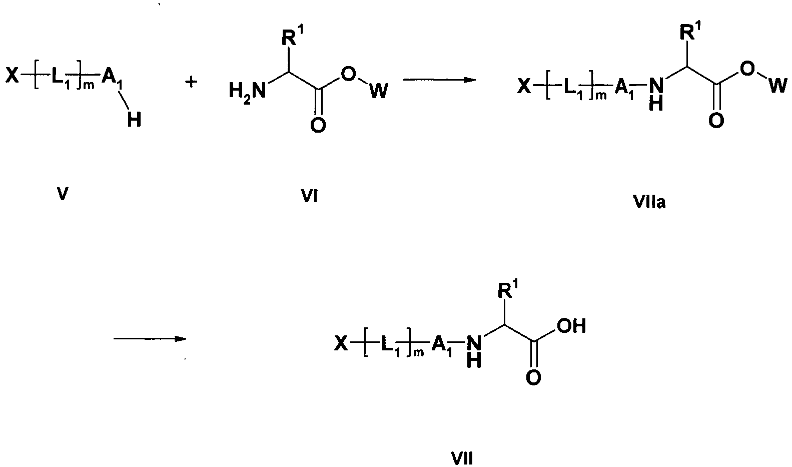 und eine Verbindung der Formel VII, worin X, L₁, m, A₁ und R¹ die in Anspruch 1 angegebenen Bedeutungen haben, mit einer Verbindung der Formel IV, worin I₁, I₂, I₃, I₄, I₅, Y, L₂, n und A₂ die in Anspruch 1 angegebenen Bedeutungen haben und Q' eine Aminoschutzgruppe ist, unter Ausbildung einer Peptidbindung zwischen der Carboxylgruppe der Verbindung der Formel VII und der Aminogruppe der Verbindung der Formel IV zu einer Verbindung der Formel la umsetzt und die Verbindung der Formel la durch Abspaltung der Schutzgruppe Q' zu einer Verbindung der Formel I umsetzt, 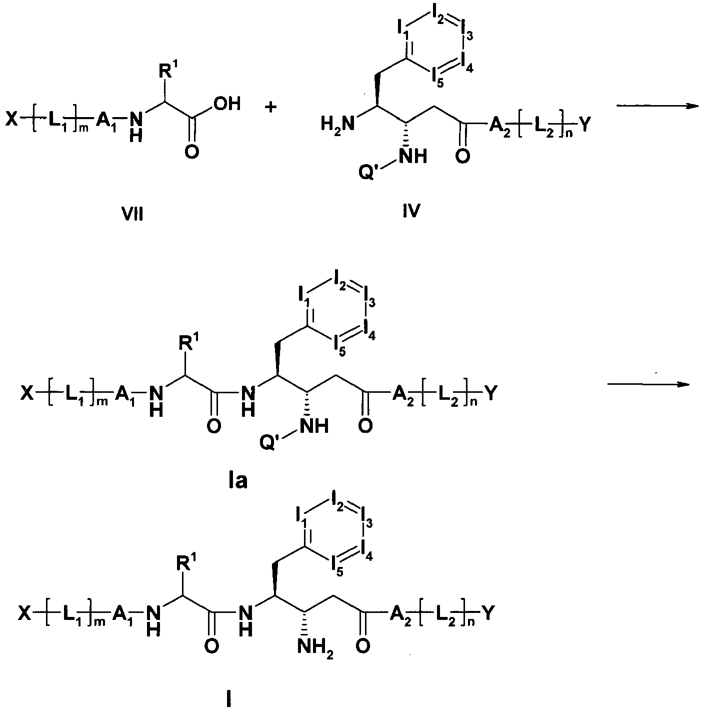
b) die Base einer Verbindung der Formel I durch Behandlung mit einer Säure in eines ihrer Salze überführt oder
c) eine Säure einer Verbindung der Formel I durch Behandlung mit einer Base in eines ihrer Salze überführt.

4. Verbindungen nach Anspruch 1 oder 2 sowie ihre physiologisch unbedenklichen Salze, Solvate und Stereoisomere, einschließlich deren Mischungen in allen Verhältnissen als Cathepsin D Inhibitoren.

5. Pharmazeutische Zubereitung, enthaltend wenigstens eine Verbindung nach Anspruch 1 oder 2 und/oder ihre physiologisch unbedenklichen Salze, Solvate und Stereoisomere, einschließlich deren Mischungen in allen Verhältnissen.

6. Pharmazeutische Zubereitung, nach Anspruch 5 enthaltend weitere Träger- und/oder Hilfsstoffe.

7. Pharmazeutische Zubereitung, enthaltend wenigstens eine Verbindung nach Anspruch 1 oder 2 und/oder ihre physiologisch unbedenklichen Salze, Solvate und Stereoisomere, einschließlich deren Mischungen in allen Verhältnissen sowie wenigstens einen weiteren Arzneimittelwirkstoff.

8. Verfahren zur Herstellung einer pharmazeutischen Zubereitung, **dadurch gekennzeichnet, dass** man eine Verbindung nach Anspruch 1 oder 2 und/oder eines ihrer physiologisch unbedenklichen Salze, Solvate und Stereoisomere, einschließlich deren Mischungen in allen Verhältnissen zusammen mit einem festen, flüssigen oder halbflüssigen Träger- oder Hilfsstoff in eine geeignete Dosierungsform bringt.

9. Arzneimittel enthaltend wenigstens eine Verbindung nach Anspruch 1 oder 2 und/oder eines ihrer physiologisch unbedenklichen Salze, Solvate und Stereoisomere, einschließlich deren Mischungen in allen Verhältnissen zur Verwendung bei der Behandlung und/oder Prophylaxe von physiologischen und/oder pathophysiologischen Zuständen.

10. Set (Kit) bestehend aus getrennten Packungen von
a) einer wirksamen Menge einer Verbindung nach Anspruch 1 oder 2 und/oder ihrer physiologisch unbedenklichen Salze, Solvate und Stereoisomere, einschließlich deren Mischungen in allen Verhältnissen und
b) einer wirksamen Menge eines weiteren Arzneimittelwirkstoffs.

11. Arzneimitteln enthaltend wenigstens eine Verbindung nach Anspruch 1 oder 2 und/oder eines ihrer physiologisch unbedenklichen Salze, Solvate und Stereoisomere, einschließlich deren Mischungen in allen Verhältnissen zur Verwendung bei der Behandlung und/oder Prophylaxe von physiologischen und/oder pathophysiologischen Zuständen, ausgewählt aus der Gruppe bestehend aus Arthrose, traumatische Knorpelverletzungen, Arthritis, Schmerz, Allodynie und Hyperalgesie.

12. Arzneimittel enthaltend wenigstens eine Verbindung nach Anspruch 1 oder 2 und/oder eines ihrer physiologisch unbedenklichen Salze, Solvate und Stereoisomere, einschließlich deren Mischungen in allen Verhältnissen zur Verwendung bei der Behandlung und/oder Prophylaxe von physiologischen und/oder pathophysiologischen Zuständen, ausgewählt aus der Gruppe bestehend aus Alzheimer Krankheit, Chorea Huntington, Mucolipidose, Krebs, insbesondere Brustkrebs, Kontaktdermatitis, Spät-Typ der Überempfindlichkeitsreaktion, Entzündungen, Endometriose, Vernarbung, gutartige Prostatahyperplasie, Osteosarkom, Rachitis, Hauterkrankungen wie beispielsweise Psoriasis, immunologische Krankheiten, Autoimmunkrankheiten und Immunschwächekrankheiten.

13. Arzneimittel nach Anspruch 11, zur Verwendung zur intraartikulären Applikation bei der Behandlung und/oder Prophylaxe von physiologischen und/oder pathophysiologischen Zuständen, ausgewählt aus der Gruppe bestehend aus Arthrose, traumatischen Knorpelverletzungen, Arthritis, Schmerz, Allodynie oder Hyperalgesie.

## Claims

1. Compounds of the formula I, 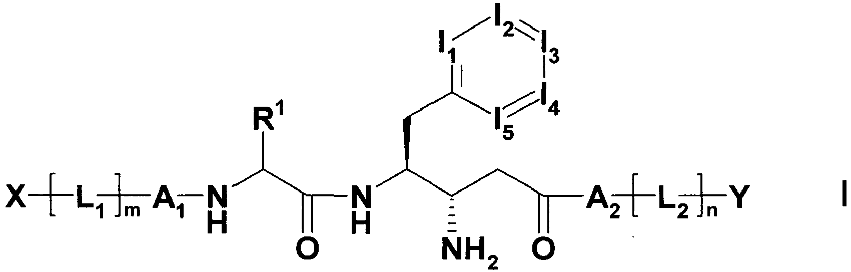 in which
I₁, I₂, I₃, I₄, I₅, independently of one another, are N or CR',
T is phenyl or naphthyl, each of which is unsubstituted or mono-, di-, tri- or tetrasubstituted by R, or a mono- or bicyclic saturated, unsaturated or aromatic heterocycle having 1 to 4 N, O and/or S atoms, which may be mono-, di- or trisubstituted by R, =S, =NR' and/or =O,
R¹ is ethyl, n-propyl, isopropyl, cyclopropyl, butyl, isobutyl, sec-butyl, cyclobutyl, tert-butyl, phenyl, benzyl, 2-oxetanyl, 3-oxetanyl, tetrahydrofuran-3-yl, tetrahydrofuran-2-yl, cyclopentyl, pentyl, methylsulfanylmethyl, ethylsulfanyl-methyl, 2-methylsulfanylethyl or 1-methylsulfanylethyl,
A₁ is 0 to 4 amino acid radicals which are bonded to one another in a peptide-like manner, selected from the group consisting of alanine, glycine, cyclopropylglycine, cyclo-butylglycine, cyclopentylglycine, cyclohexylglycine, 3-oxetanylglycine, 3-oxetanylglycine, tetrahydrofuran-3-ylglycine, tetrahydrofuran-2-ylglycine, ethylsulfanylmethyl-glycine, 2-methylsulfanylethylglycine, 1-methylsulfanyl-ethylglycine, valine, norvaline, aminobutyric acid, leucine, isoleucine, proline, tert-leucine, norleucine, methionine, phenylalanine, naphthylalanine, O-methylserine, O-ethyl-serine, -OCO-, -NRCO-, -SO₂- and -NRSO₂-,
A₂ is 0 to 4 amino acid radicals which are bonded to one another in a peptide-like manner, selected from the group consisting of alanine, glycine, cyclopropylglycine, cyclo-butylglycine, cyclopentylglycine, cyclohexylglycine, 3-oxetanylglycine, 3-oxetanylglycine, tetrahydrofuran-3-ylglycine, tetrahydrofuran-2-ylglycine, ethylsulfanylmethyl-glycine, 2-methylsulfanylethylglycine, 1-methylsulfanyl-ethylglycine, valine, norvaline, aminobutyric acid, leucine, isoleucine, proline, tert-leucine, norleucine, methionine, phenylalanine, naphthylalanine, O-methylserine and O-ethylserine,
L₁ is a single bond or -CRR'-,
L₂ is a single bond, -CRR'-, -NR-, -NRCR'R- or -NRCRR'CRR'-,
X, Y are H, T, linear or branched alkyl having 1-10 C atoms which is unsubstituted or mono-, di- or trisubstituted by =S, =NR, =O, R, T, OR, NRR', SOR, SO₂R, SO₂NRR', CN, COOR, CONRR', NRCOR', NRCONR'R", NRSO₂R' and/or NRCOR' and in which one, two or three CH₂ groups may be replaced, independently of one another, by O, S, SO, SO₂, NR, -OCO-, -NRCONR'-, -NRCO-, -NRSO₂R'-, -COO-, -CONR-, -C=C- groups and/or by -CH=CH- groups and/or, in addition, 1-20 H atoms may be replaced by F and/or Cl, or cyclic alkyl having 3-7 C atoms which is unsubstituted or mono-, di- or trisubstituted by =S, =NR, =O, R, T, OR, NRR', SOR, SO₂R, SO₂NRR', CN, COOR, CONRR', NRCOR', NRCONR'R", NRSO₂R' and/or NRCOR' and in which one, two or three CH₂ groups may be replaced, independently of one another, by O, S, SO, SO₂, NR, -OCO-, -NRCONR'-, -NRCO-, -NRSO₂R-, -COO-, -CONR-, -C=C- groups and/or by -CH=CH- groups and/or, in addition, 1-11 H atoms may be replaced by F and/or Cl,
R,R', independently of one another, are H, linear or branched alkyl having 1-10 C atoms which is unsubstituted or mono-, di- or trisubstituted by =S, =NR, =O, Hal, OH, NH₂, SO₂CH₃, SO₂NH₂, CN, CONH₂, NHCOCH₃ and/or NHCONH₂ and in which one, two or three CH₂ groups may be replaced, independently of one another, by O, S, SO, SO₂, NH, NCH₃, -OCO-, -NHCONH-, -NHCO-, -NRSO₂R'-, -COO-, -CONH-, -NCH₃CO-, -CONCH₃-, -C=C- groups and/or by -CH=CH- groups and/or, in addition, 1-20 H atoms may be replaced by F and/or Cl, or cyclic alkyl having 3-7 C atoms which is unsubstituted or mono-, di- or trisubstituted by =S, =NR, =O, OH, NH₂, SO₂CH₃, SO₂NH₂, CN, CONH₂, NHCOCH₃ and/or NHCONH₂ and in which one, two or three CH₂ groups may be replaced, independently of one another, by O, S, SO, SO₂, NH, NCH₃, -OCO-, -NHCONH-, -NHCO-, -NRSO₂R'-, -COO-, -CONH-, -NCH₃CO-, -CONCH₃- and/or by -CH=CH- groups and/or, in addition, 1-11 H atoms may be replaced by F and/or Cl,
m is 0 - 4,
n is 0 - 2, and
Hal is F, Cl, Br or I,
and physiologically acceptable salts, solvates and stereoisomers thereof, including mixtures thereof in all ratios.

2. Compounds according to Claim 1,
a.) methyl (S)-2-((2S,3S)-2-{(3S,4S)-3-amino-4-[(S)-2-((S)-2-tert-butoxycarbonylamino-4-methylpentanoylamino)butyrylamino]-5-phenylpentanoylamino}-3-methylpentanoylamino)-3-methylbutyrate
b.) methyl (S)-2-((2S,3S)-2-{(3S,4S)-3-amino-4-[(S)-2-((S)-2-amino-4-methylpentanoylamino)butyrylamino]-5-phenylpentanoylamino}-3-methylpentanoylamino)-3-methylbutyrate
c.) methyl (S)-2-((2S,3S)-2-{(3S,4S)-3-amino-4-[(S)-4-methyl-2-(3-methylbutyrylamino)pentanoylamino]-5-phenylpentanoylamino}-3-methylpentanoylamino)-3-methylbutyrate
d.) methyl (S)-2-[(2S,3S)-2-((3S,4S)-3-amino-4-{(S)-2-[(S)-4-methyl-2-(3-methylbutyrylamino)pentanoylamino]-1-oxobutylamino}-5-phenylpentanoylamino)-3-methylpentanoylamino]-3-methylbutyrate
e.) N-(2-benzo-1,3-dioxol-5-ylethyl)-(3S,4S)-3-amino-4-{(S)-2-[(S)-4-methyl-2-(3-methylbutyrylamino)pentanoylamino]butyrylamino}-5-phenylpentanamide
f.) N-[2-(3,4-dichlorophenyl)ethyl]-(3S,4S)-3-amino-4-{(S)-2-[(S)-4-methyl-2-(3-methylbutyrylamino)pentanoylamino]butyrylamino}-5-phenylpentanamide
g.) methyl (S)-2-[(2S,3S)-2-((3S,4S)-3-amino-4-{(S)-3-methyl-2-[2-(3-trifluoromethoxyphenyl)acetylamino]butyrylamino}-5-phenyl-pentanoylamino)-3-methylpentanoylamino]-3-methylbutyrate
h.) methyl (S)-2-[(2S,3S)-2-((3S,4S)-3-amino-4-{(S)-3-methyl-2-[2-(naphthalen-1-yloxy)acetylamino]butyrylamino}-5-phenylpentanoyl-amino)-3-methylpentanoylamino]-3-methylbutyrate
i.) methyl (S)-2-[(2S,3S)-2-((3S,4S)-3-amino-4-{(S)-2-[2-(3-methane-sulfonylaminophenyl)acetylamino]-3-methylbutyrylamino}-5-phenyl-pentanoylamino)-3-methylpentanoylamino]-3-methylbutyrate
j.) methyl (S)-2-[(2S,3S)-2-((3S,4S)-3-amino-4-{(S)-3-methyl-2-[(S)-4-methyl-2-(3-methylbutyrylamino)pentanoylamino]-1-oxobutyl-amino}-5-phenylpentanoylamino)-3-methylpentanoylamino]-3-methylbutyrate
k.) methyl (S)-2-[(2S,3S)-2-((3S,4S)-3-amino-4-{(S)-2-[(S)-4-methyl-2-(3-methylbutyrylamino)pentanoylamino]-3-phenylpropionylamino}-5-phenylpentanoylamino)-3-methylpentanoylamino]-3-methylbutyrate
I.) ethyl1-[(2S,3S)-2-((S)-3-(S)-amino-4-{(S)-3-methyl-2-[(S)-4-methyl-2-(3-methylbutyrylamino)pentanoylamino]butyrylamino}-5-phenylpentanoylamino)-3-methyl-pentanoylamino]cyclopropanecarboxylate
m.) methyl (2S,3S)-2-[(S)-2-((S)-3-(S)-amino-4-{(S)-3-methyl-2-[(S)-4-methyl-2-(3-methylbutyrylamino)pentanoylamino]butyrylamino}-5-phenylpentanoylamino)-3-methyl-1-(S)-oxopentylamino]-3-methyl-pentanoate
n.) methyl (S)-2-[(2S,3S)-2-((S)-3-(S)-amino-4-{(S)-3-methyl-2-[(S)-4-methyl-2-(3-methylbutyrylamino)pentanoylamino]butyrylamino}-5-phenylpentanoylamino)-3-methylpentanoylamino]-4-methyl-pentanoate
o.) methyl (S)-2-[(S)-2-((3S,4S)-3-amino-4-{(S)-3-methyl-2-[(S)-4-methyl-2-(3-methylbutyrylamino)pentanoylamino]butyrylamino}-5-phenylpentanoylamino)-3-phenylpropionylamino]-3-methylbutyrate
p.) methyl (S)-2-((2S,3S)-2-{(3S,4S)-3-amino-4-[(S)-2-((S)-2-tert-butoxycarbonylamino-4-methylpentanoylamino)-3-methylbutyryl-amino]-5-phenylpentanoylamino}-3-methylpentanoylamino)-3-methylbutyrate
q.) methyl (S)-2-((2S,3S)-2-{(3S,4S)-3-amino-4-[(S)-3-methyl-2-(3-phenylpropionylamino)butyrylamino]-5-phenylpentanoylamino}-3-methylpentanoylamino)-3-methylbutyrate
r.) methyl (S)-2-[(2S,3S)-2-((3S,4S)-3-amino-4-{(S)-4-methyl-2-[(S)-4-methyl-2-(3-methylbutyrylamino)pentanoylamino]pentanoylamino}-5-phenylpentanoylamino)-3-methylpentanoylamino]-3-methylbutyrate
s.) methyl (S)-2-[(2S,3S)-2-((3S,4S)-3-amino-4-{(S)-3-methyl-2-[(S)-2-(3-methylbutyrylamino)-3-phenylpropionylamino]butyrylamino}-5-phenylpentanoylamino)-3-methylpentanoylamino]-3-methylbutyrate
t.) methyl (S)-2-((2S,3S)-2-{(3S,4S)-3-amino-4-[(S)-2-((S)-2-tert-butoxycarbonylamino-3-phenylpropionylamino)-3-methylbutyryl-amino]-5-phenylpentanoylamino}-3-methylpentanoylamino)-3-methylbutyrate
u.) methyl (S)-2-((2S,3S)-2-{(3S,4S)-3-amino-4-[(S)-2-((S)-2-amino-3-phenylpropionylamino)-3-methylbutyrylamino]-5-phenylpentanoyl-amino}-3-methylpentanoylamino)-3-methylbutyrate
v.) methyl (S)-2-[(2S,3S)-2-((3S,4S)-3-amino-4-{(S)-2-[2-(2,4-dichloro-phenoxy)acetylamino]-3-methylbutyrylamino}-5-phenylpentanoyl-amino)-3-methylpentanoylamino]-3-methylbutyrate
w.) (S)-2-[(2S,3S)-2-((3S,4S)-3-amino-4-{(S)-3-methyl-2-[(S)-4-methyl-2-(3-methylbutyrylamino)pentanoylamino]butyrylamino}-5-phenyl-pentanoylamino)-3-methylpentanoylamino]-3-methylbutyric acid
x.) N-((1S,2S)-2-methyl-1-phenethylcarbamoylbutyl)-(S)-3-amino-4-{(S)-(S)-3-methyl-2-[(S)-4-methyl-2-(3-methylbutyrylamino)-pentanoylamino]butyrylamino}-5-phenylpentanamide
y.) N-[(1S,2S)-2-methyl-1-(pyridin-3-ylcarbamoyl)butyl]-(S)-3-amino-4-{(S)-(S)-3-methyl-2-[(S)-4-methyl-2-(3-methylbutyrylamino)-pentanoylamino]butyrylamino}-5-phenylpentanamide
z.) methyl (S)-2-((2S,3S)-2-{(3S,4S)-3-amino-4-[(R)-2-((S)-2-amino-3-phenylpropionylamino)-3-methylbutyrylamino]-5-phenylpentanoyl-amino}-3-methylpentanoylamino)-3-methylbutyrate
aa.) N-[(1S,2S)-2-methyl-1-(1-methyl-1H-pyrazol-3-ylcarbamoyl)butyl]-(S)-3-amino-4-{(S)-(S)-3-methyl-2-[(S)-4-methyl-2-(3-methylbutyryl-amino)pentanoylamino]butyrylamino}-5-phenylpentanamide
bb.) methyl (S)-2-[(S)-2-((3S,4S)-3-amino-4-{(S)-3-methyl-2-[(S)-4-methyl-2-(3-methylbutyrylamino)pentanoylamino]butyrylamino}-5-phenylpentanoylamino)-3-naphthalen-1-ylpropionylamino]-3-methylbutyrate
cc.) N-((1S,2S)-1-benzylcarbamoyl-2-methylbutyl)-(S)-3-amino-4-{(S)-(S)-3-methyl-2-[(S)-4-methyl-2-(3-methylbutyrylamino)pentanoyl-amino]butyrylamino}-5-phenylpentanamide
dd.) N-[(1S,2S)-1-(cyclopropylmethylcarbamoyl)-2-methylbutyl]-(S)-3-(S)-amino-4-{(S)-3-methyl-2-[(S)-4-methyl-2-(3-methylbutyryl-amino)pentanoylamino]butyrylamino}-5-phenylpentanamide
ee.) methyl (S)-2-[(2S,3S)-2-((3S,4S)-3-amino-4-{(S)-3,3-dimethyl-2-[(S)-4-methyl-2-(3-methyl-butyrylamino)pentanoylamino]butyrylamino}-5-phenylpentanoyl-amino)-3-methylpentanoylamino]-3-methylbutyrate
ff.) (S)-2-[(2S,3S)-2-((3S,4S)-3-amino-4-{(S)-3-methyl-2-[2-(3-trifluoromethoxyphenyl)acetylamino]butyrylamino}-5-phenylpentanoyl-amino)-3-methylpentanoylamino]-3-methylbutyric acid
gg.) (S)-2-[(2S,3S)-2-((3S,4S)-3-amino-4-{(S)-3-methyl-2-[(S)-2-(3-methylbutyrylamino)-3-phenylpropionylamino]-1-oxobutylamino}-5-phenylpentanoylamino)-3-methylpentanoylamino]-3-methylbutyric acid
hh.) N-[(1S,2S)-1-((S)-1-hydroxymethyl-2-methylpropylcarbamoyl)-2-methylbutyl]-(S)-3-(S)-amino-4-{(S)-3-methyl-2-[(S)-4-methyl-2-(3-methylbutyrylamino)pentanoylamino]butyrylamino}-5-phenylpentanamide
ii.) N-[(S)-1-(3-methylbutylcarbamoyl)ethyl]-(3S,4S)-3-amino-4-{(S)-3-methyl-2-[2-(3-trifluoromethoxyphenyl)acetylamino]butyrylamino}-5-phenylpentanamide
jj.) N-[(1S,2S)-2-methyl-1-(3-methylbutylcarbamoyl)butyl]-(S)-3-(S)-amino-4-{(S)-3-methyl-2-[2-(3-trifluoromethoxyphenyl)acetylamino]-butyrylamino}-5-phenylpentanamide
kk.) (S)-2-[(2S,3S)-2-((3S,4S)-3-amino-4-{(1S,4S)-3-methyl-2-[2-(3-trifluoromethoxyphenyl)acetylamino]pentanoylamino}-5-phenyl-pentanoylamino)-3-methylpentanoylamino]-3-methylbutyric acid
II.) N-(2,6-diethylphenyl)-(3S,4S)-3-amino-4-{(S)-3-methyl-2-[(S)-4-methyl-2-(3-methylbutyrylamino)pentanoylamino]butyrylamino}-5-phenylpentanamide
mm.) N-[(1S,2S)-1-(1-ethylpropylcarbamoyl)-2-methylbutyl]-(S)-3-(S)-amino-4-{(S)-3-methyl-2-[(S)-4-methyl-2-(3-methylbutyrylamino)-pentanoylamino]butyrylamino}-5-phenylpentanamide
nn.) N-(2,6-diethylphenyl)-(3S,4S)-3-amino-4-[(S)-3-methyl-2-(4-phenyl-butyrylamino)butyrylamino]-5-phenylpentanamide
oo.) N-{(2S,3S)-1-[1-((S)-hydroxymethyl)-2-methylpropylcarbamoyl]-2-methylbutyl}-3-(S)-amino-4-{(S)-(2S,3S)-3-methyl-2-[2-(3-trifluoromethoxyphenyl)acetylamino]pentanoylamino}-5-phenylpentanamide
pp.) N-[(1S,2S)-1-((S)-1-hydroxymethyl-2-methylpropylcarbamoyl)-2-methylbutyl]-(S)-3-(S)-amino-4-{(S)-3-methyl-2-[(S)-2-(3-methyl-butyrylamino)-3-phenylpropionylamino]butyrylamino}-5-phenylpentanamide
qq.) N-[(S)-2-methyl-1-(pyridin-3-yloxymethyl)propyl]-(3S,4S)-3-amino-4-{(S)-3-methyl-2-[(S)-4-methyl-2-(3-methylbutyrylamino)-pentanoylamino]butyrylamino}-5-phenylpentanamide
rr.) N-[(S)-1-(2,4-difluorophenoxymethyl)-2-methylpropyl]-(3S,4S)-3-amino-4-{(S)-3-methyl-2-[(S)-4-methyl-2-(3-methylbutyrylamino)-pentanoylamino]butyrylamino}-5-phenylpentanamide
ss.) N-[(S)-1-(2,4-difluorophenoxymethyl)-2-methylpropyl]-(3S,4S)-3-amino-4-{(S)-3-methyl-2-[(S)-4-methyl-2-(3-methylbutyrylamino)-pentanoylamino]butyrylamino}-5-phenylpentanamide
tt.) N-(1-benzyl-2-phenylethyl)-(3S,4S)-3-amino-4-{(S)-3-methyl-2-[(S)-4-methyl-2-(3-methylbutyrylamino)pentanoylamino]butyrylamino}-5-phenylpentanamide
uu.) tert-butyl [(S)-1-((S)-1-{(1S,2S)-2-amino-1-benzyl-3-[(S)-1-((S)-1-hydroxymethyl-2-methylpropylcarbamoyl)-2-naphthalen-1-ylethyl-carbamoyl]propylcarbamoyl}-2-methylpropylcarbamoyl)-2-phenylethyl]carbamate
vv.) N-[(1S,2S)-1-(1-ethylpropylcarbamoyl)-2-methylbutyl]-(S)-3-(S)-amino-4-{(S)-3-methyl-2-[(S)-4-methyl-2-(3-phenylpropionylamino)-pentanoylamino]-1-oxobutylamino}-5-phenylpentanamide
ww.) N-[(1S,2S)-1-(1-ethylpropylcarbamoyl)-2-methylbutyl]-(S)-3-(S)-amino-4-{(S)-2-[(S)-2-(3,3-dimethylbutyrylamino)-4-methyl-pentanoylamino]-3-methyl-1-oxobutylamino}-5-phenylpentanamide
xx.) (S)-2-[(2S,3S)-2-((S)-3-(S)-amino-4-{(S)-3-methyl-2-[(S)-4-methyl-2-(3-phenylpropionylamino)pentanoylamino]-1-oxobutylamino}-5-phenylpentanoylamino)-3-methylpentanoylamino]-3-methylbutyric acid
yy.) (S)-2-[(2S,3S)-2-((S)-3-(S)-amino-4-{(S)-3-methyl-2-[(S)-4-methyl-2-(4-trifluoromethoxybenzoylamino)pentanoylamino]-1-oxobutyl-amino}-5-phenylpentanoylamino)-3-methylpentanoylamino]-3-methylbutyric acid
zz.) (S)-2-((2S,3S)-2-{(S)-3-(S)-amino-4-[(S)-3-methyl-2-((S)-4-methyl-2-phenylacetylaminopentanoylamino)-1-oxobutylamino]-5-phenyl-pentanoylamino}-3-methylpentanoylamino)-3-methylbutyric acid
aaa.) N-[(S)-1-(1-{(S)-(S)-2-(S)-amino-1-benzyl-3-[(1S,2S)-1-(1-ethyl-propylcarbamoyl)-2-methylbutylcarbamoyl]propylcarbamoyl}-2-methylpropylcarbamoyl)-3-methylbutyl]-4-trifluoromethoxybenz-amide
bbb.) (S)-2-[(2S,3S)-2-((S)-3-(S)-amino-4-{(S)-2-[(S)-2-(3,3-dimethyl-butyrylamino)-4-methylpentanoylamino]-3-methyl-1-oxobutyl-amino}-5-phenylpentanoylamino)-3-methylpentanoylamino]-3-methylbutyric acid
ccc.) (S)-2-((2S,3S)-2-{(S)-3-(S)-amino-4-[(S)-3-methyl-2-((S)-4-methyl-2-pentanoylaminopentanoylamino)-1-oxobutylamino]-5-phenyl-pentanoylamino}-3-methylpentanoylamino)-3-methylbutyric acid
ddd.) N-[(1S,2S)-1-(1-ethylpropylcarbamoyl)-2-methylbutyl]-(S)-3-(S)-amino-4-[(S)-3-methyl-2-((S)-4-methyl-2-phenylacetylamino-pentanoylamino)-1-oxobutylamino]-5-phenylpentanamide
eee.) N-[(1S,2S)-1-(1-ethylpropylcarbamoyl)-2-methylbutyl]-(S)-3-(S)-amino-4-[(S)-3-methyl-2-((S)-4-methyl-2-pentanoylaminopentanoyl-amino)-1-oxobutylamino]-5-phenylpentanamide
and physiologically acceptable salts, solvates and stereoisomers thereof, including mixtures thereof in all ratios.

3. Process for the preparation of the compounds according to Claim 1 or 2 **characterised in that**
a) a compound of the formula II, in which I₁, I₂, I₃, I₄ and I₅ have the meanings indicated in Claim 1 and Q and Q' denote mutually orthogonal amino-protecting groups, is reacted with a compound of the formula III, in which Y, L₂, n and A₂ have the meanings indicated in Claim 1, with formation of a peptide bond between the carboxyl group of the compound of the formula II and the amino group of the compound of the formula III, to form a compound of the formula IVa, and the compound of the formula IVa is converted into a compound of the formula IV by cleaving-off of the protecting group Q, 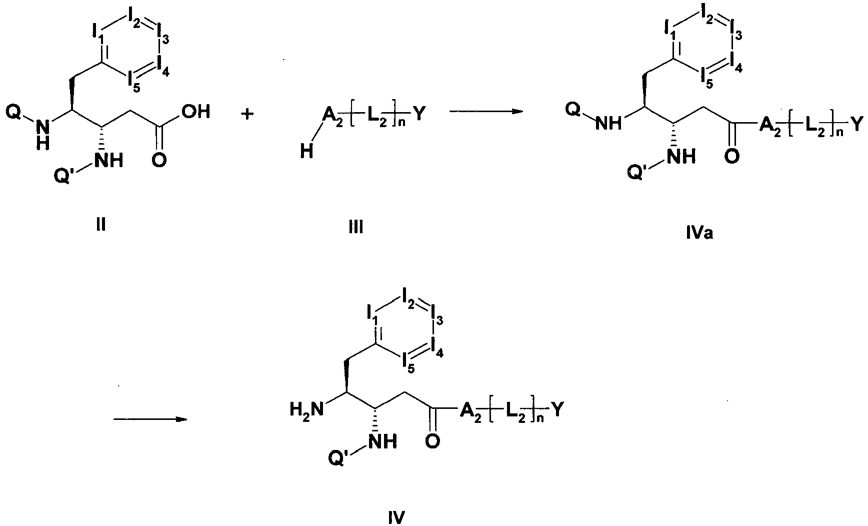 and a compound of the formula V, in which X, L₁, m and A₁ have the meanings indicated in Claim 1, is reacted with a compound of the formula VI, in which R¹ has the meaning indicated in Claim 1 and W denotes an acid-protecting group, with formation of a peptide bond between the carboxyl group of the compound of the formula V and the amino group of the compound of the formula VI, to form a compound of the formula VIIa, and the compound of the formula VIIa is converted into a compound of the formula VII by cleaving-off of the protecting group W, 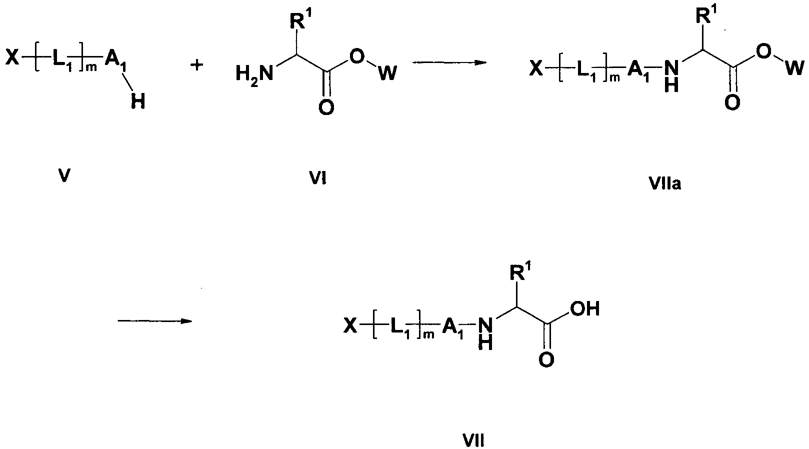 and a compound of the formula VII, in which X, L₁, m, A₁ and R¹ have the meanings indicated in Claim 1, is reacted with a compound of the formula IV, in which I₁, I₂, I₃, I₄, I₅, Y, L₂, n and A₂ have the meanings indicated in Claim 1 and Q' is an amino-protecting group, with formation of a peptide bond between the carboxyl group of the compound of the formula VII and the amino group of the compound of the formula IV, to form a compound of the formula Ia, and the compound of the formula la is converted into a compound of the formula I by cleaving-off of the protecting group Q', 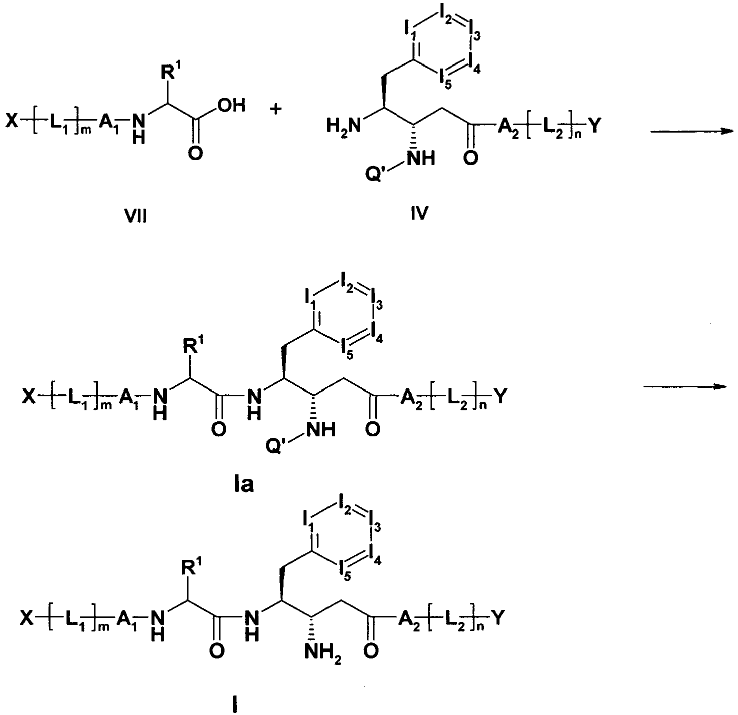
b) the base of a compound of the formula I is converted into one of its salts by treatment with an acid, or
c) an acid of a compound of the formula I is converted into one of its salts by treatment with a base.

4. Compounds according to Claim 1 or 2 and physiologically acceptable salts, solvates and stereoisomers thereof, including mixtures thereof in all ratios, as cathepsin D inhibitors.

5. Pharmaceutical preparation comprising at least one compound according to according to Claim 1 or 2 and physiologically acceptable salts, solvates and stereoisomers thereof, including mixtures thereof in all ratios.

6. Pharmaceutical preparation according to Claim 5 comprising further excipients and/or adjuvants.

7. Pharmaceutical preparation comprising at least one compound according to Claim 1 or 2 and physiologically acceptable salts, solvates and stereoisomers thereof, including mixtures thereof in all ratios, and at least one further medicament active compound.

8. Process for the preparation of a pharmaceutical preparation, **characterised in that** a compound according to Claim 1 or 2 and/or one of its physiologically acceptable salts, solvates and stereoisomers, including mixtures thereof in all ratios, is brought into a suitable dosage form together with a solid, liquid or semi-liquid excipient or adjuvant.

9. Medicament comprising at least one compound according to Claim 1 or 2 and/or one of its physiologically acceptable salts, solvates and stereoisomers, including mixtures thereof in all ratios, for use in the treatment and/or prophylaxis of physiological and/or pathophysiological states.

10. Set (kit) consisting of separate packs of
a) an effective amount of a compound according to Claim 1 or 2 and/or physiologically acceptable salts, solvates and stereoisomers thereof, including mixtures thereof in all ratios, and
b) an effective amount of a further medicament active compound.

11. Medicament comprising at least one compound according to Claim 1 or 2 and/or one of its physiologically acceptable salts, solvates and stereoisomers, including mixtures thereof in all ratios, for use in the treatment and/or prophylaxis of physiological and/or pathophysiological states selected from the group consisting of arthrosis, traumatic cartilage injuries, arthritis, pain, allodynia and hyperalgesia.

12. Medicament comprising at least one compound according to Claim 1 or 2 and/or one of its physiologically acceptable salts, solvates and stereoisomers, including mixtures thereof in all ratios, for use in the treatment and/or prophylaxis of physiological and/or pathophysiological states selected from the group consisting of Alzheimer's disease, Huntington's disease, mucolipidosis, cancer, in particular breast cancer, contact dermatitis, late-onset hypersensitivity reaction, inflammation, endometriosis, scarring, benign prostate hyperplasia, osteosarcoma, rickets, skin diseases, such as, for example, psoriasis, immunological diseases, autoimmune diseases and immunodeficiency diseases.

13. Medicament according to Claim 11 for use for intra-articular administration in the treatment and/or prophylaxis of physiological and/or pathophysiological states selected from the group consisting of arthrosis, traumatic cartilage injuries, arthritis, pain, allodynia and hyperalgesia.

## Revendications

1. Composés de formule I, 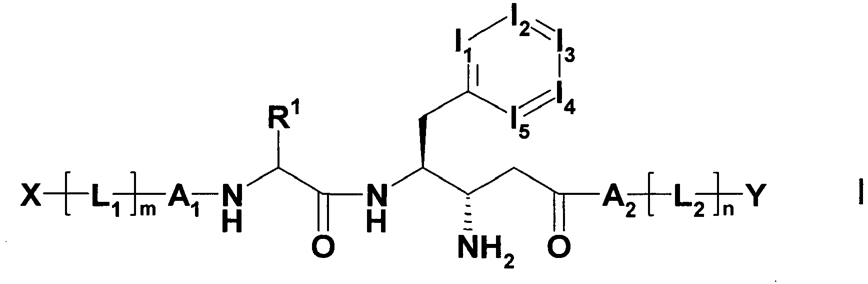 dans lesquels
I₁, I₂, I₃, I₄, I₅, indépendamment les uns des autres, sont N ou CR',
T est phényle ou naphtyle, chacun d'entre eux étant non substitué ou mono-, di-, tri- ou tétrasubstitué par R, ou un hétérocycle mono- ou bicyclique saturé, insaturé ou aromatique ayant de 1 à 4 atomes de N, O et/ou S, pouvant être mono-, di- ou trisubstitué par R, =S, =NR' et/ou =O,
R¹ est éthyle, n-propyle, isopropyle, cyclopropyle, butyle, isobutyle, sec-butyle, cyclobutyle, tertio-butyle, phényle, benzyle, 2-oxétanyle, 3-oxétanyle, tétrahydrofuran-3-yle, tétrahydrofuran-2-yle, cyclopentyle, pentyle, méthyl-sulfanylméthyle, éthylsulfanylméthyle, 2-méthylsulfanyl-éthyle ou 1-méthylsulfanyléthyle,
A₁ est de 0 à 4 radicaux d'acides aminés qui sont liés les uns aux autres d'une manière de type peptide, choisis dans le groupe constitué par l'alanine, la glycine, la cyclopropyl-glycine, la cyclobutylglycine, la cyclopentylglycine, la cyclohexylglycine, la 3-oxétanylglycine, la 3-oxétanyl-glycine, la tétrahydrofuran-3-ylglycine, la tétrahydrofuran-2-ylglycine, l'éthylsulfanylméthylglycine, la 2-méthyl-sulfanyléthylglycine, la 1-méthylsulfanyléthylglycine, la valine, la norvaline, l'acide aminobutyrique, la leucine, l'isoleucine, la proline, la tertio-leucine, la norleucine, la méthionine, la phénylalanine, la naphtylalanine, la O-méthylsérine, la O-éthylsérine, -OCO-, -NRCO-, -SO₂- et -NRSO₂-,
A₂ est de 0 à 4 radicaux d'acides aminés qui sont liés les uns aux autres d'une manière de type peptide, choisis dans le groupe constitué par l'alanine, la glycine, la cyclopropyl-glycine, la cyclobutylglycine, la cyclopentylglycine, la cyclohexylglycine, la 3-oxétanylglycine, la 3-oxétanyl-glycine, la tétrahydrofuran-3-ylglycine, la tétrahydrofuran-2-ylglycine, éthylsulfanylméthylglycine, la 2-méthyl-sulfanyléthylglycine, la 1-méthylsulfanyléthylglycine, la valine, la norvaline, l'acide aminobutyrique, la leucine, l'iso-leucine, la proline, la tertio-leucine, la norleucine, la méthionine, la phénylalanine, la naphtylalanine, la O-méthylsérine et la O-éthylsérine,
L₁ est une liaison simple ou -CRR'-,
L₂ est une liaison simple, -CRR'-, -NR-, -NRCR'R- ou -NRCRR'CRR'-,
X, Y sont H, T, alkyle linéaire ou ramifié ayant 1-10 atomes de C qui est non substitué ou mono-, di- ou trisubstitué par =S, =NR, =O, R, T, OR, NRR', SOR, SO₂R, SO₂NRR', CN, COOR, CONRR', NRCOR', NRCONR'R", NRSO₂R' et/ou NRCOR' et où un, deux ou trois groupements CH₂ peuvent être remplacés, indépendamment les uns des autres, par des groupements O, S, SO, SO₂, NR, -OCO-, -NRCONR'-, -NRCO-, -NRSO₂R'-, -COO-, -CONR-, -C=C- et/ou par des groupements -CH=CH- et/ou, en outre, 1-20 atomes de H peuvent être remplacés par F et/ou CI, ou alkyle cyclique ayant 3-7 atomes de C qui est non substitué ou mono-, di- ou trisubstitué par =S, =NR, =O, R, T, OR, NRR', SOR, SO₂R, SO₂NRR', CN, COOR, CONRR', NRCOR', NRCONR'R", NRSO₂R' et/ou NRCOR' et où un, deux ou trois groupements CH₂ peuvent être remplacés, indépendamment les uns des autres, par des groupements O, S, SO, SO₂, NR, -OCO-, -NRCONR'-, -NRCO-, -NRSO₂R'-, -COO-, -CONR-, -C=C- et/ou par des groupements -CH=CH- et/ou, en outre, 1-11 atomes de H peuvent être remplacés par F et/ou Cl,
R, R', indépendamment les uns des autres, sont H, alkyle linéaire ou ramifié ayant 1-10 atomes de C qui est non substitué ou mono-, di- ou trisubstitué par =S, =NR, =O, Hal, OH, NH₂, SO₂CH₃, SO₂NH₂, CN, CONH₂, NHCOCH₃ et/ou NHCONH₂ et où un, deux ou trois groupements CH₂ peuvent être remplacés, indépendamment les uns des autres, par des groupements O, S, SO, SO₂, NH, NCH₃, -OCO-, -NHCONH-, -NHCO-, -NRSO₂R'-, -COO-, -CONH-, -NCH₃CO-, -CONCH₃-, -C=C- et/ou par des groupements -CH=CH- et/ou, en outre, 1-20 atomes de H peuvent être remplacés par F et/ou Cl, ou alkyle cyclique ayant 3-7 atomes de C qui est non substitué ou mono-, di- ou trisubstitué par =S, =NR, =O, OH, NH₂, SO₂CH₃, SO₂NH₂, CN, CONH₂, NHCOCH₃ et/ou NHCONH₂ et où un, deux ou trois groupements CH₂ peuvent être remplacés, indépendamment les uns des autres, par O, S, SO, SO₂, NH, NCH₃, -OCO-, -NHCONH-, -NHCO-, -NRSO₂R'-, -COO-, -CONH-, -NCH₃CO-, -CONCH₃- et/ou par des groupements -CH=CH- et/ou, en outre, 1-11 atomes de H peuvent être remplacés par F et/ou CI,
m vaut 0 - 4,
n vaut 0 - 2, et
Hal est F, Cl, Br ou I,
ainsi que les sels, solvates et stéréoisomères physiologiquement acceptables de ceux-ci, y compris des mélanges de ceux-ci en toutes proportions.

2. Composés selon la revendication 1,
a.) (S)-2-((2S,3S)-2-{(3S,4S)-3-amino-4-[(S)-2-((S)-2-tertio-butoxy-carbonylamino-4-méthylpentanoylamino)butyrylamino]-5-phénylpentanoylamino}-3-méthylpentanoylamino)-3-méthylbutyrate de méthyle
b.) (S)-2-((2S,3S)-2-{(3S,4S)-3-amino-4-[(S)-2-((S)-2-amino-4-méthyl-pentanoylamino)butyrylamino]-5-phénylpentanoylamino}-3-méthyl-pentanoylamino)-3-méthylbutyrate de méthyle
c.) (S)-2-((2S,3S)-2-{(3S,4S)-3-amino-4-[(S)-4-méthyl-2-(3-méthyl-butyrylamino)pentanoylamino]-5-phénylpentanoylamino}-3-méthyl-pentanoylamino)-3-méthylbutyrate de méthyle
d.) (S)-2-[(2S,3S)-2-((3S,4S)-3-amino-4-{(S)-2-[(S)-4-méthyl-2-(3-méthylbutyrylamino)pentanoylamino]-1-oxobutylamino}-5-phénylpentanoylamino)-3-méthylpentanoylamino]-3-méthylbutyrate de méthyle
e.) N-(2-benzo-1,3-dioxol-5-yléthyl)-(3S,4S)-3-amino-4-{(S)-2-[(S)-4-méthyl-2-(3-méthylbutyrylamino)pentanoylamino]butyrylamino}-5-phénylpentanamide
f.) N-[2-(3,4-dichlorophényl)éthyl]-(3S,4S)-3-amino-4-{(S)-2-[(S)-4-méthyl-2-(3-méthylbutyrylamino)pentanoylamino]butyrylamino}-5-phénylpentanamide
g.) (S)-2-[(2S,3S)-2-((3S,4S)-3-amino-4-{(S)-3-méthyl-2-[2-(3-trifluoro-méthoxyphényl)acétylamino]butyrylamino}-5-phénylpentanoylamino)-3-méthylpentanoylamino]-3-méthylbutyrate de méthyle
h.) (S)-2-[(2S,3S)-2-((3S,4S)-3-amino-4-{(S)-3-méthyl-2-[2-(naphtalén-1-yloxy)acétylamino]butyrylamino}-5-phénylpentanoylamino)-3-méthylpentanoylamino]-3-méthylbutyrate de méthyle
i.) (S)-2-[(2S,3S)-2-((3S,4S)-3-amino-4-{(S)-2-[2-(3-méthanesulfonyl-aminophényl)acétylamino]-3-méthylbutyrylamino}-5-phénylpentanoylamino)-3-méthylpentanoylamino]-3-méthylbutyrate de méthyle
j.) (S)-2-[(2S,3S)-2-((3S,4S)-3-amino-4-{(S)-3-méthyl-2-[(S)-4-méthyl-2-(3-méthylbutyrylamino)pentanoylamino]-1-oxobutylamino}-5-phénylpentanoylamino)-3-méthylpentanoylamino]-3-méthylbutyrate de méthyle
k.) (S)-2-[(2S,3S)-2-((3S,4S)-3-amino-4-{(S)-2-[(S)-4-méthyl-2-(3-méthylbutyrylamino)pentanoylamino]-3-phénylpropionylamino}-5-phénylpentanoylamino)-3-méthylpentanoylamino]-3-méthylbutyrate de méthyle
l.) 1-[(2S,3S)-2-((S)-3-(S)-amino-4-{(S)-3-méthyl-2-[(S)-4-méthyl-2-(3-méthylbutyrylamino)pentanoylamino]butyrylamino}-5-phénylpentanoylamino)-3-méthylpentanoylamino]cyclopropane-carboxylate d'éthyle
m.) (2S,3S)-2-[(S)-2-((S)-3-(S)-amino-4-{(S)-3-méthyl-2-[(S)-4-méthyl-2-(3-méthylbutyrylamino)pentanoylamino]butyrylamino}-5-phénylpentanoylamino)-3-méthyl-1-(S)-oxopentylamino]-3-méthyl-pentanoate de méthyle
n.) (S)-2-[(2S,3S)-2-((S)-3-(S)-amino-4-{(S)-3-méthyl-2-[(S)-4-méthyl-2-(3-méthylbutyrylamino)pentanoylamino]butyrylamino}-5-phénylpentanoylamino)-3-méthylpentanoylamino]-4-méthylpentanoate de méthyle
o.) (S)-2-[(S)-2-((3S,4S)-3-amino-4-{(S)-3-méthyl-2-[(S)-4-méthyl-2-(3-méthylbutyrylamino)pentanoylamino]butyrylamino}-5-phénylpentanoylamino)-3-phénylpropionylamino]-3-méthylbutyrate de méthyle
p.) (S)-2-((2S,3S)-2-{(3S,4S)-3-amino-4-[(S)-2-((S)-2-tertio-butoxy-carbonylamino-4-méthylpentanoylamino)-3-méthylbutyrylamino]-5-phénylpentanoylamino}-3-méthylpentanoylamino)-3-méthylbutyrate de méthyle
q.) (S)-2-((2S,3S)-2-{(3S,4S)-3-amino-4-[(S)-3-méthyl-2-(3-phényl-propionylamino)butyrylamino]-5-phénylpentanoylamino}-3-méthyl-pentanoylamino)-3-méthylbutyrate de méthyle
r.) (S)-2-[(2S,3S)-2-((3S,4S)-3-amino-4-{(S)-4-méthyl-2-[(S)-4-méthyl-2-(3-méthylbutyrylamino)pentanoylamino]pentanoylamino}-5-phénylpentanoylamino)-3-méthylpentanoylamino]-3-méthylbutyrate de méthyle
s.) (S)-2-[(2S,3S)-2-((3S,4S)-3-amino-4-{(S)-3-méthyl-2-[(S)-2-(3-méthylbutyrylamino)-3-phénylpropionylamino]butyrylamino}-5-phénylpentanoylamino)-3-méthylpentanoylamino]-3-méthylbutyrate de méthyle
t.) (S)-2-((2S,3S)-2-{(3S,4S)-3-amino-4-[(S)-2-((S)-2-tertio-butoxy-carbonylamino-3-phénylpropionylamino)-3-méthylbutyrylamino]-5-phénylpentanoylamino}-3-méthylpentanoylamino)-3-méthylbutyrate de méthyle
u.) (S)-2-((2S,3S)-2-{(3S,4S)-3-amino-4-[(S)-2-((S)-2-amino-3-phényl-propionylamino)-3-méthylbutyrylamino]-5-phénylpentanoylamino}-3-méthylpentanoylamino)-3-méthylbutyrate de méthyle
v.) (S)-2-[(2S,3S)-2-((3S,4S)-3-amino-4-{(S)-2-[2-(2,4-dichloro-phénoxy)acétylamino]-3-méthylbutyrylamino}-5-phénylpentanoylamino)-3-méthylpentanoylamino]-3-méthylbutyrate de méthyle
w.) acide (S)-2-[(2S,3S)-2-((3S,4S)-3-amino-4-{(S)-3-méthyl-2-[(S)-4-méthyl-2-(3-méthylbutyrylamino)pentanoylamino]butyrylamino}-5-phénylpentanoylamino)-3-méthylpentanoylamino]-3-méthyl-butyrique
x.) N-((1S,2S)-2-méthyl-1-phénéthylcarbamoylbutyl)-(S)-3-amino-4-{(S)-(S)-3-méthyl-2-[(S)-4-méthyl-2-(3-méthylbutyrylamino)-pentanoylamino]butyrylamino}-5-phénylpentanamide
y.) N-[(1S,2S)-2-méthyl-1-(pyridin-3-ylcarbamoyl)butyl]-(S)-3-amino-4-{(S)-(S)-3-méthyl-2-[(S)-4-méthyl-2-(3-méthylbutyrylamino)-pentanoylamino]butyrylamino}-5-phénylpentanamide
z.) (S)-2-((2S,3S)-2-{(3S,4S)-3-amino-4-[(R)-2-((S)-2-amino-3-phényl-propionylamino)-3-méthylbutyrylamino]-5-phénylpentanoylamino}-3-méthylpentanoylamino)-3-méthylbutyrate de méthyle
aa.) N-[(1S,2S)-2-méthyl-1-(1-méthyl-1H-pyrazol-3-ylcarbamoyl)butyl]-(S)-3-amino-4-{(S)-(S)-3-méthyl-2-[(S)-4-méthyl-2-(3-méthylbutyryl-amino)pentanoylamino]butyrylamino}-5-phénylpentanamide
bb.) (S)-2-[(S)-2-((3S,4S)-3-amino-4-{(S)-3-méthyl-2-[(S)-4-méthyl-2-(3-méthylbutyrylamino)pentanoylamino]butyrylamino}-5-phénylpentanoylamino)-3-naphtalén-1-ylpropionylamino]-3-méthyl-butyrate de méthyle
cc.) N-((1S,2S)-1-benzylcarbamoyl-2-méthylbutyl)-(S)-3-amino-4-{(S)-(S)-3-méthyl-2-[(S)-4-méthyl-2-(3-méthylbutyrylamino)pentanoyl-amino]butyrylamino}-5-phénylpentanamide
dd.) N-[(1S,2S)-1-(cyclopropylméthylcarbamoyl)-2-méthylbutyl]-(S)-3-(S)-amino-4-{(S)-3-méthyl-2-[(S)-4-méthyl-2-(3-méthylbutyryl-amino)pentanoylamino]butyrylamino}-5-phénylpentanamide
ee.) (S)-2-[(2S,3S)-2-((3S,4S)-3-amino-4-((S)-3,3-diméthyl-2-[(S)-4-méthyl-2-(3-méthylbutyrylamino)pentanoylamino]butyrylamino}-5-phénylpentanoylamino)-3-méthylpentanoylamino]-3-méthylbutyrate de méthyle
ff.) acide (S)-2-[(2S,3S)-2-((3S,4S)-3-amino-4-{(S)-3-méthyl-2-[2-(3-trifluorométhoxyphényl)acétylamino]butyrylamino}-5-phénylpentanoylamino)-3-méthylpentanoylamino]-3-méthylbutyrique
gg.) acide (S)-2-[(2S,3S)-2-((3S,4S)-3-amino-4-{(S)-3-méthyl-2-[(S)-2-(3-méthylbutyrylamino)-3-phénylpropionylamino]-1-oxobutylamino}-5-phénylpentanoylamino)-3-méthylpentanoylamino]-3-méthyl-butyrique
hh.) N-[(1S,2S)-1-((S)-1-hydroxyméthyl-2-méthylpropylcarbamoyl)-2-méthylbutyl]-(S)-3-(S)-amino-4-{(S)-3-méthyl-2-[(S)-4-méthyl-2-(3-méthylbutyrylamino)pentanoylamino]butyrylamino}-5-phénylpentanamide
ii.) N-[(S)-1-(3-méthylbutylcarbamoyl)éthyl]-(3S,4S)-3-amino-4-{(S)-3-méthyl-2-[2-(3-trifluorométhoxyphényl)acétylamino]butyrylamino}-5-phénylpentanamide
jj.) N-[(1S,2S)-2-méthyl-1-(3-méthylbutylcarbamoyl)butyl]-(S)-3-(S)-amino-4-{(S)-3-méthyl-2-[2-(3-trifluorométhoxyphényl)acétylamino]-butyrylamino}-5-phénylpentanamide
kk.) acide (S)-2-[(2S,3S)-2-((3S,4S)-3-amino-4-{(1S,4S)-3-méthyl-2-[2-(3-trifluorométhoxyphényl)acétylamino]pentanoylamino}-5-phénylpentanoylamino)-3-méthylpentanoylamino]-3-méthylbutyrique
ll.) N-(2,6-diéthylphényl)-(3S,4S)-3-amino-4-{(S)-3-méthyl-2-[(S)-4-méthyl-2-(3-méthylbutyrylamino)pentanoylamino]butyrylamino}-5-phénylpentanamide
mm.) N-[(1S,2S)-1-(1-éthylpropylcarbamoyl)-2-méthylbutyl]-(S)-3-(S)-amino-4-{(S)-3-méthyl-2-[(S)-4-méthyl-2-(3-méthylbutyrylamino)-pentanoylamino]butyrylamino}-5-phénylpentanamide
nn.) N-(2,6-diéthylphényl)-(3S,4S)-3-amino-4-[(S)-3-méthyl-2-(4-phényl-butyrylamino)butyrylamino]-5-phénylpentanamide
oo.) N-{(2S,3S)-1-[1-((S)-hydroxyméthyl)-2-méthylpropylcarbamoyl]-2-méthylbutyl}-3-(S)-amino-4-{(S)-(2S,3S)-3-méthyl-2-[2-(3-trifluoro-méthoxyphényl)acétylamino]pentanoylamino}-5-phénylpentanamide
pp.) N-[(1S,2S)-1-((S)-1-hydroxyméthyl-2-méthylpropylcarbamoyl)-2-méthylbutyl]-(S)-3-(S)-amino-4-{(S)-3-méthyl-2-[(S)-2-(3-méthyl-butyrylamino)-3-phénylpropionylamino]butyrylamino}-5-phénylpentanamide
qq.) N-[(S)-2-méthyl-1-(pyridin-3-yloxyméthyl)propyl]-(3S,4S)-3-amino-4-{(S)-3-méthyl-2-[(S)-4-méthyl-2-(3-méthylbutyrylamino)-pentanoylamino]butyrylamino}-5-phénylpentanamide
rr.) N-[(S)-1-(2,4-difluorophénoxyméthyl)-2-méthylpropyl]-(3S,4S)-3-amino-4-{(S)-3-méthyl-2-[(S)-4-méthyl-2-(3-méthylbutyrylamino)-pentanoylamino]butyrylamino}-5-phénylpentanamide
ss.) N-[(S)-1-(2,4-difluorophénoxyméthyl)-2-méthylpropyl]-(3S,4S)-3-amino-4-{(S)-3-méthyl-2-[(S)-4-méthyl-2-(3-méthylbutyrylamino)-pentanoylamino]butyrylamino}-5-phénylpentanamide
tt.) N-(1-benzyl-2-phényléthyl)-(3S,4S)-3-amino-4-{(S)-3-méthyl-2-[(S)-4-méthyl-2-(3-méthylbutyrylamino)pentanoylamino]butyrylamino}-5-phénylpentanamide
uu.) [(S)-1-((S)-1-{(1S,2S)-2-amino-1-benzyl-3-[(S)-1-((S)-1-hydroxy-méthyl-2-méthylpropylcarbamoyl)-2-naphtalén-1-yléthylcarbamoyl]-propylcarbamoyl}-2-méthylpropylcarbamoyl)-2-phényléthyl]-carbamate de tertio-butyle
vv.) N-[(1S,2S)-1-(1-éthylpropylcarbamoyl)-2-méthylbutyl]-(S)-3-(S)-amino-4-{(S)-3-méthyl-2-[(S)-4-méthyl-2-(3-phénylpropionylamino)-pentanoylamino]-1-oxobutylamino}-5-phénylpentanamide
ww.) N-[(1S,2S)-1-(1-éthylpropylcarbamoyl)-2-méthylbutyl]-(S)-3-(S)-amino-4-{(S)-2-[(S)-2-(3,3-diméthylbutyrylamino)-4-méthyl-pentanoylamino]-3-méthyl-1-oxobutylamino}-5-phénylpentanamide
xx.) acide (S)-2-[(2S,3S)-2-((S)-3-(S)-amino-4-{(S)-3-méthyl-2-[(S)-4-méthyl-2-(3-phénylpropionylamino)pentanoylamino]-1-oxobutyl-amino}-5-phénylpentanoylamino)-3-méthylpentanoylamino]-3-méthylbutyrique
yy.) acide (S)-2-[(2S,3S)-2-((S)-3-(S)-amino-4-{(S)-3-méthyl-2-[(S)-4-méthyl-2-(4-trifluorométhoxybenzoylamino)pentanoylamino]-1-oxobutylamino}-5-phénylpentanoylamino)-3-méthylpentanoyl-amino]-3-méthylbutyrique
zz.) acide (S)-2-((2S,3S)-2-{(S)-3-(S)-amino-4-[(S)-3-méthyl-2-((S)-4-méthyl-2-phénylacétylaminopentanoylamino)-1-oxobutylamino]-5-phénylpentanoylamino}-3-méthylpentanoylamino)-3-méthyl-butyrique
aaa.) N-[(S)-1-(1-{(S)-(S)-2-(S)-amino-1-benzyl-3-[(1S,2S)-1-(1-éthyl-propylcarbamoyl)-2-méthylbutylcarbamoyl]propylcarbamoyl}-2-méthylpropylcarbamoyl)-3-méthylbutyl]-4-trifluorométhoxybenz-amide
bbb.) acide (S)-2-[(2S,3S)-2-((S)-3-(S)-amino-4-{(S)-2-[(S)-2-(3,3-diméthylbutyrylamino)-4-méthylpentanoylamino]-3-méthyl-1-oxobutylamino}-5-phénylpentanoylamino)-3-méthylpentanoyl-amino]-3-méthylbutyrique
ccc.) acide (S)-2-((2S,3S)-2-{(S)-3-(S)-amino-4-[(S)-3-méthyl-2-((S)-4-méthyl-2-pentanoylaminopentanoylamino)-1-oxobutylamino]-5-phénylpentanoylamino}-3-méthylpentanoylamino)-3-méthyl-butyrique
ddd.) N-[(1S,2S)-1-(1-éthylpropylcarbamoyl)-2-méthylbutyl]-(S)-3-(S)-amino-4-[(S)-3-méthyl-2-((S)-4-méthyl-2-phénylacétylamino-pentanoylamino)-1-oxobutylamino]-5-phénylpentanamide
eee.) N-[(1S,2S)-1-(1-éthylpropylcarbamoyl)-2-méthylbutyl]-(S)-3-(S)-amino-4-[(S)-3-méthyl-2-((S)-4-méthyl-2-pentanoylamino-pentanoylamino)-1-oxobutylamino]-5-phénylpentanamide
ainsi que les sels, solvates et stéréoisomères physiologiquement acceptables de ceux-ci, y compris des mélanges de ceux-ci en toutes proportions.

3. Procédé de préparation des composés selon la revendication 1 ou 2, **caractérisé en ce que**
a) un composé de formule II, où I₁, I₂, I₃, I₄ et I₅ revêtent les significations indiquées selon la revendication 1 et Q et Q' désignent des groupements protecteurs d'amino mutuellement orthogonaux, est réagi avec un composé de formule III, où Y, L₂, n et A₂ revêtent les significations indiquées selon la revendication 1, avec la formation d'une liaison peptidique entre le groupement carboxyle du composé de formule II et le groupement amino du composé de formule III, afin de former un composé de formule IVa, et le composé de formule IVa est converti en un composé de formule IV par l'élimination par clivage du groupement protecteur Q, 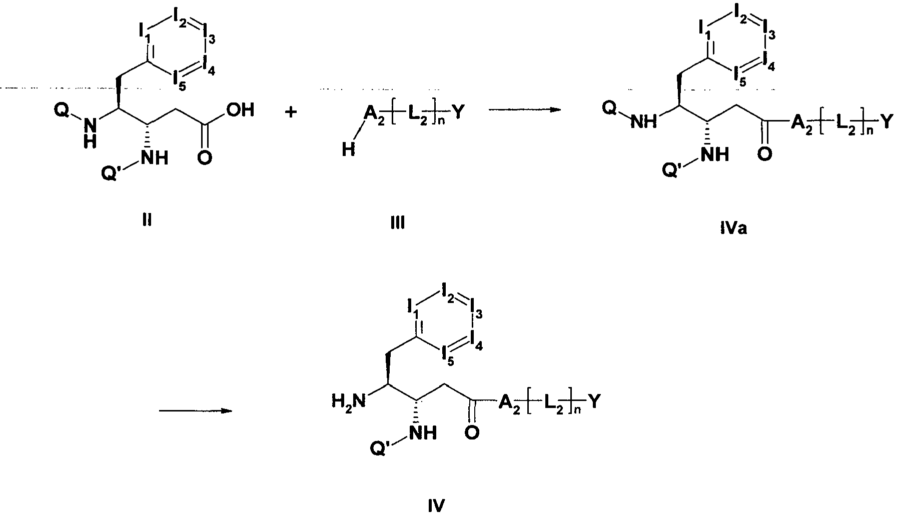 et un composé de formule V, où X, L₁, m et A₁ revêtent les significations indiquées selon la revendication 1, est réagi avec un composé de formule VI, où R¹ revêt la signification indiquée selon la revendication 1 et W désigne un groupement protecteur d'acide, avec la formation d'une liaison peptidique entre le groupement carboxyle du composé de formule V et le groupement amino du composé de formule VI, afin de former un composé de formule VIIa, et le composé de formule VIIa est converti en un composé de formule VII par l'élimination par clivage du groupement protecteur W, 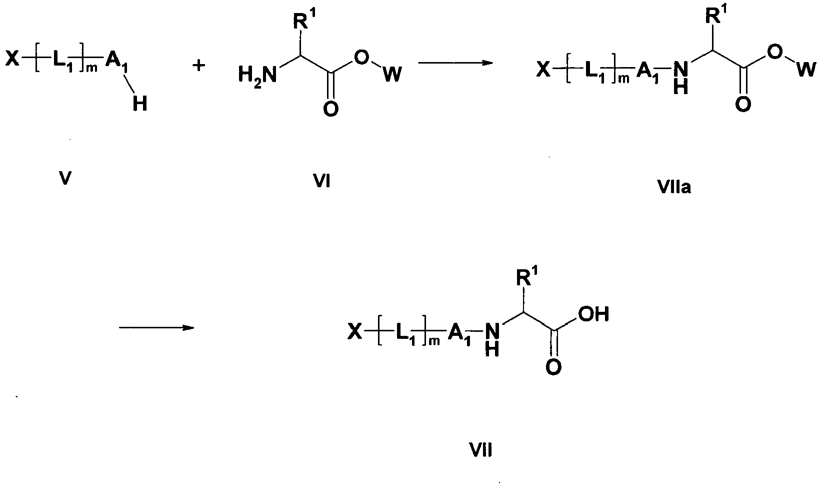 et un composé de formule VII, où X, L₁, m, A₁ et R¹ revêtent les significations indiquées selon la revendication 1, est réagi avec un composé de formule IV, où I₁, I₂, I₃, I₄, I₅, Y, L₂, n et A₂ revêtent les significations indiquées selon la revendication 1 et Q' est un groupement protecteur d'amino, avec la formation d'une liaison peptidique entre le groupement carboxyle du composé de formule VII et le groupement amino du composé de formule IV, afin de former un composé de formule la, et le composé de formule la est converti en un composé de formule I par l'élimination par clivage du groupement protecteur Q', 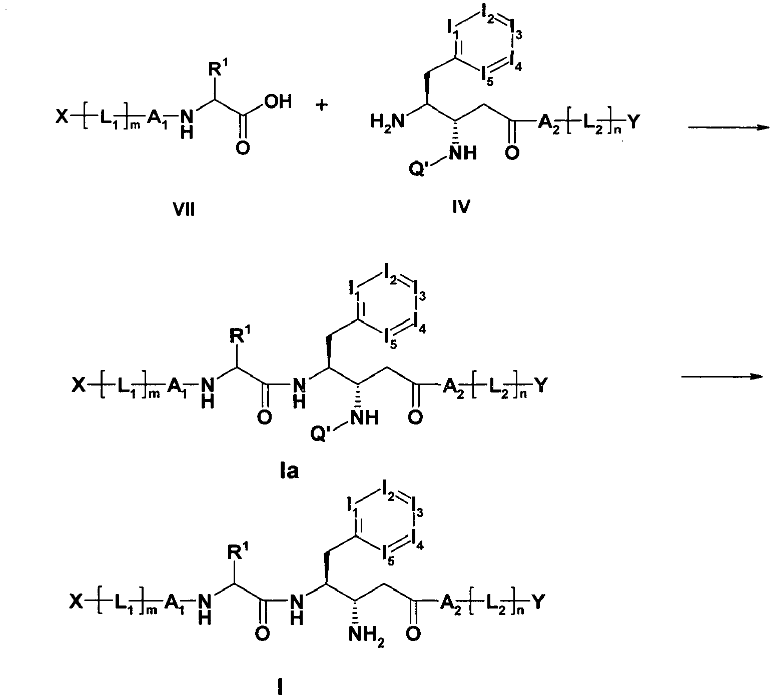
b) la base d'un composé de formule I est convertie en l'un de ses sels par un traitement par un acide, ou
c) un acide d'un composé de formule I est converti en l'un de ses sels par un traitement par une base.

4. Composés selon la revendication 1 ou 2 et des sels, solvates et stéréoisomères physiologiquement acceptables de ceux-ci, y compris des mélanges de ceux-ci en toutes proportions, comme inhibiteurs de la cathepsine D.

5. Préparation pharmaceutique comprenant au moins un composé selon la revendication 1 ou 2 et des sels, solvates et stéréoisomères physiologiquement acceptables de celui-ci, y compris des mélanges de ceux-ci en toutes proportions.

6. Préparation pharmaceutique selon la revendication 5, comprenant en outre des excipients et/ou des adjuvants.

7. Préparation pharmaceutique comprenant au moins un composé selon la revendication 1 ou 2 et des sels, solvates et stéréoisomères physiologiquement acceptables de celui-ci, y compris des mélanges de ceux-ci en toutes proportions, et au moins un autre composé actif médicamenteux.

8. Procédé de préparation d'une préparation pharmaceutique, **caractérisé en ce qu'**un composé selon la revendication 1 ou 2 et/ou l'un de ses sels, solvates et stéréoisomères physiologiquement acceptables, y compris des mélanges de ceux-ci en toutes proportions, est mis sous une forme de dosage convenable conjointement avec un excipient ou adjuvant solide, liquide ou semi-liquide.

9. Médicament comprenant au moins un composé selon la revendication 1 ou 2 et/ou l'un de ses sels, solvates et stéréoisomères physiologiquement acceptables, y compris des mélanges de ceux-ci en toutes proportions, pour une utilisation dans le traitement et/ou la prophylaxie d'états physiologiques et/ou physiopathologiques.

10. Ensemble (kit) constitué d'emballages séparés
a) d'une quantité efficace d'un composé selon la revendication 1 ou 2 et/ou de sels, solvates et stéréoisomères physiologiquement acceptables de celui-ci, y compris des mélanges de ceux-ci en toutes proportions, et
b) d'une quantité efficace d'un autre composé actif médicamenteux.

11. Médicament comprenant au moins un composé selon la revendication 1 ou 2 et/ou l'un de ses sels, solvates et stéréoisomères physiologiquement acceptables, y compris des mélanges de ceux-ci en toutes proportions, pour une utilisation dans le traitement et/ou la prophylaxie d'états physiologiques et/ou physiopathologiques, choisis dans le groupe constitué par l'arthrose, les lésions traumatiques du cartilage, l'arthrite, les douleurs, l'allodynie et l'hyperalgésie.

12. Médicament comprenant au moins un composé selon la revendication 1 ou 2 et/ou l'un de ses sels, solvates et stéréoisomères physiologiquement acceptables, y compris des mélanges de ceux-ci en toutes proportions, pour une utilisation dans le traitement et/ou la prophylaxie d'états physiologiques et/ou physiopathologiques choisis dans le groupe constitué par la maladie d'Alzheimer, la maladie d'Huntington, la mucolipidose, le cancer, en particulier le cancer du sein, la dermite de contact, la réaction d'hypersensibilité retardée, les inflammations, l'endométriose, la cicatrisation, l'hyperplasie bénigne de la prostate, l'ostéosarcome, le rachitisme, les maladies de la peau telles que par exemple le psoriasis, les maladies immunologiques, les maladies auto-immunes et les maladies d'immuno-déficience.

13. Médicament selon la revendication 11, pour une utilisation dans l'administration intra-articulaire dans le traitement et/ou la prophylaxie d'états physiologiques et/ou physiopathologiques choisis dans le groupe constitué par l'arthrose, les lésions traumatiques du cartilage, l'arthrite, les douleurs, l'allodynie et l'hyperalgésie.
